# EUROPEAN PATENT SPECIFICATION

(11) **EP 2 164 870 B1**
(45) Date of publication and mention of the grant of the patent: **09.12.2015**
(21) Application number: 08779128.1
(22) Date of filing: 25.06.2008
(51) Int. Cl.: C12N 15/11, C12N 15/63, C07K 16/18, A61P 35/00, A61K 39/395, A61P 35/04

(54) **POLYPEPTIDES AND POLYNUCLEOTIDES FOR ARTEMIN AND RELATED LIGANDS, AND METHODS OF USE THEREOF**
POLYPEPTIDE UND POLYNUKLEOTIDE FÜR ARTEMIN UND ENTSPRECHENDE LIGANDEN SOWIE VERFAHREN ZU IHRER ANWENDUNG
POLYPEPTIDES ET POLYNUCLÉOTIDES POUR L'ARTÉMINE ET LES LIGANDS APPARENTÉS, ET LEURS PROCÉDÉS D'UTILISATION

(30) Priority: 27.06.2007 US 946394 P
(43) Date of publication of application: 24.03.2010
(73) Proprietor: National University of Singapore, Singapore 119077 (SG)
(72) Inventor: LIU, Dongxu, Auckland (NZ); LOBIE, Peter Edward, Auckland (NZ)
(74) Representative: Bates, Philip Ian
(86) International application number: PCT/NZ2008/000152
(87) International publication number: WO 2009/002193

(56) References cited:
- WO-A1-00/18799
- WO-A2-00/01815
- WO-A2-00/04050
- US-A1- 2003 165 843
- US-A1- 2003 170 228
- US-A1- 2005 186 646
- US-A1- 2005 246 794
- US-A1- 2006 292 572
- WIESENHOFER BETTINA ET AL: "Glial cell line-derived neurotrophic factor (GDNF) is a proliferation factor for rat C6 glioma cells: Evidence from antisense experiments", ANTISENSE & NUCLEIC ACID DRUG DEVELOPMENT, MARY ANN LIEBERT, INC., NEW YORK, US, vol. 10, no. 5, 1 October 2000 (2000-10-01), pages 311-321, XP009109689, ISSN: 1087-2906
- DEBORAH H DAMON ET AL: "Vascular-derived artemin: a determinant of vascular sympathetic innervation?", AMERICAN JOURNAL OF PHYSIOLOGY: HEART AND CIRCULATORY PHYSIOLOGY, AMERICAN PHYSIOLOGICAL SOCIETY, US, vol. 293, no. 1, 2 March 2007 (2007-03-02) , pages H266-H273, XP002652782, ISSN: 0363-6135, DOI: 10.1152/AJPHEART.00859.2006 [retrieved on 2007-03-02]
- JENNIFER CHEN ET AL: "GDNF increases the survival of developing oculomotor neurons through a target-derived mechanism", MOLECULAR AND CELLULAR NEUROSCIENCE, vol. 24, no. 1, 1 September 2003 (2003-09-01), pages 41-56, XP55009593, ISSN: 1044-7431, DOI: 10.1016/S1044-7431(03)00098-8
- CEYHAN GÜRALP O ET AL: "The neurotrophic factor artemin promotes pancreatic cancer invasion", ANNALS OF SURGERY, J.B. LIPPINCOTT COMPANY, PHILADELPHIA, US, vol. 244, no. 2, 1 August 2006 (2006-08-01), pages 274-281, XP002505113, ISSN: 0003-4932, DOI: 10.1097/01.SLA.0000217642.68697.55
- TAO CHEN ET AL: "Functional characterization of artemin, a ferritin homolog synthesized in Artemia embryos during encystment and diapause", FEBS JOURNAL, vol. 274, no. 4, 1 February 2007 (2007-02-01), pages 1093-1101, XP55009560, ISSN: 1742-464X, DOI: 10.1111/j.1742-4658.2007.05659.x
- GÜRALP CEYHAN ET AL: "Der neurotropische Wachstumsfaktor Artemin induziert eine Steigerung der Krebszellinvasion im Pankreaskarzinom", CHIRURGISCHES FORUM 2005 FÜR EXPERIMENTELLE UND KLINISCHE FORSCHUNG : 122. KONGRESS DER DEUTSCHEN GESELLSCHAFT FÜR CHIRURGIE, DEUTSCHE GESELLSCHAFT FÜR CHIRURGIE, DE, vol. 34, 5 April 2005 (2005-04-05), pages 63-64, XP008144217, DOI: 10.1007/3-540-26560-0_21 ISBN: 978-3-540-24888-0 [retrieved on 2006-05-06]
- ITO ET AL: "Expression of glial cell line-derived neurotrophic factor family members and their receptors in pancreatic cancers", SURGERY, C.V. MOSBY CO., ST. LOUIS, US, vol. 138, no. 4, 1 October 2005 (2005-10-01), pages 788-794, XP005128517, ISSN: 0039-6060, DOI: 10.1016/J.SURG.2005.07.007
- T HIWASA: "GDNF-induced neurite formation was stimulated by protein kinase inhibitors and suppressed by Ras inhibitors", NEUROSCIENCE LETTERS, vol. 238, no. 3, 5 December 1997 (1997-12-05), pages 115-118, XP55009562, ISSN: 0304-3940, DOI: 10.1016/S0304-3940(97)00861-6

## Description

### FIELD OF THE INVENTION

The invention relates to polypeptides, polynucleotides, and antibodies, for Artemin (ARTN) and Persephin (PSPN). The invention also relates to expression vectors and host cells for producing these polypeptides, polynucleotides, or antibodies. The invention further relates to methods for diagnosis and treatment, especially for cancer, and particularly breast cancer and endometrial cancer using one or more of the disclosed polypeptides, polynucleotides, antibodies, expression vectors, host cells, or compositions thereof. Particularly related are inhibitors of Artemin and/or related ligands, and uses of these inhibitors.

### BACKGROUND OF THE INVENTION

Uncontrolled anchorage-independent cell growth is the hallmark of cancer and metastasis is the cause of cancer-related deaths. There is currently a search for new drugs that can be used to target specific pathways that are involved in cell proliferation and migration/invasion. This provides an alternative strategy for treatment of cancer, with less toxicity and high tolerability compared with traditional approaches, such as radiation, chemotherapy, and sex hormone therapy. It is well known that traditional chemotherapy agents are toxic both to healthy cells as well as to cancer cells. Moreover, prolonged exposure to chemotherapy results in the selection of drug-resistant cells.

Exemplary anti-cancer agents that target specific signalling molecules include Tykerb® (lapatinib) and Herceptin® (trastuzumab). Tykerb® is a dual tyrosine kinase inhibitor for epidermal growth factor receptor (EGFR) and human epidermal growth factor receptor 2 (HER-2), under development by GlaxoSmithKline as a treatment for solid tumours such as breast and lung cancer. Herceptin® is a humanised monoclonal antibody designed to target and block the function of HER2. An increasing number of compounds directed against the EGFR and HER2 receptors have entered clinical development and are currently in clinical trials.

Glial-cell-line-derived neurotrophic factor (GDNF) is a distant member of the transforming growth factor beta superfamily and a founding member of the GDNF family ligands (GFL). This family consists of four members: GDNF, Neurturin (NRTN), Artemin (ARTN) and Persephin (PSPN), all of which are potent neurotrophic factors (see, e.g., Airaksinen, M.S. and Saarma, M. (2002) The GDNF family: signalling, biological functions and therapeutic value. Nat. Rev. Neurosci. 3,383-394). GDNF family members are secreted proteins that include signal peptides. Each has a mature protein sequence of about 140 amino acid residues, and includes seven conserved and similarly spaced cysteine residues. The family members show approximately 40% amino acid identity to each other (Rosenblad *et al.,* 2000; Takahashi, 2001).

It is considered that the GDNF family members signal through a unique multicomponent receptor complex which includes glycosyl-phosphatidylinositol (GPI)-anchored co-receptor (GFRα) as a ligand binding component and RET receptor tyrosine kinase as a common signaling component (Takahashi, 2001). RET has a typical intracellular kinase domain, which interacts with downstream targets in the Ras/ERK-, PI3K/AKT-, p38/MAPK-, and JNK-pathways affecting cell growth, differentiation, survival, and apoptosis (Baudet *et al.,* 2000; Coulpier *et al.,* 2002). However, its extracellular domain is significantly different from other receptor tyrosine kinases. RET comprises four cadherin-like domains, which is unusual for an RTK, and a cysteine-rich domain (Santoro *et al.,* 2004; Knowles *et al.,* 2006).

Unlike any other known receptor tyrosine kinases, RET does not bind its ligands directly but requires a cell surface bound GFRα as a co-receptor. The co-receptors importantly provide signalling specificity for the binding of the ligand to the receptor complex. GDNF preferentially binds to GFRα1, NRTN to GFRα2, ARTN to GFRα3 and PSPN to GFRα4 (Airaksinen *et al.,* 2002). However, these binding specificities are not exclusive. For example, GDNF can bind to GFRα2 and GFRα3, but with lower affinity, and can activate RET. Whether this signalling has physiological significance remains unclear *(Bespalov et al.,* 2007).

The GDNF family members are expressed in many areas of the CNS during development and in adulthood. They are known to potently promote the survival of many types of neurons. Most notably, they are able to support the survival of dopaminergic and motomeurons. These neuronal populations die in the course of Parkinson's disease and amyotrophic lateral sclerosis (ALS) respectively. Studies with GDNF knock-out mice suggested that their major role is in the development of the enteric nervous system and regulation of renal organogenesis.

The GDNF family members are considered to have clinical importance for the treatment of Parkinson's disease (Gill *et al.,* 2003), Alzheimer's disease (Garces *et al.,* 2001; Golden *et al.,* 2003) and ALS (Henderson *et al.,* 1994). Other clinical applications include to be used as new targets for the treatments of alcoholism (He *et al.,* 2005), chronic pain (Gardell *et al.,* 2003), stroke (Tomac *et al.,* 2002), epilepsy (Horger *et al.,* 1998) and drug addiction (Airavaara *et al.,* 2004). For examples, GDNF and Neurturin have shown promise in clinical trials of Parkinson's disease, and Artemin is currently undergoing clinical trials for chronic pain treatment (Bespalov *et al.,* 2007). Recently, GDNF and Artemin have been demonstrated to influence neural invasion in patients with pancreatitis and pancreatic cancer (Ito *et al.,* 2005; Ceyhan *et al.,* 2006a; Ceyhan *et al.,* 2006b).

In medicine, there is a continuing need for new therapeutics, including new anti-cancer agents. In particular, there is a need to identify specific genes and proteins that can be targeted to inhibit cancer cells.

### SUMMARY OF INVENTION

The present invention is based on the discovery of the activities of the cellular ligand Artemin, which among other things, acts to promote the anchorage-independent growth, colony formation, survival, migration, and invasion of cancer cells. As such, Artemin and related ligands provide ideal therapeutic targets for cancer treatment. Any reagents that inhibit the biological activity of Artemin and/or these related ligands would have the potential for treating cancer, e.g., cancer onset, progression, metastasis, or recurrence.

According to the present invention there is provided an agent that inhibits the biological activity of Artemin on cancer cell proliferation, cancer cell survival, or cancer cell oncogenicity, wherein Artemin comprises an amino acid sequence selected from SEQ ID NO: 1 to 3, SEQ ID NO. 5 to 32 or 34, amino acids 40 - 220 of SEQ ID NO. 1, amino acids 57 - 237 of SEQ ID NO. 2, amino acids 48 - 228 of SEQ ID NO. 3, or an amino acid sequence encoded by the nucleotide sequence of any one of SEQ ID NO: 35 to 42, for use in the treatment of mammary carcinoma or endometrial carcinoma in a subject, wherein the agent is
a) an antisense nucleotide molecule directed to a nucleotide sequence for Artemin, or
b) an interfering RNA molecule directed to a nucleotide sequence for Artemin, or
c) an antibody molecule which binds to an amino acid sequence for Artemin, or
d) a vector expressing any one of a) to c), or
e) a host cell comprising any one of a) to d).

The agent may be an antibody molecule which binds to an amino acid sequence selected from the group consisting of SEQ ID NO:1-3, 5-32 or 34, or
a) a vector expressing a), or
b) a host cell comprising any one of a) to b).
The agent may be a monoclonal antibody which binds to an amino acid sequence selected from the group consisting of SEQ ID NO: 1-3, 5-32 or 34. The agent may be a monoclonal antibody which binds to a polynucleotide comprising the nucleic acid sequence of any one of SEQ ID NO:35-42.

According to the present invention in a further aspect there is provided a method of monitoring mammary carcinoma or endometrial carcinoma in a subject, the method comprising contacting a sample from the subject with at least one agent, and determining the level of the polynucleotide for Artemin or the level of the Artemin polypeptide in the sample, wherein an elevated level of the polynucleotide for Artemin or of the Artemin polypeptide in the sample relative to a control sample indicates a predisposition for the development of mammary carcinoma or endometrial carcinoma or the presence of mammary carcinoma or endometrial carcinoma,
and wherein the agent is
a) an antisense nucleotide molecule directed to a nucleotide sequence for Artemin, or
b) an interfering RNA molecule directed to a nucleotide sequence for Artemin, or
c) an antibody molecule which binds to an amino acid sequence for Artemin.

According to the present invention in a further aspect there is provided a antibody molecule which binds to an amino acid sequence selected from the group consisting of SEQ ID NO:1-3, 5-32 or 34, or a vector expressing said antibody molecule, or a host cell comprising said vector, for treating mammary carcinoma or endometrial carcinoma in a subject.

According to the present invention in a further aspect there is provided a method for assessing the presence of mammary carcinoma or endometrial carcinoma in a subject comprising contacting at least one polynucleotide which binds to the nucleic acid sequence of any one of SEQ ID NO:35-42 with a sample from the subject; and determining the level of the nucleic acid sequence in the sample, wherein an elevated level of the nucleic acid sequence in the sample relative to a control sample indicates the presence of mammary carcinoma or endometrial carcinoma.

According to the present invention in a further aspect there is provided a method for assessing the presence of mammary carcinoma or endometrial carcinoma in a subject comprising contacting at least one antibody molecule which binds to an amino acid sequence selected from the group consisting of SEQ ID NO:1-3, 5-32 or 34 with a sample from the subject; and determining the level of the amino acid sequence in the sample, wherein an elevated level of the amino acid sequence in the sample relative to a control sample indicates the presence of mammary carcinoma or endometrial carcinoma.

According to the present invention in a further aspect there is provided a composition comprising an agent of the disclosure, or an antibody of the disclosure, and one or more pharmaceutically acceptable diluents, carriers, and/or excipients for use in the treatment of mammary carcinoma or endometrial carcinoma in a subject. The agent may be a monoclonal antibody which binds to an amino acid sequence selected from the group consisting of SEQ ID NO: 1-3, 5-32 or 34.

Described herein is a composition comprising an isolated polynucleotide, polypeptide, or antibody for Artemin or a related ligand. In alternate aspects, the composition can comprise an expression vector, or host cell comprising an expression vector.

The composition can include any one of the biologically active modifications described herein. The composition can include at least one fusion or conjugate. The composition can be formulated, for example, as a pharmaceutical composition, as described in detail herein.

In various aspects, the methods described herein utilize *in vivo* or *in vitro* expression systems. In other aspects, the methods employ polynucleotides or polypeptides produced by recombinant, synthetic, or semi-synthetic means, or polynucleotides or polypeptides produced by endogenous means (e.g., naturally occurring components).

Other aspects and embodiments of the invention are described herein below.

### BRIEF DESCRIPTION OF THE DRAWINGS

These and other aspects of the present invention, which should be considered in all its aspects, will become apparent from the following description, which is given by way of example only, with reference to the accompanying figures:

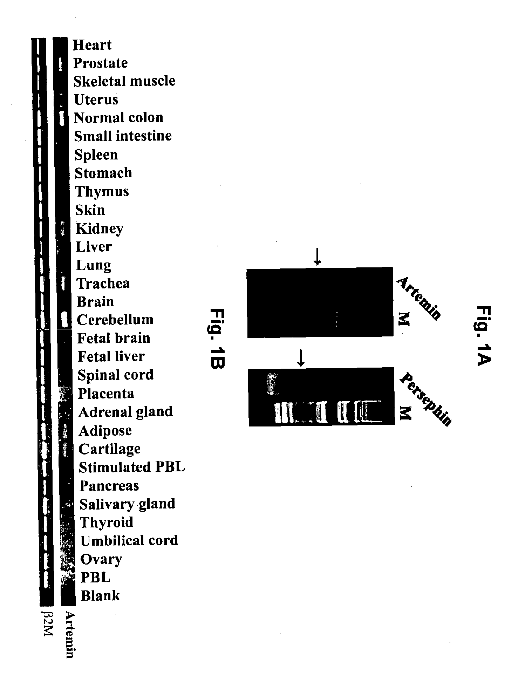

**Figs. 1A-C****. mRNA expression patterns of human Artemin in human normal tissues and carcinoma cell lines. (A)** Expression of Artemin and Persephin in mammary carcinoma MCF-7 cells. Total RNA was isolated from MCF-7 cells. The expression of Artemin and Persephin was detected by RT-PCR with specific primers using a One-Step RT-PCR Kit (QIAGEN). Arrows (→) indicate the specific bands amplified. *M*, the 1 kb plus DNA ladder (Invitrogen). **(B)** Artemin was amplified by PCR with specific primers in a panel of cDNAs made from different human tissues (Primgen). The tissue of origin is marked above each lane. The β2-microglobulin (*β2M*) gene was used as the cDNA input control. **(C)** The expression of Artemin in human cancer cell lines was examined by RT-PCR with the same Artemin specific primer pairs using a One-step RT-PCR kit (Qiagen). β-actin was included as the RNA input control. **(D)** The expression of GFRα1-4 and RET in three breast cancer cell lines by RT-PCR as in (B). The number of PCR cycles is indicated on the left side of the figure.

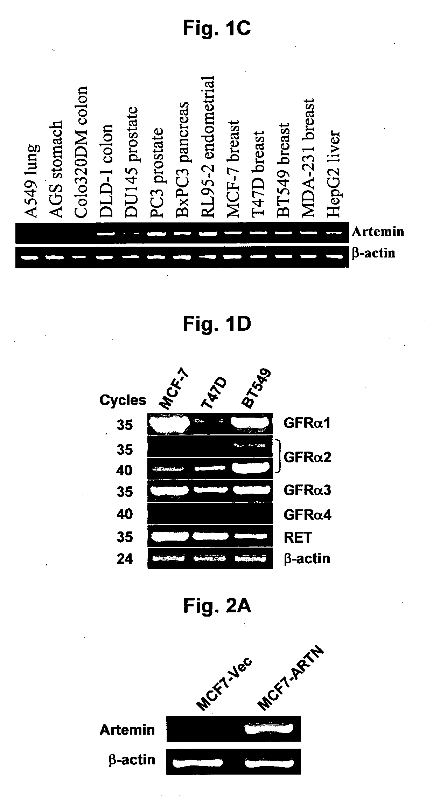

**Figs. 2A-G****. Characterisation of the effects of forced expression of Artemin in mammary carcinoma MCF-7 cells.** Cells were stably transfected with plasmids expressing Artemin (*MCF7-ARTN*) or the empty vector plasmid (*MCF7-Vec*) as a control, as indicated. **(A)** Total RNA was isolated and the mRNA levels of Artemin and β-actin were detected by RT-PCR using a One-step RT-PCR kit (Qiagen). **(B)** Western blotting analyses of forced expression and secretion of Artemin in MCF-7 cells. Soluble whole cellular extracts or concentrated conditioned media were run on a 12.5% SDS-PAGE, transferred to nitrocellulose, and immunoblotted using goat anti-Artemin polyclonal antibodies (R&D Systems). β-actin was used as loading control for cell lysates. **(C, D)** Total cell number assays. MCF7-ARTN (*ARTN*) and MCF7-Vec *(Vector)* cells were seeded at a density of 50,000 cells per well in six-well plates in triplicate in 3 ml of RPMI 1640 with (C) 10% FBS (*SERUM*) or (D) low serum (0.2%) (*SERUMFREE*)*.* Cells were cultured up to 8 days with media changed every second day. Cell number was determined after trypsinization every 2 days. **(E)** BrdU incorporation assay. MCF7-ARTN (*ARTN*) and MCF7-Vec *(Vector)* cells were seeded on glass cover slips in six-well plates in full media, and incubated overnight followed by an 18h of serum starvation in serum-free media. Cells were then pulse labelled with BrdU. Proliferating cells were detected by BrdU staining with an anti-BrdU mouse monoclonal antibody using a kit (Zymed). The cell nuclei were counterstained with hematoxylin. Cells with dark brown nuclear staining were counted as BrdU-labelled cells. The percentage of BrdU-positive cell nuclei relative to the total number of cell nuclei was calculated from 10 random fields per cover slip. **(F, G)** Apoptosis assays. MCF7-ARTN (*ARTN*) and MCF7-Vec *(Vector)* cells were seeded in six-well plates in full RPMI media containing 10% serum. One day later, the cells were serum-starved for 24h. Cells were then fixed and were stained for the assessment of apoptosis either by Hoechst 33258 dye (F) or with fluorescent isothiocyanate-conjugated Annexin V (*AV*) and propidium iodide (*PI*) (G). Nuclear morphology of Hoechst 33258 stained cells was examined under a UV-visible fluorescence microscope. Cells with apoptotic nuclear characteristics such as nuclear condensation and fragmentation were scored as apoptotic. Both early (AV-positive and PI-negative; shaded box) and late (AV- and PI-positive; open box) apoptosis were presented with the percentage. *, p<0.05; **, p<0.01.

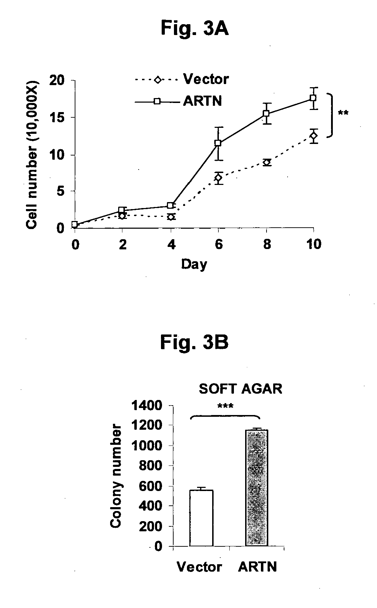

**Figs. 3A-F****. Forced overexpression of Artemin significantly promotes anchorage-independent growth and enhances cell migration and invasion. (A)** Suspension culture. MCF7-ARTN (*ARTN*) and MCF7-Vec *(Vector)* cells were placed in each well of six-well plates precoated with poly-HEMA to prevent cell attachment. At the indicated times, viable cells were counted using a hemocytometer. **(B)** Soft agar assay. Cells were seeded in 0.35% agarose in full RPMI 1640 medium containing 10% serum in six-well plates in triplicates. Colonies formed were counted after incubation of 14 days. **(C)** Representative photomicrographic pictures of colonies formed by the cells growing in Matrigel. **(D)** Phalloidin staining of MCF7-ARTN and MCF7-Vec cells. **(E)** Migration/invasion assays. Cells in serum-free media were loaded into the upper chambers of non-coated *(Migration)* or Matrigel-coated (*Invasion*) 8 µm pore size Transwell inserts which were placed into lower chambers with full media containing 10% FBS. The loaded chambers were incubated for 24h or 48h. Cells in the upper chamber were removed with cotton swabs and those that had migrated to the lower surface of the inner chamber were identified microscopically after staining with crystal violet. **(F)** Wound healing assay. Cells were grown on tissue culture Petri dishes to confluence in growth media. "Scratch" wounds were created *in vitro* by scraping the cell monolayer with a sterile pipette tip. Photographs were taken at the time indicated. **, p<0.01; ***, p<0.001.

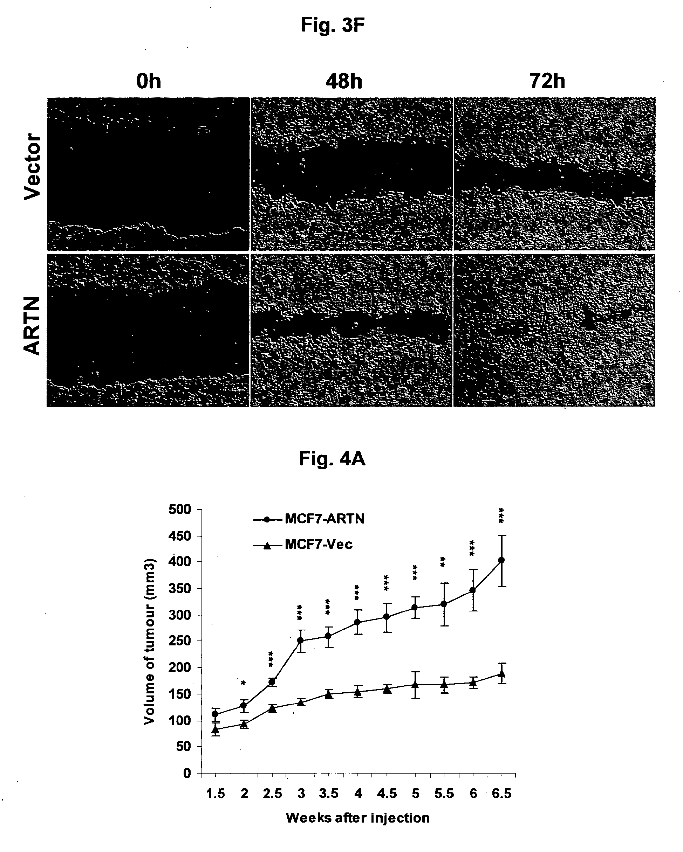

**Figs. 4A-D****. Effect of forced expression of Artemin on *in vivo* growth of MCF-7 xerographic nude mice.** Cells (5 x 10⁶) of either the MCF7-ARTN cell line overexpressing Artemin or the MCF7-Vec control cell line in Matrigel were transplanted s.c. into the flank of immunodeficient nude mice. The length and width of the tumors were measured twice a week and volumes calculated. **(A)** Tumour volume in relation to the day of surgery is shown. **(B)** Histological staining of representative tumours with hematoxylin and eosin (H&E). **(C, D)** Cell proliferation was assessed by BrdU incorporation at the end of the experiment, and apoptosis was measured by TUNEL labelling. ***,** *p* < 0.05; **, p<0.01; ***, p<0.001.

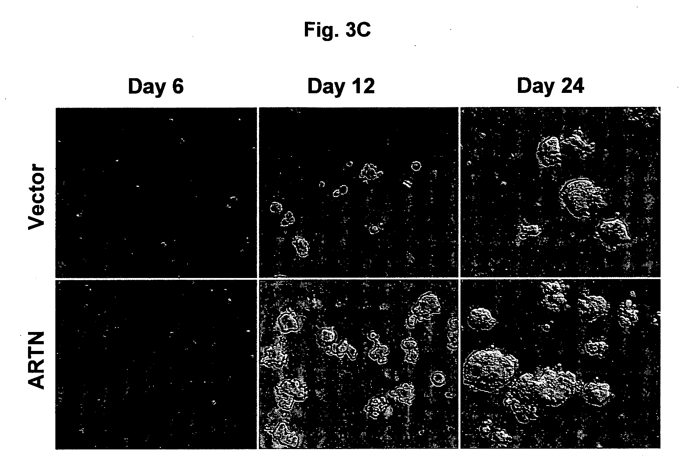

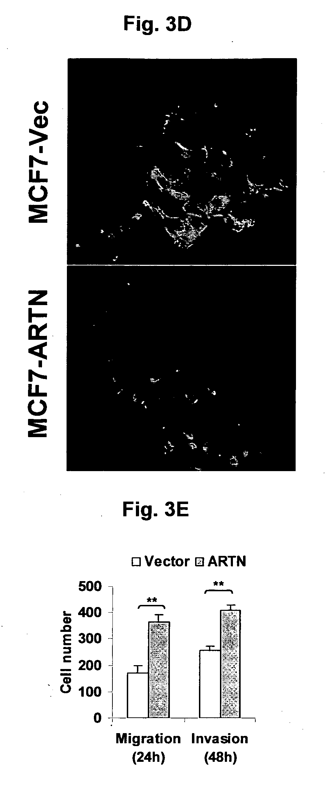

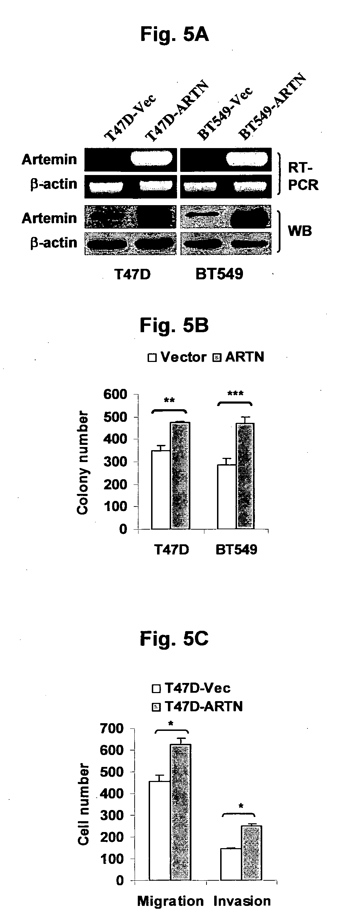

**Figs. 5A-E****. Forced overexpression of Artemin significantly enhances anchorage-independent growth, cell migration and invasion in T47D and BT549 cells.** T47D and BT549 cells were stably transfected with plasmids expressing Artemin (*T47D-ARTN* and *BT549-ARTN*) or the empty vector plasmid (*T47D-Vec* and *BT549-Vec*) as controls. The expression of Artemin in these cell lines was confirmed by RT-PCR and Western blot **(A).** Soft agar **(B),** migration and invasion (C, D) assays were performed as noted for Figure 3 with Artemin stably overexpressing T47D-ARTN and its control T47D-Vec; and BT549-ARTN and its control BT549-Vec cells. The morphology of BT549-ARTN and BT549-Vec cells is also shown **(E). *,** p<0.05; **, p<0.01; ***, p<0.001.

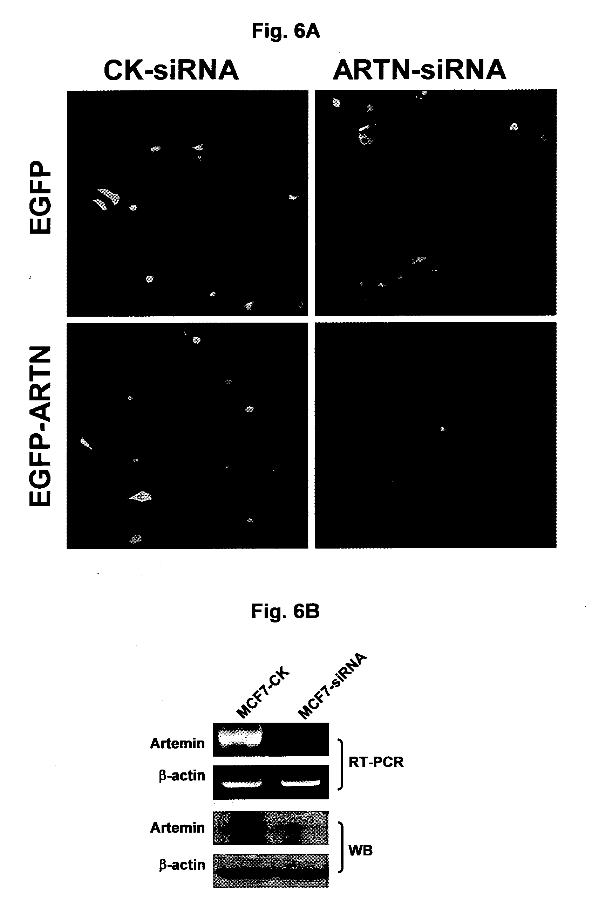

**Figs. 6A-F****. Depletion of endogenous Artemin mRNA by siRNA in MCF-7 cells decreases the survival of mammary carcinoma cell proliferation in serum starvation and significantly increases apoptotic cell death. (A)** The pSilencer-ARTN plasmid (*ARTN-siRNA*), encoding siRNA targeted to Artemin transcript, or the negative control siRNA plasmid, pSilencer-CK (*CK-siRNA*)*,* were transiently transfected into MCF-7 cells together with the pEGFP-C1 vector (*EGFP*) or the pEGFP-ARTN plasmid (*EGFP-ARTN*), in which the Artemin cDNA was inserted within the 3'UTR of EGFP coding sequence. After 24h of transfection, the expression of EGFP was visualised with a UV-visible fluorescence microscope. **(B)** RT-PCR and Western blot. MCF-7 cells were stably transfected with the pSilencer-ARTN plasmid (*MCF7-siRNA*) or with the negative control pSilencer-CK plasmid (*MCF7-CK*)*.* Total RNA was isolated and the mRNA levels of Artemin and β-actin were detected by RT-PCR using a One-step RT-PCR kit (Qiagen). Soluble whole cellular extracts were run on a 12.5% SDS-PAGE, transferred to nitrocellulose, and immunoblotted using goat anti-Artemin polyclonal antibodies (R&D Systems). β-actin was used as loading control for cell lysates. **(C, D)** Total cell number assays. MCF7-siRNA *(siRNA)* and MCF7-CK (CK) cells were seeded at a density of 50,000 cells per a well in six-well plates in triplicates in 3 ml of RPMI 1640 with (C) 10% FBS (*SERUM*) or (D) low serum (0.2%) (*SERUM FREE*)*.* Cells were cultured up to 8 days with media changed every other day. Cell number was determined after trypsinization every 2 days. **(E, F)** Apoptosis assays. Cells were seeded in six-well plates in full RPMI media containing 10% serum. One day later, the cells were serum-starved for 24h. Cells were then fixed and were stained for the assessment of apoptosis either by Hoechst 33258 dye (E) or with fluorescent isothiocyanate-conjugated Annexin V (*AV*) and propidium iodide (*PI*) (F). Nuclear morphology of Hoechst 33258 stained cells was examined under a UV-visible fluorescence microscope. Cells with apoptotic nuclear characteristics such as nuclear condensation and fragmentation were scored as apoptotic. Both early (AV-positive and PI-negative; shaded box) and late (AV- and PI-positive; open box) apoptosis were presented with the percentage. *, p<0.05; **, p<0.01.

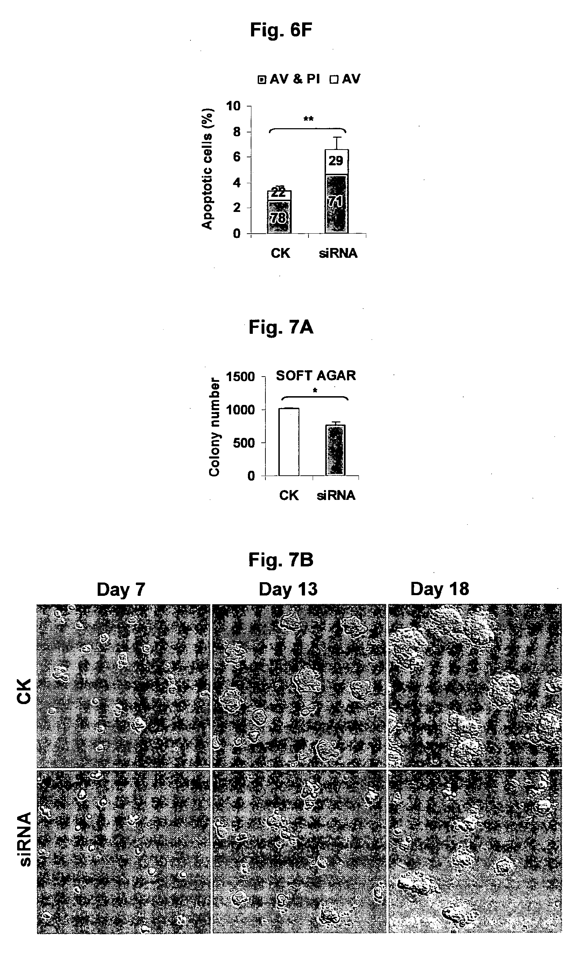

**Figs. 7A-D****. Depletion of endogenous Artemin by siRNA significantly decreases anchorage-independent growth and impairs cell migration and invasion. (A)** Soft agar assay. MCF7-siRNA *(siRNA)* and MCF7-CK (*CK*) cells were seeded in 0.35% agarose in full RPMI 1640 medium containing 10% serum in six-well plates, in triplicate. Colonies formed were counted after incubation for 14 days. **(B)** Representative photomicrographs of colonies formed by the cells growing in Matrigel. **(C)** Migration/invasion assays. Cells in serum-free media were loaded into the upper chambers of non-coated (*Migration*) or Matrigel-coated (*Invasion*) Transwell inserts, 8 µm pore size. These were placed into lower chambers with full media containing 10% FBS. The loaded chambers were incubated for 24h. Cells in the upper chamber were removed with cotton swabs and those that had migrated to the lower surface of the inner chamber were identified microscopically after staining with crystal violet. **(D)** Wound healing assays. Cells were grown on tissue culture Petri dishes to confluence in growth media. "Scratch" wounds were created *in vitro* by scraping the cell monolayer with a sterile pipette tip. Photographs were taken at the time indicated. *, p<0.05; **, p<0.01.

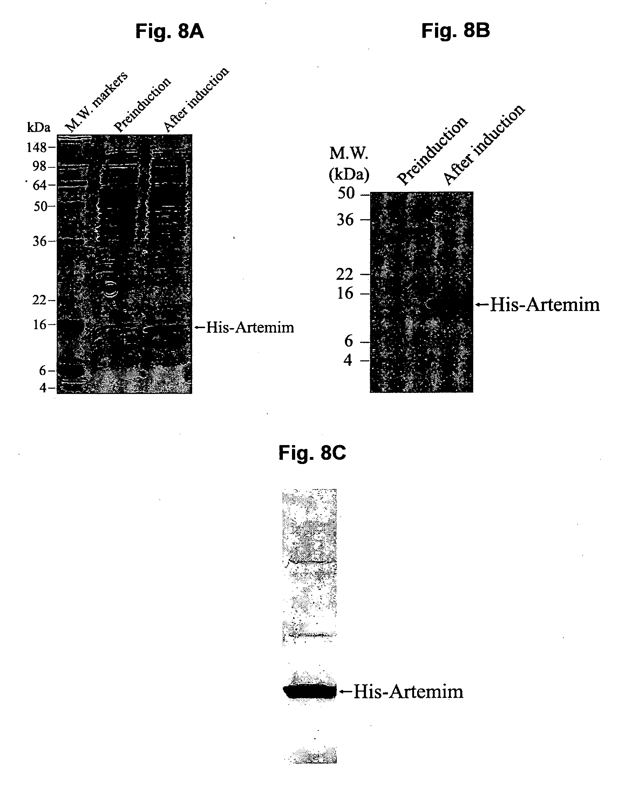

**Figs. 8A-C****. Production of recombinant human Artemin protein in bacteria. (A).** The pQE30-ARTN plasmid was transformed into bacteria and the expression of His-Artemin was induced by addition of IPTG. Whole cell lysates, pre- and post-induction, were analysed by SDS-PAGE and visualised by Coomassie blue staining. **(B)** Detection of His-Artemin in the whole cell lysates, pre- and post-induction, by Western blotting with goat anti-Artemin polyclonal antibodies (R&D Systems). **(C)** The His-Artemin protein was purified from the lysate using Ni-NTA (Qiagen) and was analyzed by SDS-PAGE and visualised by Coomassie blue staining. Arrows indicate the 13.5 kDa band of His-Artemin. Molecular mass markers are also indicated in kDa by bars on the left side of the figure.

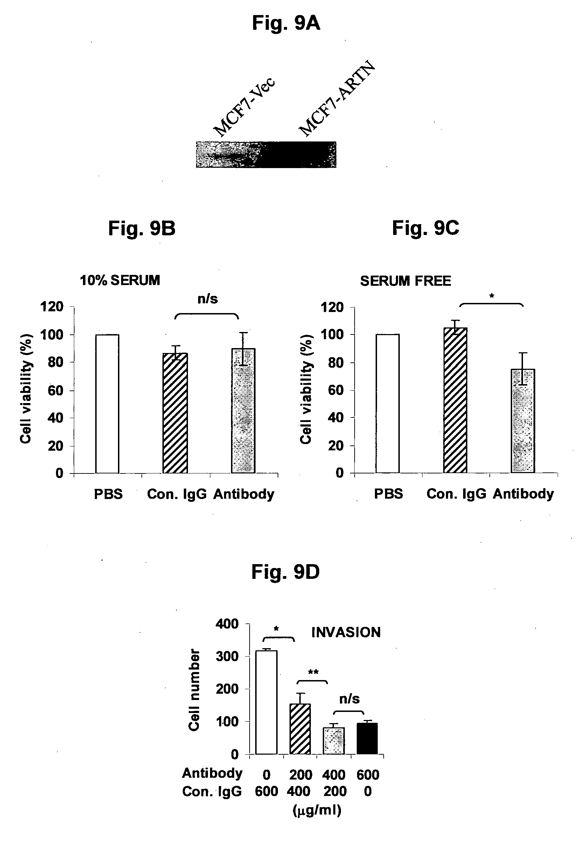

**Figs. 9A-D****. Rabbit polyclonal antibodies against Artemin reduce viability and inhibit invasion of MCF-7** cells. **(A)** Characterization of the antiserum. Artemin expressing MCF7-ARTN and the control MCF7-Vec stable cells were subjected to Western blot analyses using rabbit polyclonal antibodies. **(B,** C) Cell viability was assessed by colorimetric MTT assay using the procedure described herein below, using 500 µg/ml of rabbit polyclonal antibodies (*Antibody*) or rabbit control IgG (*Con. IgG).* Cell viability was presented relative to treatment without antibody (*PBS*)*.* (D) Inhibition of invasion of MCF-7 cells by rabbit polyclonal antiserum. *, p<0.05; **, p<0.01.

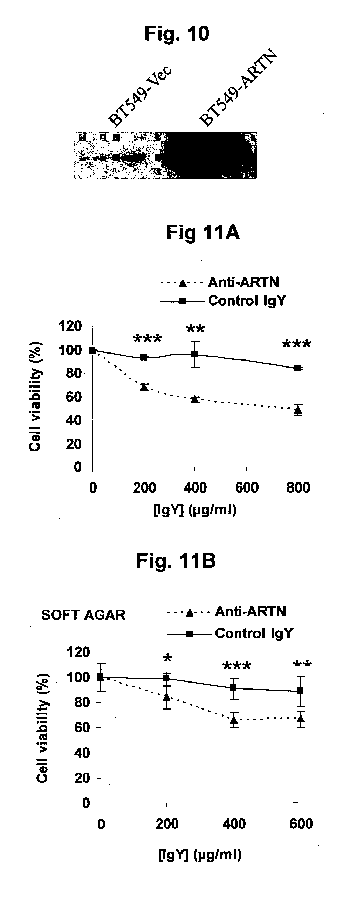

**Fig. 10****. Characterization of chicken polyclonal antibodies against Artemin. Artemin** overexpressing BT549-ARTN and the control BT549-Vec stable cells were subjected to Western blot analyses using chicken polyclonal antibodies against Artemin.
**Figs. 11A-C****. Chicken polyclonal antibodies against Artemin reduce cell viability and anchorage-independent growth and inhibit invasion of MCF-7 cells. (A)** Cell viability of cells grown in media containing low serum (0.2%) for 3 days was assessed by colorimetric MTT assay using the procedure described herein below using chicken polyclonal antibodies against Artemin (*Anti-ARTN*) or the preimmune chicken control IgY at the concentrations indicated. Cell viability was presented relative to the PBS treatment without antibody. **(B)** Soft agar colony formation assay was performed in 96-well plates with MCF-7 cells in the presence of chicken polyclonal antibodies against Artemin (*Anti*-*ARTN*) or the preimmune control IgY at the concentrations indicated. The viability of cells was then assessed by colorimetric MTT assay using the procedure described herein below, and presented as the percentage relative to the PBS control without any treatments. (C) Inhibition of invasion of MCF-7 cells by chicken polyclonal antibodies against Artemin. *, p<0.05; **, p<0.01; ***, p<0.001.

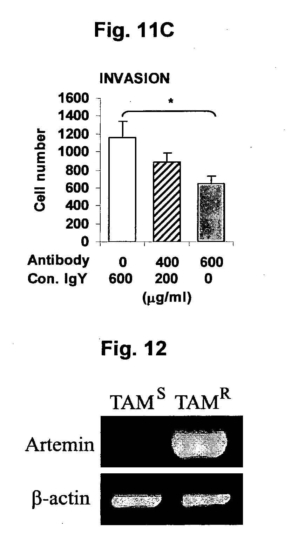

**Fig. 12****. Up-regulation of Artemin expression in Tamoxifen-resistant MCF-7 cells.** MCF7 cells are normally Tamoxifen sensitive (*TAM^{S}*) After progressive exposure to increasing concentrations of the drug, Tamoxifen-resistant (*TAM^{R}*) MCF-7 cells were established. The expression of Artemin was determined by RT-PCR with β-action as the total RNA input control.

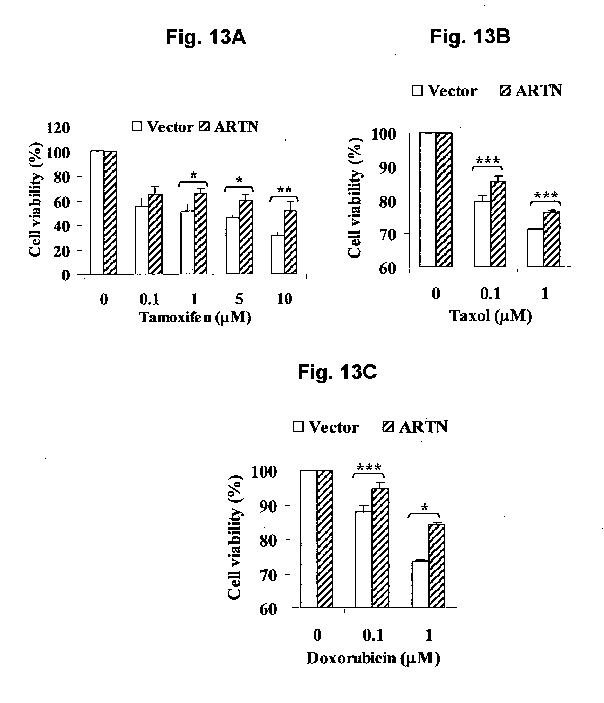

**Figs. 13A-C****. Forced overexpression of Artemin significantly abrogates chemotherapy drug induced death.** MCF7-ARTN (*ARTN*) and MCF7-Vec *(Vector)* cells were seeded at a density of 10,000 cells per a well in 96 wells microplates, in triplicate, in RPMI 1640 media containing either 10% serum, in the presence or absence of Tamoxifen **(A),** Doxorubicin **(B)** or Taxol **(C)** at indicated concentrations. Cell viability was assessed by colorimetric MTT assay using the procedure described herein below, relative to treatment without drug. *, p<0.05; **, p<0.01; ***, p<0.001.

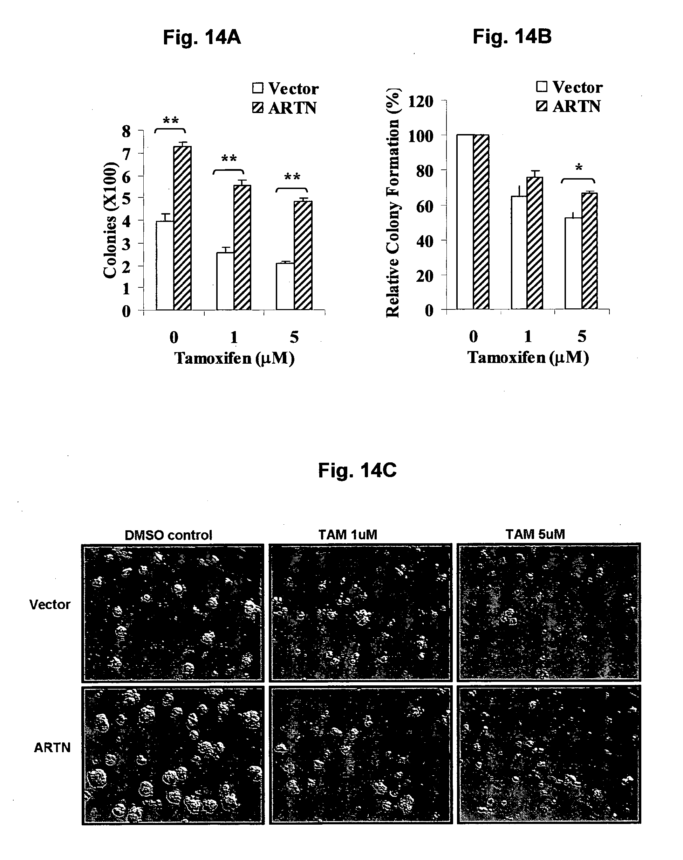

**Figs. 14A-C****. Forced overexpression of Artemin significantly increases anchorage-independent growth in the presence of Tamoxifen.** Soft agar assays were performed with MCF7-ARTN (*ARTN*) and MCF7-Vec *(Vector)* cells by seeding cells in 0.35% agarose in full RPMI 1640 medium with 10% serum in the presence of Tamoxifen in six-well plates, at the concentrations indicated, in triplicate. Colonies formed were counted after incubation for 14 days and presented in **(A).** The relative colony formation was presented in **(B),** with number of colonies formed in the absence of Tamoxifen set as 100%. (C) 3D-structure of colonies formed in Matrigel in the presence or absence of Tamoxifen at indicated concentrations. *, p<0.05; **, p<0.01.

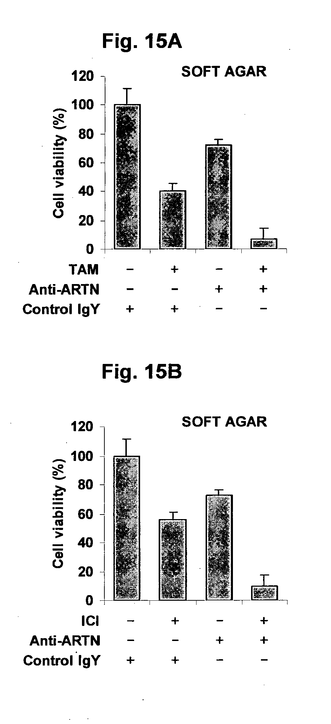

**Figs. 15A-B****. Synergistic inhibition of anchorage-independent growth of MCF-7 cells by chicken polyclonal antibodies against Artemin combined with anti-estrogens.** Soft agar colony formation assays were performed in 96-well plates with MCF-7 cells in the presence or absence of anti-estrogenic Tamoxifen (*TAM*) at a concentration 1µM **(A)** or Faslodex (ICI 182,780) (*ICI*) at a concentration of 100 nM **(B)** alone or together with chicken polyclonal antibodies against Artemin (*Anti-ARTN*) or the preimmune control IgY both at a concentration of 400 µg/ml. The viability of cells was assessed by colorimetric MTT assay using the procedure described herein below, and presented as the percentage relative to treatment with the control IgY.

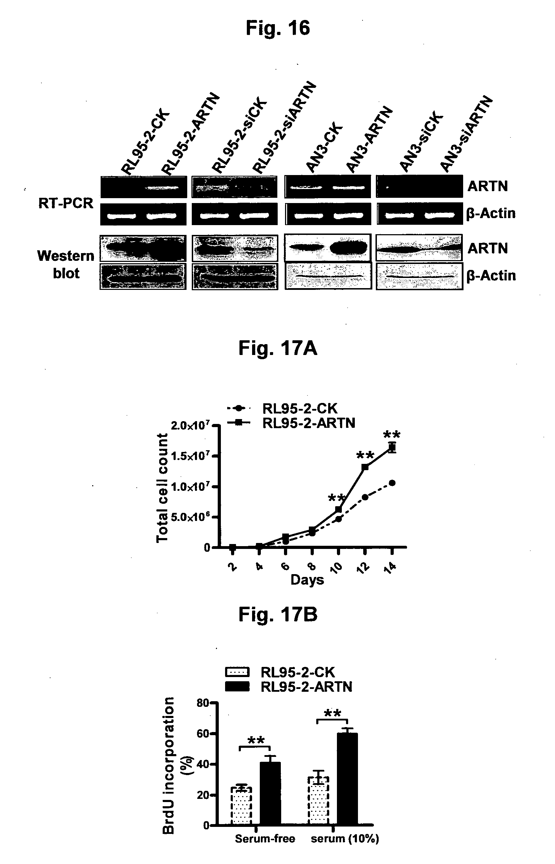

**Fig. 16****. Characterization of endometrial carcinoma cell lines with increased or decreased expression of Artemin.** To force the expression of Artemin, RL95-2 and AN3 cells were stably transfected with either Artemin expressing plasmid, pIRESneo3-ARTN (cells designated RL95-2-ARTN & AN3-ARTN, respectively), or the empty pIRESneo3 vector as control (cells designated as RL95-2-CK & AN3-CK). To deplete the endogenous Artemin expression, cells were stably transfected with the pSilencer-ARTN plasmid (cells designated as RL95-2-siARTN & AN3-siARTN) or with the negative control pSilencer-CK plasmid (cells designated as RL95-2-siCK & AN3-siCK). The expression of Artemin (*ARTN*) was determined at mRNA level by RT-PCR and at protein level by Western blot as indicated. β-actin was used as the control.
**Figs. 17A-E****. Forced expression of Artemin increases cell proliferation and decreases apoptosis of human endometrial carcinoma RL95-2 cells. (A)** Total cell number assays. RL95-2-ARTN cells overexpressing Artemin as well as RL95-2-CK control cells were seeded at a density of 50,000 cells per well in six-well plates, in triplicate, in 3 ml of full growth media 10% FBS. Cells were cultured up to 14 days, with media changed every second day. Cell numbers were determined after trypsinization every 2 days. **(B)** BrdU incorporation assay. Cells were seeded on glass cover slips in six-well plates in serum-free media or in full media containing 10% serum (FBS), and incubated overnight followed by an 18h of serum starvation in serum-free media. Cells were then pulse labelled with BrdU. Proliferating cells were detected by BrdU staining with an anti-BrdU mouse monoclonal antibody. (C) Apoptosis assays. Cells were seeded in six-well plates in full growth media containing 10 % serum. One day later, the cells were serum-starved for 48h. Cells were then fixed and stained with fluorescent isothiocyanate-conjugated Annexin V (*AV*) and propidium iodide (*PI*). Both early (AV-positive and PI-negative; shaded bar) and late (AV- and PI-positive; solid bar) apoptosis were presented. **(D, E)** Real-time PCR. The relative expression levels of several key markers involved in cell cycle control & DNA damage repair **(D),** and apoptosis and cell senescence **(E)** were determined by real-time PCR. Relative expression level in RL95-2-ARTN cells relative to the control RL95-2-CK cells was presented, with fold expression. ***, *p*<0.05; **, *p*<0.001.

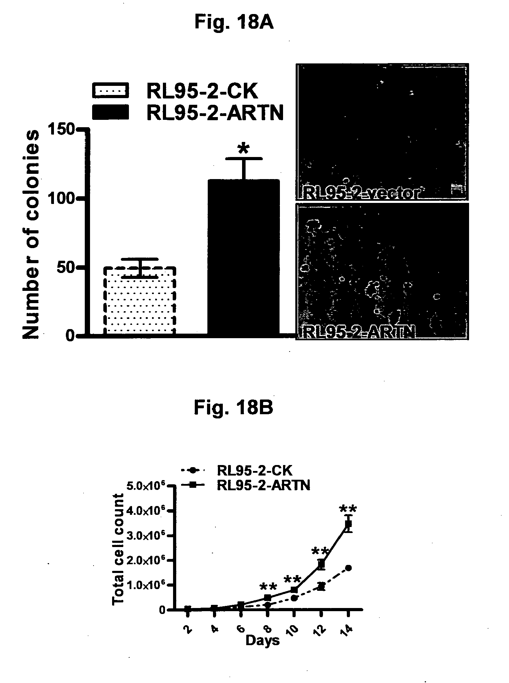

**Figs. 18A-E****. Forced expression of Artemin enhances anchorage-independent growth of human endometrial carcinoma RL95-2 cells. (A)** Soft agar assay. RL95-2-ARTN cells overexpressing Artemin as well as RL95-2-CK control cells were seeded in 0.35% agarose in full medium containing 10% serum in six-well plates, in triplicate. Colonies formed were counted after incubation for 14 days. Representative photomicrographic pictures of colonies formed by the cells growing are shown on the right. **(B)** Suspension culture. Cells were placed in each well of six-well plates precoated with poly-HEMA to prevent cell attachment. At the indicated times, viable cells were counted using a hemocytometer. **(C, D)** Colony morphology. Cells were grown in growth factor reduced Matrigel (C) or on plates coated with Matrigel (D). Phase contrast microscopic graphs were taken at indicated time after plating. **(E)** Focus-Formation Assay. Cells were grown in full medium containing 10% serum and stained with 0.2% crystal violet in 70% ethanol and photographed. *, *p*<0.05; **, *p*<0.001.

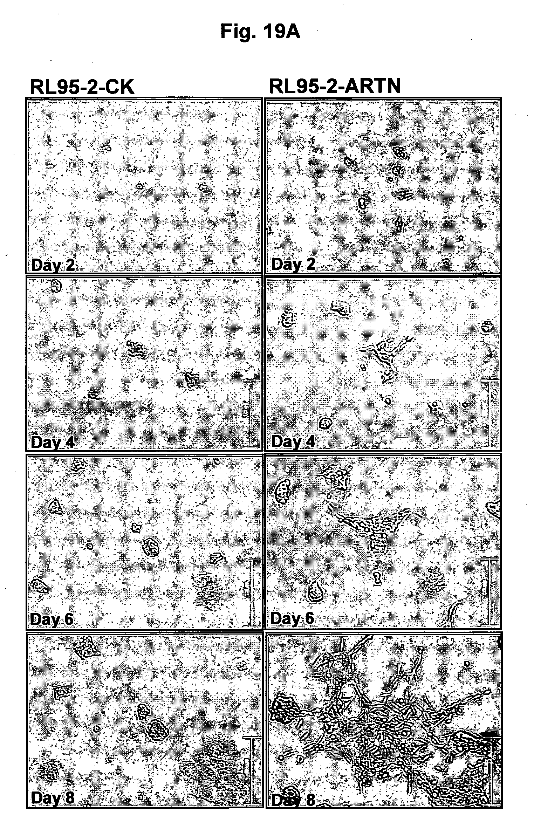

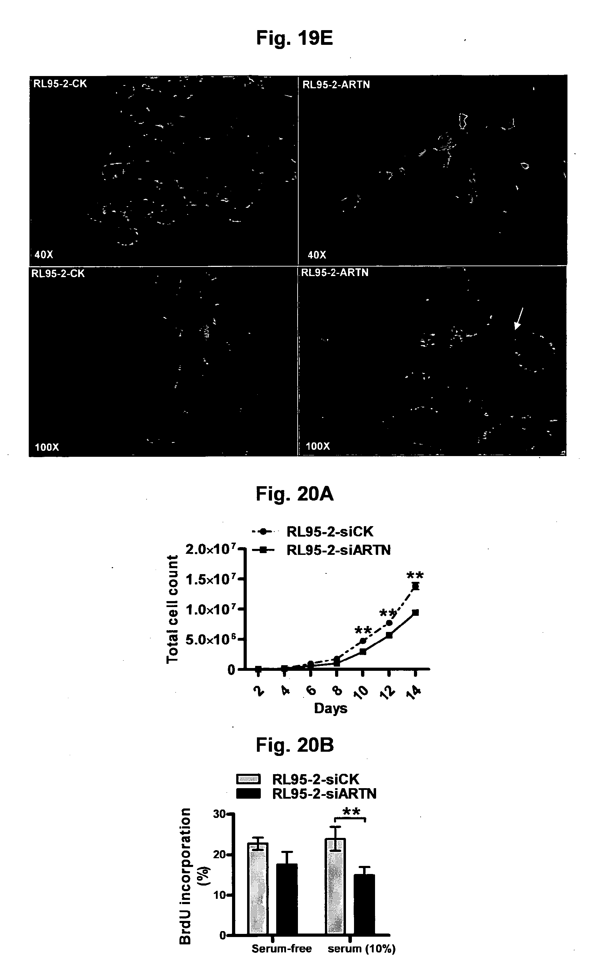

**Figs. 19A-E****. Forced expression of Artemin enhances migration and invasion of human endometrial carcinoma RL95-2 cells. (A)** RL95-2-ARTN cells overexpressing Artemin as well as RL95-2-CK control cells were cultured in tissue-culture treated plastic Petri-dishes in full media with 10% FBS. Cell growth was monitored and photographed at the times indicated. **(B)** Migration/invasion assays. Cells in serum-free media were loaded into the upper chambers of non-coated (*Migration*) or Matrigel-coated (*Invasion*) Transwell inserts, 8 µm pore size. These were placed into lower chambers with full media containing 10% FBS. The loaded chambers were incubated for 24h. Cells in the upper chamber were removed with cotton swabs and those that had migrated to the lower surface of the inner chamber were identified microscopically after staining with crystal violet. **(C, D)** Real-time PCR. The relative expression levels of several key markers involved in invasion and metastasis (C), and cell re-modeling (D) were determined by real-time PCR. The expression level in RL95-2-ARTN cells relative to RL95-2-CK control cells was presented. **(E)** Cells were stained with Tetramethylrhodamine B isothiocyanate (TRITC)-conjugated phalloidin (*Green*) and nuclei were counter-stained with DAPI (*blue*). Formation of lamellipodia (red arrow) and filopodia (white arrow) were indicated. *, *p*<0.05; **, *p*<0.001.
**Figs. 20A-E****. Depletion of endogenous Artemin by siRNA impairs cell oncogenic potential of human endometrial carcinoma RL95-2 cells. (A)** Total cell number assays. Artemin-depleted RL95-2-siARTN cells as well as control RL95-2-siCK cells were seeded at a density of 50,000 cells per well in six-well plates, in triplicate, in 3 ml of full growth media 10% FBS. Cells were cultured up to 14 days with media changed every second day. Total cell number was determined after trypsinization every 2 days. (B) BrdU incorporation assay. Cells were seeded on glass cover slips in six-well plates in serum-free media or in full media containing 10% serum (FBS), and incubated overnight followed by an 18h of serum starvation in serum-free media. Cells were then pulse labelled with BrdU. Proliferating cells were detected by BrdU staining with an anti-BrdU mouse monoclonal antibody. (C) Apoptosis assays. Cells were seeded in six-well plates in full growth media containing 10% serum. One day later, the cells were serum-starved for 48h. Cells were then fixed and were stained for the assessment of apoptosis with fluorescent isothiocyanate-conjugated Annexin V (*AV*) and propidium iodide (*PI*). Both early (AV-positive and PI-negative; shaded bar) and late (AV- and PI-positive; solid bar) apoptosis were presented. **(D)** Soft agar assay. Cells were seeded in 0.35% agarose in full medium containing 10% serum in six-well plates in triplicates. Colonies formed were counted after incubation of 14 days. **(E)** Migration/invasion assays. Cells in serum-free media were loaded into the upper chambers of non-coated (*Migration*) or Matrigel-coated *(Invasion)* Transwell inserts, 8 µm pore size. These were placed into lower chambers with full media containing 10% FBS. The loaded chambers were incubated for 24h. Cells in the upper chamber were removed with cotton swabs and those that had migrated to the lower surface of the inner chamber were identified microscopically after staining with crystal violet. *, *p*<0.05; **, *p*<0.001.

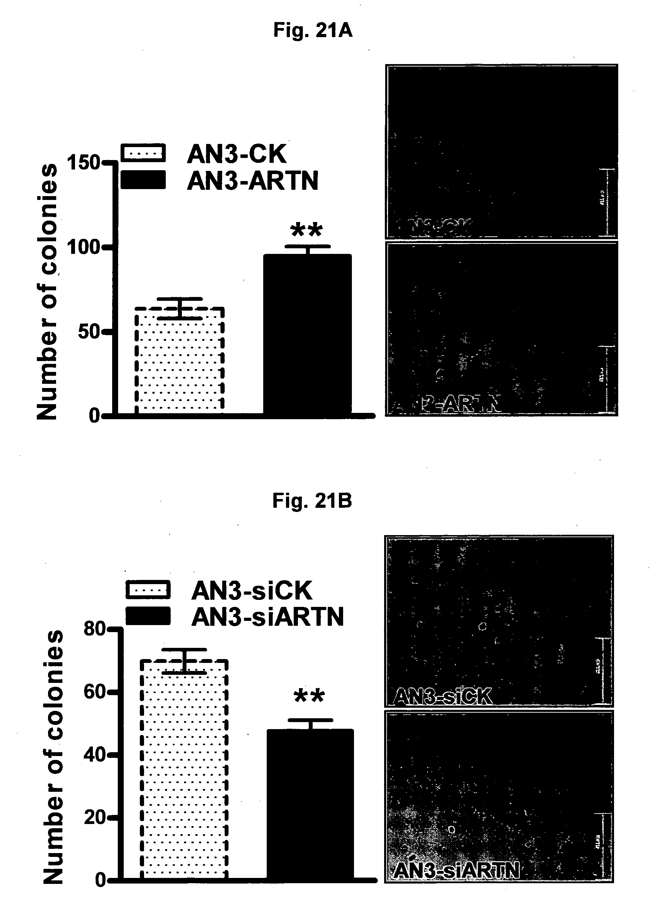

**Figs. 21A-D****. Artemin expression significantly correlates to anchorage-independent growth as well** as **cell migration and invasion in endometrial carcinoma AN3 cells. (A, B)** Soft agar assay. AN3-ARTN cells overexpressing Artemin as well as AN3-CK control cells (A), or Artemin-depleted AN3-siARTN cells as well as control AN3-siCK cells, were seeded in 0.35% agarose in full medium containing 10% serum in six-well plates, in triplicate. Colonies formed were counted after incubation for 14 days. Representative photomicrographic pictures of colonies formed by the cells growing are shown on the right. **(C, D)** Migration/invasion assays. Cells in serum-free media were loaded into the upper chambers of non-coated (*Migration*) or Matrigel-coated *(Invasion)* Transwell inserts, 8 µm pore size. These were placed into lower chambers with full media containing 10% FBS. The loaded chambers were incubated for 24h. Cells in the upper chamber were removed with cotton swabs and those that had migrated to the lower surface of the inner chamber were identified microscopically after staining with crystal violet. *, *p*<0.05; **, *p*<0.001.

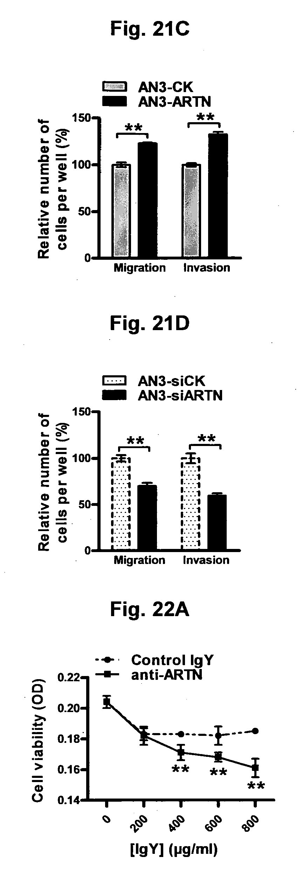

**Figs. 22A-C****. Chicken polyclonal antibodies against Artemin reduce cell viability and anchorage-independent growth and inhibit cell invasion of endometrial carcinoma cells. (A)** Cell viability of RL95-2 cells grown in media containing low serum (0.2%) for 3 days was assessed by colorimetric MTT assay using the procedure described herein below using chicken polyclonal antibodies against Artemin (*Anti-ARTN*) or the preimmune chicken control IgY at the concentrations indicated. Cell viability was presented relative to treatment without antibody. **(B)** Soft agar colony formation assays were performed in 96-well plates with RL95-2 and AN3 cells in the presence of 400 µg/ml of chicken polyclonal antibodies against Artemin (*Anti-ARTN*) or the preimmune control IgY. The viability of cells was then assessed by colorimetric MTT assay using the procedure described herein below, and presented as the percentage relative to the control without any treatment (*PBS*)*.* **(C)** Inhibition of invasion of MCF-7 cells by chicken polyclonal antibodies against Artemin. **, p<0.01.

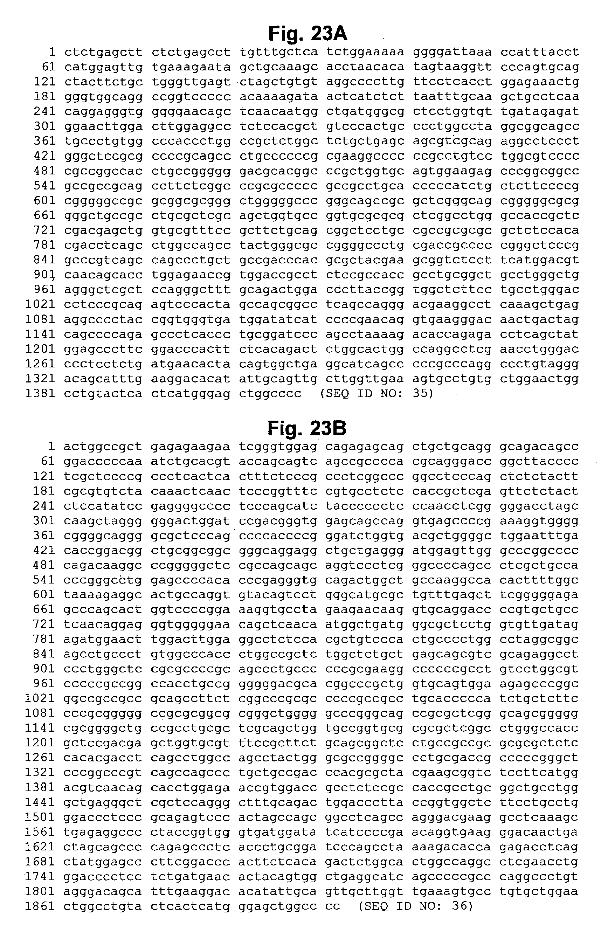

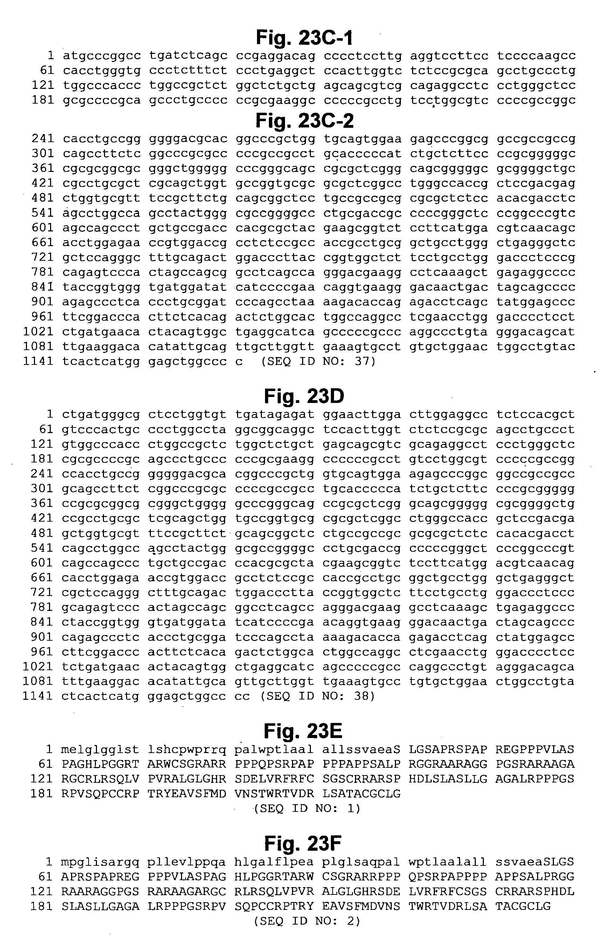

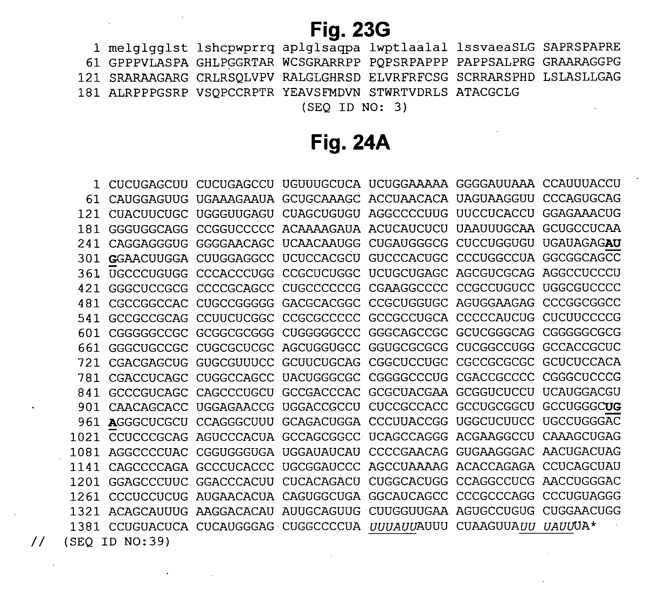

**Figs. 23A-G****. Polynucleotide and polypeptide sequence information for Artemin. (A)** Nucleotide sequence of human Artemin (ARTN) transcript variant 1 cDNA (GenBank accession #: NM_003976). **(B)** Nucleotide sequence of human Artemin (ARTN) transcript variant 2 cDNA (GenBank accession #: NM_057091). **(C)** Nucleotide sequence of human Artemin (ARTN) transcript variant 3 cDNA (GenBank accession #: NM_057160). **(D)** Nucleotide sequence of human Artemin (ARTN) transcript variant 3 cDNA (GenBank accession #: NM_057090). **(E)** Amino acid sequence of human Artemin (ARTN) isoform 1 precursor encoded by transcript 1 and 2 (Entrez Protein IDs: NP_003967 and NP_476432) with signal leading peptides in lower case. **(F)** Amino acid sequence of human Artemin (ARTN) isoform 2 precursor encoded by transcript 3 (Entrez Protein ID: NP_476501) with signal leading peptides in lower case. **(G)** Amino acid sequence of human Artemin (ARTN) isoform 3 precursor encoded by transcript 4 (Entrez Protein ID: NP_476431) with signal leading peptides in lower case.

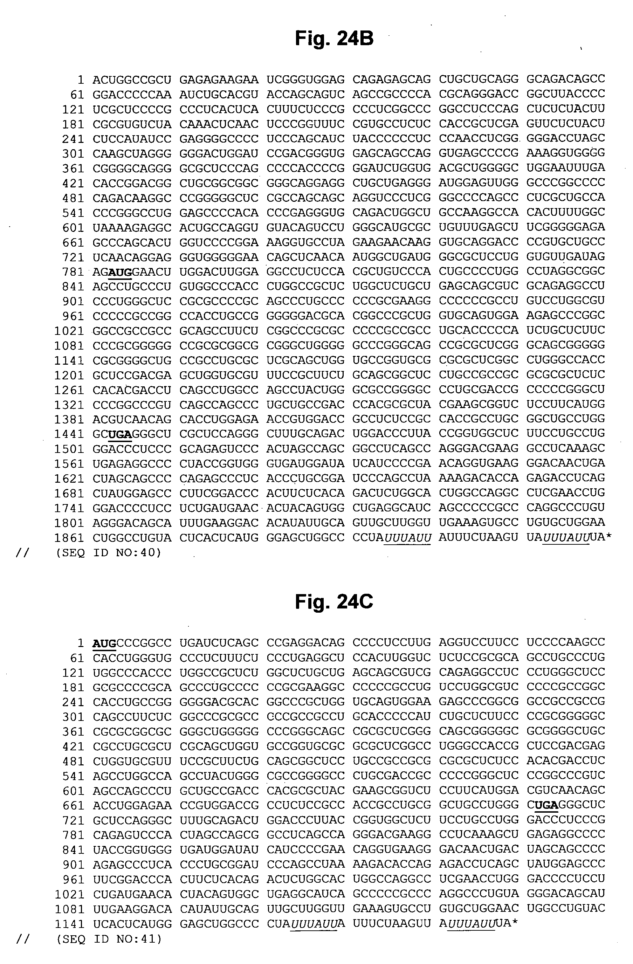

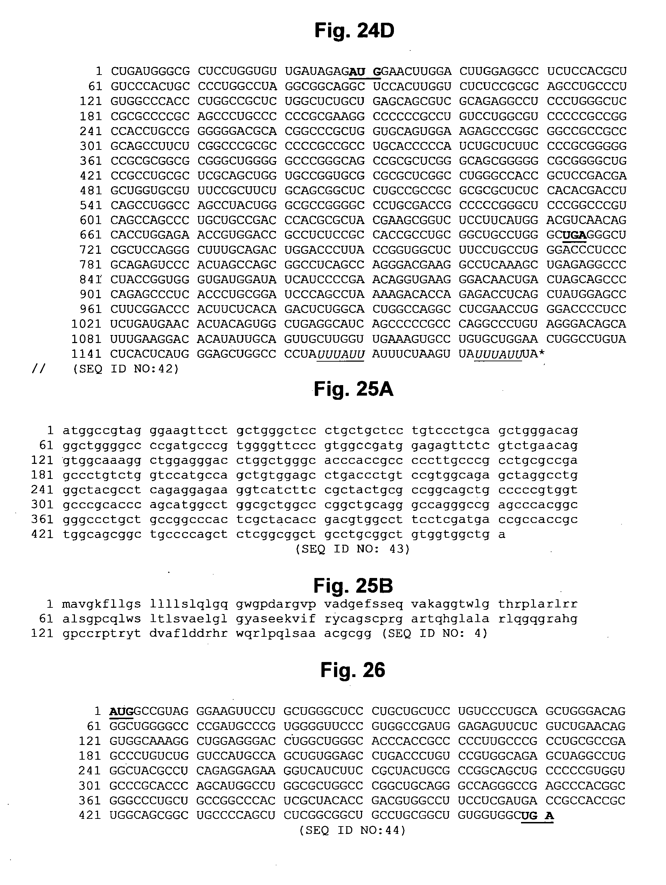

**Figs. 24A-D****. Nucleotide sequence of human Artemin mRNA transcripts.** The sequences were deduced from cloned cDNA (e.g. GenBank accession #: BC062375) and genomic sequence. **(A)** Transcript variant 1 cDNA (GenBank accession #: NM_003976). (B) Transcript variant 2 cDNA (GenBank accession #: M_057091). **(C)** Transcript variant 3 cDNA (GenBank accession #: NM_057160). **(D)** Transcript variant 3 cDNA (GenBank accession #: NM_057090). The initiation codon AUG and the termination codon UGA are underlined and in bold. Potential polyadenylation signal, UUUAUU, which is the complementary sequence of AAUAAA, is also underlined and in italics. * indicates the possible position of poly(A) tails.
**Fig. 25A-B****. Polynucleotide and polypeptide sequence information for Persephin. (A)** Nucleotides sequence of human Persephin (PSPN) mRNA (GenBank accession #: NM_004158). **(B)** Amino acid sequence of human Persephin (PSPN) precursor (Entrez Protein ID: NP_004149).
**Fig. 26****. Nucleotide sequence of human Persiphin mRNA transcript.** The sequence was deduced from cloned cDNA (GenBank accession #: NM_004558). The initiation codon AUG and the termination codon UGA are underlined and in bold.

### DETAILED DESCRIPTION

The following is a description of the present invention, including preferred embodiments thereof, given in general terms. The invention is further elucidated from the disclosure given under the section "Examples" which provides experimental data supporting the invention and specific examples thereof.

### Definitions

The term "antibody" should be understood in the broadest possible sense and is intended to include intact monoclonal antibodies and polyclonal antibodies. It is also intended to cover modified antibodies so long as they exhibit the desired biological activity. Antibodies encompass immunoglobulin molecules and immunologically active portions of immunoglobulin (lg) molecules, i.e., molecules that contain an antigen binding site that specifically binds (immunoreacts with) an antigen. These include, but are not limited to, polyclonal, monoclonal, chimeric, single chain, Fc, Fab, Fab', and Fab₂ fragments, and a Fab expression library. Antibody molecules relate to any of the classes IgG, IgM, IgA, IgE, and IgD, which differ from one another by the nature of heavy chain present in the molecule. These include subclasses as well, such as IgG1, IgG2, and others. The light chain may be a kappa chain or a lambda chain. Reference herein to antibodies includes a reference to all classes, subclasses, and types. Also included are chimeric antibodies, for example, monoclonal antibodies or modifications thereof that are specific to more than one source, e.g., a mouse or human sequence. Further included are camelid antibodies or nanobodies. It will be understood that each reference to "antibodies" or any like term, herein includes intact antibodies, as well as any modifications thereof.

The term "antisense" should be interpreted broadly. It is intended to mean any nucleic acid (preferably RNA, but including single stranded DNA) capable of binding to a transcript for Artemin and/or a related ligand. It includes oligonucleotides and their derivatives (as well as salts thereof where salt-forming groups are present) that are specifically hybridizable with DNA or RNA, preferably mRNA. Such an oligonucleotide or oligonucleotide derivative can comprise nucleotide units or nucleotide analogues/derivatives thereof sufficient in number and identity to allow such hybridization. In most cases, antisense oligonucleotides and their derivatives specifically bind (hybridize) to the complementary sequence of DNA, pre-mRNA or mature mRNA, as defined by Watson-Crick base pairing.

"Altered" polynucleotides, as used herein, include those with deletions, insertions, or substitutions of different nucleotides resulting in polynucleotides that encode the same or functionally equivalent. The polypeptides and antibodies may also be "altered" and contain deletions, insertions, or substitutions of amino acid residues which produce a silent change and result in functionally equivalent sequences. Deliberate amino acid substitutions may be made on the basis of similarity in polarity, charge, solubility, hydrophobicity, hydrophilicity, and/or the amphipathic nature of the residues as long as at least one biological activity (e.g., effect on cell proliferation, cell survival, cell motility, and/or oncogenicity) or immunogenic/immunological activity of the sequence is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine. Guidance in making substitutions and/or deletions or additions can be obtained, for example, by sequence comparisons to homologues, orthologues, or paralogues, as shown in the figures, herein.

The term "antigen binding site" or "antigen binding portion" refers to the part of the immunoglobulin molecule, or fragment, or modification thereof, that participates in antigen interaction. The antigen binding site is generally formed by amino acid residues of the N-terminal variable ("V") regions of the heavy ("H") and light ("L") chains. Three highly divergent stretches within the V regions of the heavy and light chains, referred to as "hypervariable regions," are interposed between more conserved flanking stretches known as "framework regions". Thus, the term "framework region" or "FR" refers to amino acid sequences which are naturally found between, and adjacent to, hypervariable regions in immunoglobulins. In an antibody molecule, the three hypervariable regions of a light chain and the three hypervariable regions of a heavy chain are disposed relative to each other in three dimensional space to form an antigen binding surface. The antigen binding surface is generally complementary to the three-dimensional surface of a bound antigen, and the three hypervariable regions of each of the heavy and light chains are referred to as "complementarity-determining regions," or "CDRs." The assignment of amino acids to each domain is in accordance with accepted definitions (see, e.g., Kabat Sequences of Proteins of Immunological Interest, National Institutes of Health, Bethesda, MD, 1987 and 1991; and Chothia &Lesk J. Mol. Biol. 196:901-917, 1987, Chothia et al. Nature 342:878-883, 1989).

"Amino acid sequence", as used herein, refers to a sequence of an oligopeptide, peptide, polypeptide, protein, or antibody, and any fragment thereof, and to any naturally occurring, recombinant, synthetic, or semi-synthetic molecules. The sequences comprise at least 5, 6, 7, 8, 9, 10, 11, or 12 amino acids, preferably at least 5 to 10, 5 to 15, 10 to 15, or 12 to 15 amino acids. Preferably, the sequences retain the biological activity (e.g., effect on cell proliferation, cell survival, cell motility, and/or oncogenicity) or the immunogenicity/immunological activity of the original amino acid sequence. It will be understood that "amino acid sequence" and like terms are not limited to the complete, original sequence associated with the full-length molecule, but include also any modifications thereof.

"Artemin and related ligands" as used herein refers to GDNF family ligands (GFL), including Artemin (ARTN) and Persephin (PSPN), which can include similar sequences and/or activities, including, but not limited to Artemin (ARTN) transcript variant 1, Artemin (ARTN) transcript variant 2, Artemin (ARTN) transcript variant 3, Artemin (ARTN) transcript variant 3, Artemin (ARTN) isoform 1 precursor, Artemin (ARTN) isoform 2 precursor, Artemin (ARTN) isoform 3 precursor, Persephin (PSPN), and Persephin (PSPN) precursor. For use with the invention, human sequences are preferred, but other homologs and orthologs can also be used. It will be understood that each reference to these factors (e.g., Artemin (ARTN) and related ligands, such as Persephin (PSPN), or like terms), herein, will include the full length sequences as well as any fragments, or modifications (including variants) thereof.

The terms "cancer" and "cancerous" refer to a physiological condition in mammals that is typically characterized by abnormal or unregulated cell proliferation, cell survival, cell motility, and/or oncogenicity. Cancer and cancer pathology can be associated, for example, with metastasis, interference with the normal functioning of neighbouring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc. Specifically included are cancers and precancerous conditions, for example, breast cancers, which can include epithelial tumours, nonepithelial tumours, carcinomas, for example, carcinomas *in situ,* as well as invasive breast cancers. Also included are colon cancer, prostate cancer, endometrial cancer, lung cancer, stomach cancer, and liver cancer, among others.

The terms "complementary" or "complementarity," as used herein, refer to the natural binding of polynucleotides under permissive salt and temperature conditions by base-pairing. For the sequence A-G-T, the complementary sequence is T-C-A, the reverse complement is A-C-T, and the reverse sequence is T-G-A. Complementarity between two single stranded molecules may be partial, in which only some of the nucleic acids bind, or it may be complete when total complementarity exists between the single stranded molecules. The degree of complementarity between nucleic acid strands has significant effects on the efficiency and strength of hybridization between nucleic acid strands. This is of particular importance in amplification reactions, which depend upon binding between nucleic acids strands and in the design and use of iRNAs and PNAs.

The term "derivative," as used herein, refers to the chemical modification of a polynucleotide, or a polynucleotide complementary thereto. Such modifications include, for example, replacement of hydrogen by an alkyl, acyl, or amino group. In preferred aspects, a polynucleotide derivative encodes a polypeptide which retains the biological or immunological function of the natural molecule. A derivative polypeptide or antibody is one which is modified by glycosylation, pegylation, or any similar process which retains one or more biological functions (e.g., effect on cell proliferation, cell survival, cell motility, and/or oncogenicity) or immunogenic/immunological function of the sequence from which it was derived. In reference to antibodies, the term "derivatives" includes, for example, hybrid and recombinant antibodies. "Hybrid" and "recombinant" versions of an antibody include, for example, humanised antibodies, diabodies, triabodies, and single chain antibodies.

As used herein, the term "epitope" includes any polypeptide or peptide determinant capable of specific binding to an immunoglobulin, or a related molecule, e.g., an scFv, or a T cell receptor. Epitopic determinants generally include chemically active surface groupings of molecules such as amino acids and/or sugar side chains, and usually have specific three dimensional structural characteristics, as well as specific charge characteristics. Epitopes include two main types, namely, sequential epitopes (SEs), where the antibody binds to a contiguous stretch of amino acid residues that are linked by peptide bond, and conformational epitopes (CEs), where the antibody binds to non-contiguous residues, brought together by folding of polypeptide chain. Conformational epitopes are also referred to herein as native epitopes. It is known from the analyses of the crystal structures of antigen-antibody complexes that, to be recognized by an antibody, the residues must be generally accessible for interactions, and thus presented near the surface of antigen. A commercially available algorithm was used to predict SE and CE An antibody is said to specifically bind an antigen when the dissociation constant is ≤ 1 µM; preferably ≤ 100 nM, and most preferably ≤ 10 nM. In certain aspects, an inhibitor can specifically bind with or react with a ligand as described herein. For an antibody, "specifically bind" or "specifically immunoreacts with" is meant that the antibody reacts with one or more antigenic determinants of the desired antigen and does not react (i.e., bind) with other polypeptides or binds at much lower affinity (e.g., K_{d} > 10⁻⁶) with other polypeptides.

The term "expression" includes production of polynucleotides and polypeptides, in particular, the production of RNA (e.g., mRNA) from a gene or portion of a gene, and includes the production of a polypeptide encoded by an RNA or gene or portion of a gene, and the appearance of a detectable material associated with expression. For example, the formation of a complex, for example, from a polypeptide-polypeptide interaction, polypeptide-nucleotide interaction, or the like, is included within the scope of the term "expression". Another example is the binding of a binding ligand, such as a hybridization probe or antibody, to a gene or other polynucleotide or oligonucleotide, a polypeptide or a protein fragment, and the visualization of the binding ligand. Thus, increased intensity of a spot on a microarray, on a hybridization blot such as a Northern blot, or on an immunoblot such as a Western blot, or on a bead array, or by PCR analysis, is included within the term "expression" of the underlying biological molecule.

The term "homology", as used herein, refers to a degree of complementarity. There may be partial homology (i.e., a certain % identity) or complete homology (i.e., 100% identity). A partially complementary sequence that at least partially inhibits an identical sequence from hybridizing to a target nucleic acid is referred to using the functional term "substantially homologous." The inhibition of hybridization of the completely complementary sequence to the target sequence may be examined using a hybridization assay (e.g., Southern or northern blot, solution hybridization, and the like) under conditions of low stringency. A substantially homologous sequence or hybridization probe will compete for and inhibit the binding of a completely homologous sequence to the target sequence under conditions of low stringency. This is not to say that conditions of low stringency are such that nonspecific binding is permitted; low stringency conditions require that the binding of two sequences to one another be a specific (i.e., selective) interaction.

The term "hybridization", as used herein, refers to any process by which a strand of nucleic acid binds with a complementary strand through base pairing.

An "insertion" or "addition", as used herein, refers to a change in an amino acid or nucleotide sequence resulting in the addition of one or more amino acid residues or nucleotides, respectively, as compared to the naturally occurring molecule.

"Inhibition" or "inhibiting" for Artemin and/or a related ligand is intended to refer to blocking or reducing biological activity and/or levels of these ligands. While it may be desirable to completely inhibit activity, this need not be essential. "Inhibition" may occur at the level of expression and production (e.g., transcriptional or translational levels) or by targeting function, for example. The terms "inhibit" or "inhibition" for Artemin and/or a related ligand, as used herein, refer to a decrease, for example, in DNA levels (e.g., decreased DNA synthesis, increased turnover, and/or decreased stability), RNA levels (e.g., decreased transcription, increased turnover, and/or decreased stability), or polypeptide levels (e.g., decreased translation, increased turnover, and/or decreased stability) or activity, or post-translational modification. An inhibitor can also decrease or block the activities or expression levels of downstream or upstream agents in the relevant pathway. In particular aspects, the inhibitory agents of the invention are useful for inhibiting cell proliferation, cell survival, cell motility, and/or oncogenicity, especially for cancer cells, as described herein. In particular, the agents may be useful for one or more of: decreasing cell proliferation (e.g., by decreasing cell division), increasing cell death (e.g., by increasing apoptosis or necrosis), or decreasing cellular invasion and/or metastasis (e.g., by decreasing cytoskeletal activity, cell motors).

The terms "modified" or "modification" refer to altered sequences and to sequence fragments, variants, and derivatives, as described herein. The term includes polypeptides, polynucleotides, antibodies, and like agents described herein.

The term "monoclonal antibody," "MAb," or "monoclonal antibody composition," as used herein, refers to a population of antibody molecules that contain a molecular species of antibody molecule including a unique light chain gene product and a unique heavy chain gene product. In particular, the complementarity determining regions (CDRs) of the monoclonal antibody are unique. MAbs include an antigen binding site capable of immunoreacting with a particular epitope of the antigen characterized by a unique binding affinity for it.

"Nucleic acid sequence" or "nucleotide sequence" as used herein, refers to a sequence of a polynucleotide, oligonucleotide, or fragments thereof, and to DNA or RNA of natural, recombinant, synthetic or semi-synthetic, origin which may be single or double stranded, and can represent sense or antisense strand, or coding or non-coding regions. The sequences, preferably, comprise at least 15, 21, 27, 33, 36, 39, 45, 51, 57, or 66 nucleotides, preferably at least 15 to 36, 15 to 66, 36 to 66, or 45 to 66 nucleotides, or at least 100 nucleotides, or at least 1000 nucleotides. It will be understood that each reference to a "nucleic acid sequence" or "nucleotide sequence," herein, will include the original, full length sequence, as well as any complements or modifications thereof. It will be further understood that any reference to a "polynucleotide" (or "oligonucleotide," or "probe," or "primer," etc.) having a particular SEQ ID NO. will encompass both the DNA and the counterpart RNA sequences.

The term "oligonucleotide" refers to a polynucleotide, typically a probe or primer, including, without limitation, single stranded DNAs, single or double stranded RNAs, RNA:DNA hybrids, and double stranded DNAs. Oligonucleotides, such as single stranded DNA probe oligonucleotides, are often synthesized by chemical methods, for example using automated oligonucleotide synthesizers that are commercially available, or by a variety of other methods, including *in vitro* expression systems, recombinant techniques, and expression in cells and organisms.

The term "patient" or "subject" includes human and non-human animals. Non-human animals include, but are not limited to, birds and mammals, in particular, mice, rabbits, cats, dogs, pigs, sheep, goats, cows, and horses.

"Peptide nucleic acid" or "PNA" as used herein, refers to an antisense molecule or anti-gene agent which comprises bases linked via a peptide backbone.

As used herein, the phrase "pharmaceutically acceptable diluents, carriers, and/or excipients" is intended to include substances that are useful in preparing a pharmaceutical composition, and may be co-administered with an agent described herein while allowing same to perform its intended function. These are generally safe, non-toxic, and neither biologically nor otherwise undesirable. Examples of pharmaceutically acceptable diluents, carriers, and/or excipients include solutions, solvents, dispersion media, delay agents, emulsions, and the like. Diluents, carriers, and/or excipients may contain minor amounts of additives such as substances that enhance isotonicity and chemical stability.

The term "polynucleotide" (e.g., "Artemin or a related ligand," which can be used to discuss a polynucleotide), when used in the singular or plural, generally refers to any nucleic acid sequence, e.g., any polyribonucleotide or polydeoxribonucleotide, which may be unmodified RNA or DNA or modified RNA or DNA. This includes, without limitation, single and double stranded DNA, DNA including single and double- stranded regions, single and double stranded RNA, and RNA including single and double stranded regions, hybrid molecules comprising DNA and RNA that may be single stranded or, more typically, double stranded or include single and double stranded regions. Also included are triple-stranded regions comprising RNA or DNA or both RNA and DNA. Specifically included are mRNAs, cDNAs, and genomic DNAs, and any fragments thereof. The term includes DNAs and RNAs that contain one or more modified bases, such as tritiated bases, or unusual bases, such as inosine. The polynucleotides can encompass coding or non-coding sequences, or sense or antisense sequences, or iRNAs such as siRNAs. It will be understood that each reference to a "polynucleotide" or like term, herein, will include the full length sequences as well as any complements or modifications thereof.

"Polypeptide" (e.g., "Artemin or a related ligand," which can be used to discuss a polypeptide), as used herein, refers to an oligopeptide, peptide, or protein, or fragment thereof, and to naturally occurring, recombinant, synthetic, or semi-synthetic molecules. Where these terms are recited herein to refer to an amino acid sequence of a naturally occurring protein molecule, the terms are not meant to limit the amino acid sequence to the complete, original sequence for the full length molecule. It will be understood that each reference to "polypeptide" or like term, herein, will include the full length sequence, as well as any modifications thereof.

The terms "stringent conditions" or "stringency," as used herein, refer to the conditions for hybridization as defined by the nucleic acid, salt, and temperature. These conditions are well known in the art and may be altered in order to identify or detect identical or related polynucleotide sequences. See, e.g., Sambrook, J. et al. (1989) Molecular Cloning, A Laboratory Manual, Cold Spring Harbor Press, Plainview, NY, and Ausubel, F. M. et al. (1989) Current Protocols in Molecular Biology, John Wiley & Sons, New York, NY. Numerous equivalent conditions comprising either low or high stringency depend on factors such as the length and nature of the sequence (e.g., DNA, RNA, base composition), nature of the target (e.g., DNA, RNA, base composition), milieu (e.g., in solution or immobilized on a solid substrate), concentration of salts and other components (e.g., formamide, dextran sulfate and/or polyethylene glycol), and temperature of the reactions (e.g., within a range from about 5°C below the melting temperature of the probe to about 20°C to 25°C below the melting temperature). One or more factors be may be varied to generate conditions of either low or high stringency different from, but equivalent to, the above listed conditions.

"SEQ ID NO:" as referred to herein, can indicate each sequence identifier individually, or any combination thereof, or all such sequence identifiers.

The terms "substantially purified" or "isolated" as used herein, refer to nucleic or amino acid sequences that are removed from their cellular, recombinant, or synthetic environment, and are at least 60% free, preferably 75% free, and most preferably at least 90% free or at least 99% free from other components with which they are associated in their environment. "Isolated" polynucleotides and polypeptides have been identified and separated from at least one contaminant nucleic acid molecule with which they are associated in their natural state. Accordingly, it will be understood that isolated polynucleotides and polypeptides are in a form which differs from the form or setting in which they are found in nature. It will further be appreciated that "isolated" does not necessarily reflect the exact extent (e.g., a specific percentage) to which the sequence has been purified.

"Treatment" and like terms refer to methods and compositions to prevent, cure, or ameliorate a medical disorder (e.g., medical disease, condition, or syndrome), or reduce at least a symptom of such disorder. In particular, this includes methods and compositions to prevent or delay onset of a medical disorder; to cure, correct, reduce, slow, or ameliorate the physical or developmental effects of a disorder; and/or to prevent, end, reduce, or ameliorate the pain or suffering caused the disorder. The term "treatment" is to be considered in its broadest context. The term does not necessarily imply that the subject is treated until total recovery. Accordingly, "treatment" as used herein broadly includes inhibiting, reducing or preventing cell proliferation, cell survival, cell motility, and/or oncogenicity; ameliorating the symptoms or severity of cell proliferation, cell survival, cell motility, and/or oncogenicity; or preventing or otherwise reducing the risk of developing cell proliferation, cell survival, cell motility, and/or oncogenicity, for example cancer, and in particular, breast cancer, colon cancer, prostate cancer, endometrial cancer, lung cancer, stomach cancer, liver cancer, or another cancer.

A "variant" of polypeptide, as used herein, refers to an amino acid sequence that is altered by one or more amino acids. Similarly, a variant antibody is altered by one or more amino acids. A variant polynucleotide is altered by one or more nucleotides. A variant may result in "conservative" changes, wherein a substituted amino acid has similar structural or chemical properties, e.g., replacement of leucine with isoleucine. More rarely, a variant may result in "nonconservative" changes, e.g., replacement of a glycine with a tryptophan. Analogous minor variations may also include amino acid deletions or insertions, or both. Guidance in determining which amino acid residues may be substituted, inserted, or deleted without abolishing biological or immunogenic activity may be found using computer programs well known in the art, for example, LASERGENE software (DNASTAR).

Disclosed herein are also variants which retain at least one biological activity (e.g., effect on cell proliferation, cell survival, cell motility, and/or oncogenicity) or immunogenic/immunological function. A preferred variant is one having at least 80%, and more preferably at least 90%, sequence identity to a disclosed sequence. A most preferred variant is one having at least 95%, at least 97%, at least 98%, or at least 99% sequence identity to a sequence disclosed herein. The percentage identity is determined by aligning the two sequences to be compared as described below, determining the number of identical residues in the aligned portion, dividing that number by the total number of residues in the (queried) sequence, and multiplying the result by 100. A useful alignment program is AlignX (Vector NTI).

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, Molecular Cloning: A Laboratory Manual, 2nd edition, Sambrook et al., 1989; also, Molecular Cloning: A Laboratory Manual, 3rd Edition, Sambrook et al., 2000; Oligonucleotide Synthesis, MY Gait, ed., 1984; Animal Cell Culture, R.I. Freshney, ed., 1987; Methods in Enzymology, Academic Press, Inc.; Handbook of Experimental Immunology, 4th edition, D .M. Weir & CC. Blackwell, eds., Blackwell Science Inc., 1987; Gene Transfer Vectors for Mammalian Cells, JAM. Miller & MAP. Calos, eds., 1987; Current Protocols in Molecular Biology, FEM. Ausubel et al., eds., 1987; and PCR: The Polymerase Chain Reaction, Mullis et al., eds., 1994.

### Artemin

The glial cell line derived neurotrophic factor (GDNF) family consists of four members, namely, Artemin, Neurturin, Persephin, and GDNF. In the current understanding in the field, the members act by binding to a co-receptor GFRα, which activates a common tyrosine kinase receptor RET to trigger downstream targets, which in turn affect cell growth, differentiation, survival, and apoptosis (Baudet *et al.,* 2000; Coulpier *et al.,* 2002). ₌Mammary carcinoma MCF-7 cells express all of the family members. The present disclosure demonstrates that Artemin, in particular, enhances anchorage-independent growth and colony formation (i.e., oncogenicity), decreases apoptosis, and increases cell migration/invasion. Depletion of endogenous Artemin in MCF-7 cells by siRNA results in opposite effects to forced expression. Therefore, the present study shows that Artemin, apart from its normal role in neural cells, affects cancer cell apoptosis, migration, and invasion, as well as anchorage-independent growth and colony formation (i.e., oncogenicity). Inhibition of these biological functions can be used to dramatically limit the onset, progression, metastasis, and recurrence of cancer, especially breast cancer, colon cancer, prostate cancer, endometrial cancer, lung cancer, stomach cancer, liver cancer, or another Thus, the data disclosed herein demonstrates that Artemin expression promotes survival, migration, and invasion of breast carcinoma MCF-7 cells, in addition to anchorage-independent growth and colony formation (i.e., oncogenicity), and that these activities can be effectively inhibited by reducing the expression levels of Artemin via siRNA or activity via antibodies. As such, Artemin and related ligands represent ideal new targets for cancer treatment and diagnosis, particularly for breast cancer, colon cancer, prostate cancer, endometrial cancer, lung cancer, stomach cancer, liver cancer, or another cancer. Reagents that inhibit the biological activity and/or levels of Artemin and/or a related ligand can be used to inhibit cell proliferation, cell survival, cell motility, and/or oncogenicity, for example, for cancer cells. These reagents can include, for example, chemical compounds (e.g., small molecules), antagonists, antibodies, and iRNAs. Similarly, diagnostic agents (e.g., polynucleotides and antibodies) can be used to determine levels for Artemin and/or a related ligand, and detect a cancerous condition, e.g., cancer onset, progression, metastasis, or recurrence.

### Artemin polynucleotides and polypeptides

The disclosure encompasses polypeptides for Artemin or a related ligand, including those comprising an amino acid sequence selected from SEQ ID NO: 1 to 3, SEQ ID NO. 5 to 32 or 34, amino acids 40 - 220 of SEQ ID NO: 1, amino acids 57 - 237 of SEQ ID NO. 2, amino acids 48 - 228 of SEQ ID NO. 3, or an amino acid sequence encoded by the nucleotide sequence of any one of SEQ ID NO: 35 to 42, and modifications thereof. The invention encompasses the use of these polypeptides in the diagnosis of cancer, especially breast cancer and endometrial cancer. The disclosure further encompasses the use of the polypeptides for preparing antibodies to inhibit the cell proliferation, cell survival, cell motility, and/or oncogenicity, for example, for cancer cells. The polypeptides may be expressed and used in various assays to determine their biological activity. The polypeptides may be used for large-scale synthesis and isolation protocols, for example, for commercial production. Such polypeptides may be used to raise antibodies, to isolate corresponding amino acid sequences, and to quantitatively determine levels of the amino acid sequences. The polypeptides can also be used as compositions, for example, pharmaceutical or kit compositions. The polypeptides can also be used to provide health benefits. For such benefits, the polypeptides can be used to prepare antibodies, and compositions thereof.

The polypeptides comprise at least one sequence selected from the group consisting of: an amino acid sequence selected from SEQ ID NO: 1 to 3, SEQ ID NO. 5 to 32 or 34, amino acids 40 - 220 of SEQ ID NO. 1, amino acids 57 - 237 of SEQ ID NO. 2, amino acids 48 - 228 of SEQ ID NO. 3, or an amino acid sequence encoded by the nucleotide sequence of any one of SEQ ID NO: 35 to 42. All of these sequences are collectively referred to herein as polypeptides of the disclosure.

The disclosure encompasses polynucleotides for Artemin or a related ligand including those of SEQ ID NO: 1 to 3, SEQ ID NO. 5 to 32 or 34, amino acids 40 - 220 of SEQ ID NO. 1, amino acids 57 - 237 of SEQ ID NO. 2, amino acids 48 - 228 of SEQ ID NO. 3, or an amino acid sequence encoded by the nucleotide sequence of any one of SEQ ID NO: 35 to 42, and complements, and modified sequences thereof. The invention encompasses the use of these polynucleotides in the diagnosis of cancer, especially breast cancer, endometrial cancer.

The disclosure further encompasses the use of these polynucleotides for the inhibition of cell proliferation, cell survival, cell motility, and/or oncogenicity, for example, for cancer cells. Accordingly, the disclosure encompasses the use of the polynucleotides for preparing expression vectors and host cells, and for preparing antisense polynucleotides and iRNAs. The isolated polynucleotides also have utility in genome mapping, in physical mapping, and in cloning of genes of more or less related species. Probes designed using the polynucleotides may be used to detect the presence and examine the expression patterns of genes in any organism having sufficiently homologous DNA and RNA sequences in their cells, using techniques that are well known in the art, such as slot blot techniques or microarray analysis. Primers designed using the polynucleotides may be used for sequencing and PCR amplifications. The polynucleotides may also be used as compositions, for example, pharmaceutical compositions. The polynucleotides can also be used to provide health benefits. For such benefits, the polynucleotides can be presented as expression vectors or host cells comprising expression vectors.

The polynucleotides comprise at least one sequence selected from the group consisting of: an amino acid sequence selected from SEQ ID NO: 1 to 3, SEQ ID NO. 5 to 32 or 34, amino acids 40 - 220 of SEQ ID NO. 1, amino acids 57 - 237 of SEQ ID NO. 2, amino acids 48 - 228 of SEQ ID NO. 3, or an amino acid sequence encoded by the nucleotide sequence of any one of SEQ ID NO: 35 to 42. All of these polynucleotides and oligonucleotide probes and primers are collectively referred to herein, as polynucleotides of the disclosure.

It will be appreciated by those skilled in the art that as a result of the degeneracy of the genetic code, a multitude of nucleotide sequences encoding the polypeptides some bearing minimal homology to the nucleotide sequences of any known and naturally occurring gene, may be produced. Thus, the disclosure contemplates each and every possible variation of nucleotide sequence that could be made by selecting combinations based on possible codon choices. These combinations are made in accordance with the standard triplet genetic code as applied to naturally occurring amino acid sequences, and all such variations are to be considered as being specifically disclosed.

Nucleotide sequences for Artemin or a related ligand, or modifications thereof, are preferably capable of hybridizing to the nucleotide sequence of the naturally occurring sequence under appropriately selected conditions of stringency. However, it may be advantageous to produce nucleotide sequences, or modifications thereof, possessing a substantially different codon usage. Codons may be selected to increase the rate at which expression of the polypeptide occurs in a particular prokaryotic or eukaryotic host in accordance with the frequency with which particular codons are utilized by the host. Other reasons for substantially altering the nucleotide sequence and its derivatives without altering the encoded amino acid sequences include the production of RNA transcripts having more desirable properties, such as a greater half-life, than transcripts produced from the naturally occurring sequence.

The disclosure also encompasses production of polynucleotides for Artemin or a related ligand, or modifications thereof, entirely by synthetic chemistry. After production, the synthetic sequence may be inserted into any of the many available expression vectors and cell systems using reagents that are well known in the art. Moreover, synthetic chemistry may be used to introduce mutations into a nucleotide sequence, or any derivatives thereof. Also encompassed are polynucleotide sequences that are capable of hybridizing to the claimed nucleotide sequences, or complements, or modified sequences thereof, under various conditions of stringency as taught in Wahl, G. M. and S. L. Berger (1987; Methods Enzymol. 152:399-407) and Kimmel, A. R. (1987; Methods Enzymol. 152:507-511). Methods for DNA sequencing which are well known and generally available in the art may be used to practice any of the embodiments of the invention. The methods may employ such enzymes as the Klenow fragment of DNA polymerase I, SEQUENASE (U.S. Biochemical Corp, Cleveland, OH), Taq polymerase (Perkin Elmer), thermostable T7 polymerase (Amersham Pharmacia Biotech, Piscataway, NJ), or combinations of polymerases and proofreading exonucleases such as those found in the ELONGASE Amplification System (Life Technologies, Gaithersburg, MD). Preferably, the process is automated with machines such as the Hamilton Micro Lab 2200 (Hamilton, Reno, NV), Peltier Thermal Cycler (PTC200; MJ Research, Watertown, MA) the ABI Catalyst and 373 and 377 DNA Sequencers (Perkin Elmer), or the Genome Sequencer 20™ (Roche Diagnostics).

The nucleic acid sequences may be extended utilizing a partial nucleotide sequence and employing various methods known in the art to detect upstream sequences such as promoters and regulatory elements. For example, one method which may be employed, "restriction-site" PCR, uses universal primers to retrieve unknown sequence adjacent to a known locus (Sarkar, G. (1993) PCR Methods Applic. 2:318-322). In particular, genomic DNA is first amplified in the presence of primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Capillary electrophoresis systems which are commercially available may be used to analyze the size or confirm the nucleotide sequence of sequencing or PCR products. In particular, capillary sequencing may employ flowable polymers for electrophoretic separation, four different fluorescent dyes (one for each nucleotide), which are laser activated, and detection of the emitted wavelengths by a charge coupled device camera. Output/light intensity may be converted to electrical signal using appropriate software (e.g., GENOTYPER and Sequence NAVIGATOR, Perkin Elmer) and the entire process from loading of samples to computer analysis and electronic data display may be computer controlled. Capillary electrophoresis is especially preferable for the sequencing of small pieces of DNA which might be present in limited amounts in a particular sample.

In another embodiment of the disclosure, the polynucleotides or modification thereof may be used in recombinant DNA molecules to direct expression of polypeptides for Artemin or a related ligand, or modifications thereof, in appropriate host cells. Due to the inherent degeneracy of the genetic code, other DNA sequences which encode substantially the same or a functionally equivalent amino acid sequence may be produced, and these sequences may be used to clone and express polypeptides. The nucleotide sequences can be engineered using methods generally known in the art in order to alter amino acid encoding sequences for a variety of reasons, including but not limited to, alterations which modify the cloning, processing, and/or expression of the gene product. DNA shuffling by random fragmentation and PCR reassembly of gene fragments and synthetic oligonucleotides may be used to engineer the nucleotide sequences. For example, site-directed mutagenesis may be used to insert new restriction sites, alter glycosylation patterns, change codon preference, introduce mutations, and so forth.

In another embodiment of the disclosure, a natural, modified, or recombinant nucleic acid sequence encoding a polypeptide may be ligated to a heterologous sequence to encode a fusion protein. For example, it may be useful to encode a chimeric sequence that can be recognized by a commercially available antibody. A fusion protein may also be engineered to contain a cleavage site located between the polypeptide and the heterologous protein sequence, so that the polypeptide may be cleaved and purified away from the heterologous moiety.

In another embodiment, nucleotide sequences may be synthesized, in whole or in part, using chemical methods well known in the art (see Caruthers, M. H. et al. (1980) Nucl. Acids Res. Symp. Ser. 215-223, Horn, T. et al. (1980) Nucl. Acids Res. Symp. Ser. 225-232). Alternatively, the polypeptide itself may be produced using chemical methods to synthesize the amino acid sequence, or a modification thereof. For example, polypeptide synthesis can be performed using various solid-phase techniques (Roberge, J. Y. et al. (1995) Science 269:202-204; Merrifield J. (1963) J. Am. Chem. Soc. 85:2149-2154) and automated synthesis may be achieved, for example, using the ABI 431A Peptide Synthesizer (Perkin Elmer). Various fragments of polypeptides may be chemically synthesized separately and combined using chemical methods to produce the full length molecule.

The newly synthesized polypeptide may be isolated by preparative high performance liquid chromatography (e.g., Creighton, T. (1983) Proteins Structures and Molecular Principles, WH Freeman and Co., New York, NY). The composition of the synthetic polypeptides may be confirmed by amino acid analysis or sequencing (e.g., the Edman degradation procedure; Creighton, supra). Additionally, the amino acid sequence of the polypeptide, or any part thereof, may be altered during direct synthesis and/or combined using chemical methods with sequences from other proteins, or any part thereof, to produce a modified molecule.

In order to express a biologically active polypeptides, the nucleotide sequences encoding the polypeptide or functional equivalents, may be inserted into appropriate expression vector, e.g., a vector which contains the necessary elements for the transcription and translation of the inserted coding sequence. Methods which are well known to those skilled in the art may be used to construct expression vectors containing sequences encoding the polypeptide and appropriate transcriptional and translational control elements. These methods include *in vitro* recombinant DNA techniques, synthetic techniques, and *in vivo* genetic recombination. Such techniques are described in Sambrook, J. et al. (1989) Molecular Cloning, A Laboratory Manual, Cold Spring Harbor Press, Plainview, NY; also, Sambrook, J. et al. (2000) Molecular Cloning, A Laboratory Manual, Cold Spring Harbor Laboratory Press, Plainview, NY, and Ausubel, F. M. et al. (1989) Current Protocols in Molecular Biology, John Wiley & Sons, New York, NY.

A variety of expression vector/host systems may be utilized to contain and express sequences encoding the polypeptides described herein.

These include, but are not limited to, microorganisms such as bacteria transformed with recombinant phage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems. For bacteria, useful plasmids include pET, pRSET, pTrcHis2, and pBAD plasmids from Invitrogen, pET and pCDF plasmids from Novagen, and Director™ plasmids from Sigma-Aldrich. In particular, *E. coli* can be used with the expression vector pET. The disclosure is not limited by the expression vector or host cell employed.

The "control elements" or "regulatory sequences" are those non-translated regions (e.g., enhancers, promoters, 5' and 3' untranslated regions) which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the BLUESCRIPT phagemid (Stratagene, LaJolla, CA) or pSPORT1 plasmid (Life Technologies) and the like may be used. The baculovirus polyhedrin promoter may be used in insect cells. Promoters or enhancers derived from the genomes of plant cells (e.g., heat shock, RUBISCO, and storage protein genes) or from plant viruses (e.g., viral promoters or leader sequences) may be cloned into the vector.

In bacterial systems, a number of expression vectors may be selected depending upon the use intended for the polypeptide. For example, when large quantities of polypeptide are needed, vectors which direct high level expression of fusion proteins that are readily purified may be used. Such vectors include, but are not limited to, the multifunctional *E. coli* cloning and expression vectors such as BLUESCRIPT (Stratagene), in which the sequence encoding a polypeptide may be ligated into the vector in frame with sequences for the amino-terminal Met and the subsequent 7 residues of β-galactosidase so that a hybrid protein is produced; pIN vectors (Van Heeke, G. and S. M. Schuster (1989) J. Biol. Chem. 264:5503-5509); and the like.

pGEX vectors (Promega, Madison, WI) may also be used to express the polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor Xa protease cleavage sites so that the cloned polypeptide of interest can be released from the GST moiety at will. In the yeast, *Saccharomyces cerevisiae,* a number of vectors containing constitutive or inducible promoters such as alpha factor, alcohol oxidase, and PGH may be used. For reviews, see Ausubel et al. (supra) and Grant et al. (1987) Methods Enzymol. 153:516-544.

Specific initiation signals may also be used to achieve more efficient translation of sequences encoding the polypeptides of the disclosure.

Such signals include the ATG initiation codon and adjacent sequences. In cases where sequences encoding a polypeptide, its initiation codon, and upstream sequences are inserted into the appropriate expression vector, no additional transcriptional or translational control signals may be needed. However, in cases where only coding sequence, or a modification thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell may be chosen for its ability to modulate the expression of the inserted sequences or to process the expressed polypeptide in the desired fashion. Such modifications of the sequence include, but are not limited to, acetylation, carboxylation, glycosylation, phosphorylation, lipidation, and acylation. Post-translational processing which cleaves a "prepro" form of the polypeptide may also be used to facilitate correct insertion, folding, and/or function. Different host cells which have specific cellular machinery and characteristic mechanisms for post-translational activities are available from the American Type Culture Collection (ATCC; Bethesda, MD) and may be chosen to ensure the correct modification and processing of the sequence. Specific host cells include, but are not limited to, *Rhodotorula, Aureobasidium, Saccharomyces, Sporobolomyces, Pseudomonas, Erwinia* and *Flavobacterium*; or such other organisms as *Escherichia, Lactobacillus, Bacillus, Streptomyces,* and the like. Particular host cells include *Escherichia coli,* which is particularly suited for use with the present invention, *Saccharomyces cerevisiae, Bacillus thuringiensis, Bacillus subtilis, Streptomyces lividans,* and the like.

There are several techniques for introducing nucleic acids into eukaryotic cells cultured *in vitro.* These include chemical methods (Felgner et al., Proc. Natl. Acad. Sci., USA, 84:7413 7417 (1987); Bothwell et al., Methods for Cloning and Analysis of Eukaryotic Genes, Eds., Jones and Bartlett Publishers Inc., Boston, Mass. (1990), Ausubel et al., Short Protocols in Molecular Biology, John Wiley and Sons, New York, NY (1992); and Farhood, Annal. NY Acad. Sci., 716:23 34 (1994)), use of protoplasts (Bothwell, supra) or electrical pulses (Vatteroni et al., Mutn. Res., 291:163 169 (1993); Sabelnikov, Prog. Biophys. Mol. Biol., 62: 119 152 (1994); Bothwell et al., supra; and Ausubel et al., supra), use of attenuated viruses (Davis et al., J. Virol. 1996, 70(6), 3781 3787; Brinster et al. J. Gen. Virol. 2002, 83(Pt 2), 369 381; Moss, Dev. Biol. Stan., 82:55 63 (1994); and Bothwell et al., supra), as well as physical methods (Fynan et al., supra; Johnston et al., Meth. Cell Biol., 43(Pt A):353 365 (1994); Bothwell et al., supra; and Ausubel et al., supra).

Successful delivery of nucleic acids to animal tissue can be achieved by cationic liposomes (Watanabe et al., Mol. Reprod. Dev., 38:268 274 (1994)), direct injection of naked DNA or RNA into animal muscle tissue (Robinson et al., Vacc., 11:957 960 (1993); Hoffman et al., Vacc. 12:1529 1533; (1994); Xiang et al., Virol., 199:132 140 (1994); Webster et al., Vacc., 12:1495 1498 (1994); Davis et al., Vacc., 12:1503 1509 (1994); Davis et al., Hum. Molec. Gen., 2:1847 1851 (1993); Dalemans et al. Ann NY Acad. Sci. 1995, 772, 255 256. Conry, et al. Cancer Res. 1995, 55(7), 1397-1400), and embryos (Naito et al., Mol. Reprod. Dev., 39:153 161 (1994); and Burdon et al., Mol. Reprod. Dev., 33:436 442 (1992)), intramuscular injection of self replicating RNA vaccines (Davis et al., J Virol 1996, 70(6), 3781 3787; Balasuriya et al. Vaccine 2002, 20(11 12), 1609 1617) or intradermal injection of DNA using "gene gun" technology (Johnston et al., supra).

A variety of protocols for detecting and measuring the expression of the polypeptides using either polyclonal or monoclonal antibodies specific for the protein are known in the art. Examples include enzyme-linked immunosorbent assay (ELISA), radioimmunoassay (RIA), and fluorescence activated cell sorting (FACS). A two-site, monoclonal-based immunoassay can be used with monoclonal antibodies reactive to two non-interfering epitopes on the polypeptide, but a competitive binding assay can also be used. These and other assays are described, among other places, in Hampton, R. et al. (1990; Serological Methods, a laboratory Manual, APS Press, St Paul, MN) and Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides include oligolabeling, nick translation, end-labeling or PCR amplification using a labeled nucleotide. Alternatively, the sequences, or any modifications thereof, may be cloned into a vector for the production of an mRNA probe. Such vectors are known in the art, are commercially available, and may be used to synthesize RNA probes *in vitro* by addition of an appropriate RNA polymerase such as T7, T3, or SP6 and labeled nucleotides. These procedures may be conducted using a variety of commercially available kits Amersham Pharmacia Biotech, Promega, and US Biochemical. Suitable reporter molecules or labels, which may be used for ease of detection, include radionuclides, enzymes, fluorescent, chemiluminescent, or chromogenic agents as well as substrates, cofactors, inhibitors, magnetic particles, and the like.

Expression vectors or host cells transformed with expression vectors may be cultured under conditions suitable for the expression and recovery of the polypeptide from culture. The culture can comprise components for *in vitro* or *in vivo* expression. *In vitro* expression components include those for rabbit reticulocyte lysates, *E. coli* lysates, and wheat germ extracts, for example, Expressway™ or RiPs systems from Invitrogen, Genelator™ systems from iNtRON Biotechnology, EcoPro™ or STP3™ systems from Novagen, TNT® Quick Coupled systems from Promega, and EasyXpress systems from QIAGEN. The polypeptide produced from culture may be secreted or contained intracellularly depending on the sequence and/or the vector used. In particular aspects, expression vectors which encode a phage polypeptide can be designed to contain signal sequences which direct secretion of the polypeptide through a prokaryotic or eukaryotic cell membrane.

Other constructions may include an amino acid domain which will facilitate purification of the polypeptide. Such domains include, but are not limited to, metal chelating peptides such as histidine-tryptophan (e.g., 6X-HIS) modules that allow purification on immobilized metals, protein A domains that allow purification on immobilized immunoglobulin, and the domain utilized in the FLAG® extension/affinity purification system (Immunex Corp., Seattle, WA). Useful epitope tags include 3X-FLAG®, HA, VSV-G, V5, HSV, GST, GFP, MBP, GAL4, and β-galactosidase. Useful plasmids include those comprising a biotin tag (e.g., PinPoint™ plasmids from Promega), calmodulin binding protein (e.g., pCAL plasmids from Stratagene), streptavidin binding peptide (e.g., InterPlay™ plasmids from Stratagene), a c-myc or FLAG® tag (e.g., Immunoprecipitation plasmids from Sigma-Aldrich), or a histidine tag (e.g., QIAExpress plasmids from QIAGEN).

To facilitate purification, expression vectors can include a cleavable linker sequences such as those specific for Factor Xa or enterokinase (Invitrogen, San Diego, CA). For example, the vector can include one or more linkers between the purification domain and the polypeptide. One such expression vector provides for expression of a fusion protein comprising a polypeptide of the invention and a nucleic acid encoding 6 histidine residues preceding a thioredoxin or an enterokinase cleavage site. The histidine residues facilitate purification on IMAC (immobilized metal ion affinity chromatography as described in Porath, J. et al. (1992) Prot. Exp. Purif. 3: 263-281) while the enterokinase cleavage site provides a means for purifying the polypeptide from the fusion protein. A discussion of vectors which contain fusion proteins is provided in Kroll, D. J. et al. (1993; DNA Cell Biol. 12:441-453).

### Polynucleotides for inhibition of Artemin and/or related ligands

As mentioned herein before, in one embodiment, polynucleotides may be utilised to inhibit Artemin and/or a related ligand in accordance with the invention. Such polynucleotides may be DNA, RNA, single stranded, or double stranded. Polynucleotides for use with the invention may be referred to herein as "isolated" polynucleotides. Isolated polynucleotides may be obtained using a number of techniques known in the art. For example, recombinant DNA technology may be used as described for example in Sambrook., J. et al. (1989) Molecular Cloning: A Laboratory Manual (Third 2nd edition), cold Spring Harbor Laboratory Press, Plainview, NY and Sambrook and Russell (2000) Molecular Cloning: A Laboratory Manual (3rd) Edition), Cold Spring Harbor Laboratory Press, Plainview, NY. Similarly, chemical synthesis, for example, using phosphoramidite and solid phase chemistry, may be used. Polynucleotides may be designed on the basis of the disclosed nucleic acid sequence data, the known relative interactions between nucleotide bases, known sequence homology, and the particular nucleic acid technology to be employed, as may be exemplified herein after.

In one embodiment, interfering RNAs (iRNAs or siRNAs) may be utilised to inhibit Artemin and/or a related ligand. Polynucleotides of use in iRNA techniques will typically have 100% complementarity to their target. However, it should be appreciated that this need not be the case, provided the iRNA retains specificity for its target and the ability to block translation. Exemplary iRNA molecules may be in the form of∼18 to 21 bp double stranded RNAs with 3' dinucleotide overhangs, although shorter or longer molecules may be appropriate. In cases where the iRNA is produced *in vivo* by an appropriate nucleic acid vector, it will typically take the form of an RNA molecule having a stem-loop structure, for example, an approximately 19 nucleotide stem and a 9 nucleotide loop with 2-3 Us at the 3' end. Algorithms of use in designing siRNA are available from Cenix (Dresden, Germany, via Ambion, TX).

Exemplary target sequences for Artemin siRNA include:
AACTGGCCTGTACTCACTCAT (SEQ ID NO:46)

Exemplary insert sequences for an Artemin siRNA expression vector include:
CTGGCCTGTACTCACTCATTTCAAGAGAATGAGTGAGTACAGGCCAGTTTTTTGGAA (SEQ ID NO:47)

For these inserts, the sense strand is shown in bold, the anti-sense strand is shown with underlining, and the loop sequence is shown in italics. Any sequence can be attached to either or both ends of these inserts, for example, restriction sites can be attached for ease in cloning. Similar siRNA sequences can be designed, for example, for Persephin.

iRNA molecules can be produced in accordance with techniques described within the section entitled "Examples" herein. Further information regarding how to produce and design such molecules can be gained, for example, from: McManus MT and Sharp PA (2002) Gene silencing in mammals by small interfering RNAs. Nature Rev. Genet. 3: 737747; Dillin A (2003) The specifics of small interfering RNA specificity. Proc. Natl. Acad. Sci. USA 100(11): 6289-6291; and Tuschl T (2002) Expanding small RNA interference. Nature Biotechnol. 20: 446-448.

It will be understood by one of skill in the art that siRNA refers to short/small interfering RNA, which comprises double-stranded RNA, typically including 21 to 23 base pairs, which can be chemically synthesized. By comparison, shRNA refers to short hairpin RNA, also called vector based siRNA, which comprises single strand RNA, for example, transcribed *in vitro* or *in vivo.* Typically, shRNA includes a sequence homologous to the target mRNA (sense sequence), a "loop" region and a sequence complementary to the target sequence (anti-sense sequence). The shRNA forms a hair-pin secondary structure, and an enzyme dicer cleaves the structure, removes the hairpin, and converts it into siRNA. Thus, the sequences disclosed herein can be used to produce shRNAs, and then converted to siRNAs, as desired.

In another embodiment of the invention, an antisense molecule is used. As used herein, the term "antisense" should be taken broadly. It is intended to mean any nucleic acid (preferably RNA, but including single stranded DNA) capable of binding to a transcript for Artemin and/or a related ligand. Typically, antisense molecules or oligonucleotides comprise about 15 to 25 nucleotides which are completely complementary to their target mRNA. However, it should be appreciated that larger antisense oligonucleotides can be used including full length sequences. Also, it should be appreciated that antisense molecules which are not completely complementary to their targets may be utilised provided they retain specificity for their target and the ability to inhibit expression.

Persons of ordinary skill in the art to which the invention relates will appreciate antisense molecules of use in the invention having regard to the description provided herein, and available sequence data for Artemin and related ligands. Further information regarding antisense technology can be gained, for example, from: Kandimalla ER, Manning A, Lathan C, Byrn RA, Agrawal S. Design, biochemical, biophysical and biological properties of cooperative antisense oligonucleotides; Nucleic Acids Res. 1995 Sep 11;23(17):3578-84; Tseng BY, Brown KD. Antisense oligonucleotide technology in the development of cancer therapeutics; Cancer Gene Ther. 1994 Mar;1(1):65-71; Brysch W, Schlingensiepen KH. Design and application of antisense oligonucleotides in cell culture, in vivo, and as therapeutic agents; Cell Mol Neurobiol. 1994 Oct;14(5):557-68; Han J, Zhu Z, Hsu C, Finley WH. Selection of antisense oligonucleotides on the basis of genomic frequency of the target sequence; Antisense Res Dev. 1994 Spring;4(1):53-65.

It should be appreciated that DNAzymes, single stranded DNA, ribozymes, and triple helix DNA may also be of use in inhibiting Artemin and/or a related ligand in accordance with the invention. Ribozymes, DNAzymes, triple helix, and single stranded DNA may be readily appreciated by persons of general skill in the art to which the invention relates having regard to the description provided herein, available sequence data and current methodologies. However, by way of example methodology associated with these technologies is described in Joseph Sambrook and David W. Russell. Molecular Cloning: A Laboratory Manual (Third Edition), Cold Spring Harbor Laboratory Press, NY. Polynucleotides of use in the invention, including antisense, iRNA, ribozymes and DNAzymes may be chemically modified to increase stability or prevent degradation or otherwise. For example, the nucleic acid molecules may include analogs with unnatural bases, modified sugars, especially at the 2' position of the ribose, or altered phosphate backbones. Polynucleotides of use in the invention may also include sequences which allow for targeted degradation of any transcript to which they bind. For example, a sequence specific for RNase H, may be included. Another example is the use of External Guide Sequences (EGSs), which may recruit a ribozyme (RNase P) to digest the transcript to which an antisense molecule is bound.

One can help ensure specificity of the likes of antisense oligonucleotides, iRNAs, ribozymes, DNAzymes, and cDNAs by screening candidate sequences for homology with other sequences in the transcriptome, the full complement of activated genes, mRNAs, or transcripts in a particular cell. Also, skilled persons may appreciate appropriate algorithms of use in designing and ensuring specificity of such polynucleotides.

Polynucleotides of use in the invention may be used in the form of nucleic acid molecules produced *in vitro,* for example, single stranded DNA, iRNA, antisense RNA, or DNAzymes. Alternatively, where appropriate, they may be used in the form of a vector adapted to produce appropriate nucleic acids, for example, antisense molecules, iRNA, or ribozymes. The inventors contemplate the use of any vectors as may be known in the art. For example, naked plasmids that employ CMV promoters may be used. Viral vectors may also be suitable, such as adeno-associated virus (AAV) and lentiviruses. Other examples of suitable promoters and viral vectors are provided herein after. One advantage of using such viral vectors is that they may allow for systemic administration, as opposed to localised administration to a tissue or tumour.

The vectors or constructs of use in the invention may include appropriate genetic elements, such as promoters, enhancers, origins of replication as are known in the art, including inducible, constitutive, or tissue-specific promoters. In a specific embodiment, a vector comprises an inducible promoter operably linked to the region coding a nucleic acid described herein (for example, antisense or suitable siRNA), such that expression of the nucleic acid is controllable by controlling the presence or absence of the appropriate inducer of transcription. In another embodiment, nucleic acid molecules are flanked by regions that promote homologous recombination at a desired site in the genome, thus providing for intrachromosomal integration of the desired nucleic acids (Koller and Smithies, 1989, Proc. Natl. Acad. Sci. USA 86:8932-8935; Zijlstra et al., 1989, Nature 342:435-438). Of course, the vectors may remain extrachromosomal.

In another embodiment of the invention, PNAs are used. PNAs are peptide-nucleic acid hybrids in which the phosphate backbone has been replaced by an achiral and neutral backbone made from N-(2-aminoethyl)-glycine units (see, e.g., Eurekah Bioscience Collection. PNA and Oligonucleotide Inhibitors of Human Telomerase. G. Gavory and S. Balasubramanian, Landes Bioscience, 2003). The bases A, G, T, C are attached to the amino nitrogen on the backbone via methylenecarbonyl linkages (P.E. Nielsen et al., Science 1991. 254: 1497-1500; M. Egholm et al., Nature 1993. 365: 566-568). PNAs bind complementary sequences with high specificity, and higher affinity relative to analogous DNA or RNA (M. Egholm et al., supra). PNA/DNA or PNA/RNA hybrids also exhibit higher thermal stability compared to the corresponding DNA/DNA or DNA/RNA duplexes (M. Egholm et al., supra). PNAs also possess high chemical and biological stability, due to the unnatural amide backbone that is not recognized by nucleases or proteases (V. Demidov et al., Biochem Pharmacol 1994. 48: 1310-1313). Typically, PNAs are at least 5 bases in length, and include a terminal lysine. PNAs may be pegylated to further extend their lifespan (Nielsen, P. E. et al. (1993) Anticancer Drug Des. 8:53-63). As a non-limiting example, the following antigene PNA is complementary to unique sequences in the coding DNA strand the Artemin gene, and is designed to inhibit mRNA synthesis. H-GCTGAGCAGCGTCGCAG-NH2 (SEQ ID NO: 48). Similar PNA sequences can be designed, for example, for GDNF, Neurturin, and/or Persephin.

In addition to polynucleotide inhibitors, small molecule inhibitors can be identified for Artemin and/or a related ligand. In particular, the 3D crystal structure of Artemin and that of the Artemin-GFRα3 complex can be used as guidance for the design of highly specific small molecules capable of inhibiting Artemin and/or a related ligand, including GDNF, Neurturin, and/or Persephin (see, e.g., Silvian L, Jin P, Carmillo P, Boriack-Sjodin PA, Pelletier C, Rushe M, Gong B, Sah D, Pepinsky B, Rossomando A. Artemin crystal structure reveals insights into heparin sulfate binding. Biochemistry. 2006 Jun 6;45(22):6801-12; Wang X, Baloh RH, Milbrandt J, Garcia KC. Structure of artemin complexed with its receptor GFRalpha3: convergent recognition of glial cell line-derived neurotrophic factors. Structure. 2006 Jun; 14(6):1083-92).

### Antibodies for inhibition of Artemin and/or related ligands

The disclosure encompasses antibodies for Artemin and/or a related ligand, for example, antibodies that bind to at least a portion of or a modified sequence thereof. In certain aspects of the disclosure, antibodies may be used to inhibit these ligands. It will be understood that, for the purposes of the disclosure, a fragment other modification of an antibody need not act fully as an antibody. That is to say, the fragment or other modification need not be capable of recruiting immune system cells to the site of binding to Artemin *in vivo.* It is not necessary to produce neutralising antibodies. Those of ordinary skill in the art to which the invention relates will recognise methods to generate antibody fragments.

Antibody fragments can encompass a portion of one of the intact antibodies, generally the antigen binding or variable region of the antibody. However, by way of general example proteolytic digestion of intact antibodies may be used, or the fragments may be directly produced via recombinant nucleic acid technology.

It is understood that the basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgA, and IgE, respectively. Within light and heavy chains, the variable and constant regions are joined by a "J" region of about 12 or more amino acids, with the heavy chain also including a "D" region of about 10 more amino *acids. See* generally, Fundamental Immunology Ch. 7 (Paul, W., ea., 2nd ed. Raven Press, N.Y. (1989)). The variable regions of each light/heavy chain pair form the antibody binding site. The antibodies of the disclosure can be characterised on the basis of isotype and epitope mapping and, in some cases, their ability to block binding of the Artemin and/or a related ligand to one or more receptors. Reagents that block ligand binding or activation have application in methods for treatment of various disorders as disclosed herein. Antibodies that recognize Artemin and/or a related ligand, but fail to block receptor binding are also contemplated. Such antibodies find use in detecting and purifying such ligands, as well as in methods for diagnosing and monitoring progression of the disorders in a subject as detailed herein after. Such antibodies might also find use in analysing the post-translational modifications of the ligand, or other modified sequences thereof.

Humanisation of antibodies may be used to reduce the immunogenicity of antibodies generated in other animals. Production of humanised antibodies or humanization of antibodies can be achieved using techniques known in the art, for example in the case of humanisation of murine antibodies by epitope-guided selection. The most frequently used strategies for the humanization of rodent monoclonal antibodies are CDR grafting (Reichmann, L., M. Clark, H. Waldman, and G. Winter. 1998. Reshaping human antibodies for therapy. Nature 332L323-327; Jones PT, Dear PH, Foote J, Neuberger MS, Winter G. 1986. Replacing the complementarity determining regions in a human antibody with those from a mouse. Nature 321:522-25) and resurfacing (Pedersen, J. T., A. H. Henry, S. J. Searle, B. C. Guild, M. Roguska, and A. R. Rees. 1994. Comparison of surface accessible residues in human and murine immunoglobulin Fv domains. Implication for humanization of murine antibodies. J. Mol. Biol. 235:959-973). Humanization of antibodies can also be achieved epitope-guided selection (Wang et al, J. Immunological Methods 241: 171-184, 2000). The methods of Maynard, J., and Georgiou, G. 2000. Antibody engineering. Annu Rev Biomed Eng 2: 339-376, provide further examples.

Humanised antibodies can be produced based on a rational design approach and iterative optimization, i.e., site-directed mutagenesis of framework residues aided by computer modelling. Other selective humanization strategies using phage display may be used (Baca, M., L. G. Presta, S. J. O'Connor, and J. A. Wells. 1997. Antibody humanization using monovalent phage display. J. Biol. Chem. 272:10678-10684; Hoogenboom, H. R., A. P. de Bruine, S. E. Hufton, R. M. Hoet, J. W. Arends, and R. C. Roovers. 1998. Antibody phage display technology and its applications. Immunotechnology. 4:1-20; Jespers, L. S., A. Roberts, S. M. Mahler, G. Winter, and H. R. Hoogenboom. 1994. Guiding the selection of human antibodies from phage display repertoires to a single epitope of an antigen. Biotechnology (NY) 12:899-903; Rader, C. and C. F. Barbas, III. 1997. Phage display of combinatorial antibody libraries. Curr. Opin. Biotechnol. 8:503-508; Rader, C., D. A. Cheresh, and C. F. Barbas, III. 1998. A phage display approach for rapid antibody humanization: designed combinatorial V gene libraries. Proc. Natl. Acad. Sci. U. S. A 95:8910-8915; Rosok, M. J., D. E. Yelton, L. J. Harris, J. Bajorath, K. E. Hellstrom, I. Hellstrom, G. A. Cruz, K. Kristensson, H. Lin, W. D. Huse, and S. M. Glaser. 1996. A combinatorial library strategy for the rapid humanization of anticarcinoma BR96 Fab. J. Biol. Chem. 271:22611-22618).

In other aspects, human antibodies can be produced using a transgenic animal strain reconstituted with human immunoglobulin loci, e.g., XenoMouse strains. Such strains make it possible to generate fully human antibodies in an animal host (Mendez, M. J., L. L. et al. 1997. Functional transplant of megabase human immunoglobulin loci recapitulates human antibody response in mice. Nat. Gen. 15:146-156). XenoMouse animals, in particular, comprise yeast artificial chromosomes (YACs) containing 66 human heavy-chain and 32 kappa light-chain immunoglobulin genes in their genome, where endogenous heavy-chain and kappa loci are functionally inactivated by targeted deletion (Mendez et al., supra). The mice express human mu, delta, gamma 2, and kappa chains and mouse lambda chains, with a human kappa-to-mouse lambda ratio of 75:1 (Mendez et al., supra). XenoMouse animals have been previously shown to produce human antibodies to protein antigens (Green, L. L., et al. 1994. Antigen-specific human monoclonal antibodies from mice engineered with human Ig heavy and light chain YACs. Nat. Gen. 7:13-21; Mendez et al., supra), and may also respond to T-independent antigens such as polysaccharides. As one example, two or more genetically distinct groups of XenoMouse animals can used to produce antibodies, for example, Xm2a-3 strains, reconstituted with one double YAC containing both heavy- and light-chain genes; and Xm2a-5 strains, reconstituted with two YACs, one with heavy-chain and the other with light-chain genes (Jakobovits, A. 1995. Production of fully human antibodies by transgenic mice. Curr. Opin. Biotechnol. 6:561-566; see, also, Russell ND et al., Production of protective human antipneumococcal antibodies by transgenic mice with human immunoglobulin loci. Infect Immun. 2000 Apr;68(4):1820-6).

Those of skill in the art to which the invention relates will appreciate the terms "diabodies" and "triabodies". These are molecules which comprise a heavy chain variable domain (VH) connected to a light chain variable domain (VL) by a short peptide linker that is too short to allow pairing between the two domains on the same chain. This promotes pairing with the complementary domains of one or more other chains and encourages the formation of dimeric or trimeric molecules with two or more functional antigen binding sites. The resulting antibody molecules may be monospecific or multispecific (e.g., bispecific in the case of diabodies). Such antibody molecules may be created from two or more antibodies using methodology standard in the art to which the invention relates (see, e.g., Todorovska et al. Design and application of diabodies, triabodies, and tetrabodies for cancer targeting. J. Immunol. Methods. 2001 Feb 1;248(1-2):47-66; Holliger P, Prospero T, and Winter G, Diabodies: small bivalent and bispecific antibody fragments, Proc Natl Acad Sci USA, 90, 6444-6448, 1993; and Tomlinson I and Holliger P, Methods for generating multivalent and bispecific antibody fragments, Methods Enzymol, 326, 461-479, 2000).

The production of antibodies may be carried out according to standard methodology in the art. For example, in the case of the production of polyclonal antibodies the methodology described by Bean (Eric S. Bean (2001) Polyclonal Antibodies. In: Basic Methods in Antibody Production and Characterization antibodies. Howard, G, and Bethel D. (ed.), CRC Press, 5:21-50, 2000) may be used. Monoclonal antibodies may be prepared, for example, in accordance with the methodology of Stewart (Sandy J. Stewart (2001) Monoclonal Antibody Production. In: Basic Methods in Antibody Production and Characterization antibodies. Howard, G. and Bethel D. (ed.), CRC Press, 6:51-68, 2000) or in "Monocolonal Antibody Production Techniques and Applications," Lawrence B Schook eds., Marcel Dekker Inc., New York, 1987. Hybridomas may be subcloned, grown, and maintained using standard techniques in the art. For example, they may be grown and maintained *in vitro* in media such as DMEM or RPMI-1640. Alternatively, this may be done *in vivo* as ascites tumours in an animal of choice. Antibodies of use in the invention may also be produced via standard recombinant techniques, see, e.g., Siegel (2002) Siegel DL, Recombinant monoclonal antibody technology, Transfus Clin Biol, 9(1):15-22 2002; Welschof, M., C. Christ, I. Hermes, A. Keller, C. Kleist, and M. Braunagel. 2003. Generation and screening of a modular human scFv expression library from multiple donors. Methods Mol. Biol. 207:103-121). The inventors consider recombinant techniques to be a preferable means of producing antibodies on a commercial scale. Polynucleotides encoding an antibody may be readily identified on the basis of the amino acid sequence of the antibody, the genetic code, and the understood degeneracy therein. Polynucleotides encoding antibodies may be isolated from hybridoma cells, for example, and subsequently characterised using procedures standard in the art. For example, a polynucleotide probe may be designed based on the amino acid sequence of a portion of an antibody and then used to isolate genes encoding the heavy and/or light chains of the antibody. Alternatively, polynucleotides may be generated by standard chemical synthesis methodology, for example, using phosphoramidite and solid phase chemistry. The amino acid sequence of an antibody may be determined using standard methodology; for example, Edman degradation and HPLC or mass spectroscopy analysis, may be used.

It should be appreciated that inasmuch as the disclosure also extends to fragments, as well as modifications of the antibodies specifically referred to herein, nucleic acid encoding the antibodies may be appropriately modified. For example, the coding sequence for heavy-and light-chain constant domains may be replaced with a homologous human domain. Alternatively, the CDR (complementarity determining region or antigen binding site) regions may be transplanted to a homologous human beta-sheet framework. In this way, antibody modifications, such as humanised antibodies may be generated via recombinant techniques. As mentioned herein, antibodies of the disclosure may be produced via standard recombinant procedures. Accordingly, the present disclosure also extends to polynucleotides encoding an antibody, antibody fragment, or modification thereof, constructs comprising same, and host cells comprising said constructs. Polynucleotides in accordance with the disclosure may be DNA, RNA, or cDNA, for example, double stranded or single stranded, sense or antisense sequences, as described herein.

The antibodies may be isolated for example, from culture supernatants, ascites fluid, or serum using standard procedures known in the art to which the invention relates. An example of such techniques is provided herein below. However, as other examples, isolation or purification may occur via one or more of procedures such as affinity chromatography, ion exchange chromatography, interaction chromatography, gel filtration chromatography, thiophilic gel chromatography, chromatofocusing, Protein-A or G Sepharose columns, hydroxyapatite columns, detergent extraction, electrophoresis, osmotic shock treatment, inclusion body purification, ammonium sulphate precipitation, centrifugation with liquid polymers, filtration, and dialysis. A recombinant antibody may be recovered from a transformed host cell, or culture media, or transgenic organism using a variety of techniques that are standard in the art. It will be appreciated that the amino acid sequence of an antibody may be determined using standard methodology, for example, using Edman degradation and HPLC, or mass spectroscopy analysis (M.W. Hunkapiller, R.M. Hewick, W.J. Dreyer, and L.E. Hood. 1983. High-Sequencing with a Gas-Phase Sequenator. Methods Enzymol. 91: 399).

Standard methods can be used to identify amino acid sequences useful for antibody production. Antigenic segments of a polypeptide can be predicted, for example, by Abie Pro 3.0: Peptide Antibody Design (hypertext transfer protocol://world wide web.changbioscience.com/abie/abie.html). More particularly, antigenic segments can be predicted according to the Hopp-Woods scale (Hopp TP, Woods KR. Prediction of protein antigenic determinants from amino acid sequences. Proc Natl Acad Sci USA. 1981 Jun;78(6):3824-8.) and/or the Kyte and Doolittle scale (Kyte J, Doolittle RF. A simple method for displaying the hydropathic character of a protein. J Mol Biol. 1982 May 5;157(1):105-32.). Particular computer programs include MAPAG (Aguilar RC, Retegui LA, Roguin LP Int J Biomed Comput. 1994 Nov-Dec;37(3):225-35); PEOPLE (Alix AJ. : Vaccine. 1999 Sep; 18(3-4):311-4); and HYDRPHIL (E A Mesri et al. J Clin Microbiol. 1990 June; 28(6): 1219-1224). Such epitopes may be conformational specific, in that they may include non-contiguous residues, and may constitute various portions of the predicted antigenic sequences (e.g., portions of any one of SEQ ID NO: 9 to 32 or 34 to 36, see below), or a combination of one or more portions of the predicted antigenic sequences (e.g., combinations of one or more of SEQ ID NO: 9 to 32 or 34 to 36), or one or more of the full length sequences (e.g., one or more of SEQ ID NO: 9 to 32 or 34 to 36). Antigenic sequences can comprise any combination of amino acids or their derivatives that would form a similar 3-D structure (e.g., surface residues) as would be encountered in the native polypeptide.

For Artemin and/or a related ligand, sequence analysis can be used to predict antigenic sequences useful for antibody production. The sequence information for these ligands is widely available (see, e.g., Rosenblad C, Gronborg M, Hansen C, Blom N, Meyer M, Johansen J, Dago L, Kirik D, Patel UA, Lundberg C, Trono D, Bjorklund A, Johansen TE. In vivo protection of nigral dopamine neurons by lentiviral gene transfer of the novel GDNF-family member neublastin/artemin. Mol Cell Neurosci. 2000 Feb;15(2):199-214. Erratum in: Mol Cell Neurosci 2001 Sep;18(3):332-3). In exemplary sequence analysis, Abie Pro 3.0: Peptide Antibody Design (hypertext transfer protocol://world wide web.changbioscience.com/abie/abie.html) shows that are two highly antigenic segments according to the Hopp-Woods scale and the Kyte and Doolittle scale (Hopp TP, Woods KR. Prediction of protein antigenic determinants from amino acid sequences. Proc Natl Acad Sci USA. 1981 Jun;78(6):3824-8; Kyte J, Doolittle RF. A simple method for displaying the hydropathic character of a protein. J Mol Biol. 1982 May5;157(1):105-32).

These antigenic sequences for Artemin include PPPQPSRPAPPPPAPPSALPRGGRAAR AGGPGSRARAAGARG (residues 1-43, relative to the largest processed mature protein of 140 amino acids; SEQ ID NO: 5) and GALRPPPGSRPVSQP (residues 92-106; SEQ ID NO: 6). In addition, 27 antigenic peptides of 14 amino acids are predicted as follows: PPPQPSRPAPPPPA (residues 1-14; SEQ ID NO: 7); PPQPSRPAPPPPAP (residues 2-15; SEQ ID NO: 8); PQPSRPAPPPPAPP (residues 3-16; SEQ ID NO: 9); QPSRPAPPPPAPPS (residues 4-17; SEQ ID NO: 10); PSRPAPPPPAPPSA (residues 5-18; SEQ ID NO: 11); SRPAPPPPAPPSAL (residues 6-19; SEQ ID NO: 12); RPAPPPPAPPSALP (residues 7-20; SEQ ID NO: 13); PAPPPPAPPSALPR (residues 8-21; SEQ ID NO: 14); APPPPAPPSALPRG (residues 9-22; SEQ ID NO: 15); PPPPAPPSALPRGG (residues 10-23; SEQ ID NO: 16); PPPAPPSALPRGGR (residues 11-24; SEQ ID NO: 17); PPAPPSALPRGGRA (residues 12-25; SEQ ID NO: 18); APPSALPRGGRAAR (residues 14-27; SEQ ID NO: 19); PPSALPRGGRAARA (residues 15-28; SEQ ID NO: 20); PSALPRGGRAARAG (residues 16-29; SEQ ID NO: 21); LPRGGRAARAGGPG (residues 19-32; SEQ ID NO: 21); PRGGRAARAGGPGS (residues 20-33; SEQ ID NO: 23); RGGRAARAGGPGSR (residues 21-34; SEQ ID NO: 24); GGRAARAGGPGSRA (residues 22-35; SEQ ID NO: 25); GRAARAGGPGSRAR (residues 23-36; SEQ ID NO: 26); RAARAGGPGSRARA (residues 24-37; SEQ ID NO: 27); ARAGGPGSRARAAG (residues 26-39; SEQ ID NO: 28); AGGPGSRARAAGAR (residues 28-41; SEQ ID NO: 29); GGPGSRARAAGARG (residues 29-43; SEQ ID NO: 30); GALRPPPGSRPVSQ (residues 92-105; SEQ ID NO: 31); ALRPPPGSRPVSQP (residues 93-106; SEQ ID NO: 32).

In addition to therapeutic use, antibodies directed against Artemin and/or a related ligand, may find use in purification or in diagnostic applications. For example, the antibodies may be immobilised on a solid phase. This would aid in purification and/or quantitation of polypeptide or peptide levels in a sample. In the case of diagnostic procedures and purification, it is not necessary for the antibody to have inhibitor activity or decrease expression levels of the ligand. Although, as may be useful for certain applications, antibodies may be modified by labelling with a compound which provides a detectable signal; for example, enzymes, fluorescent agents, and radioisotopes can be used. Those of general skill in the art to which the invention relates will readily identify such suitable labelling systems. Additionally, antibodies may be used as carriers, for example to carry toxins, radionucleotides, isotopes, genes, or other therapeutic molecules to cells or tissues to aid in therapy. Persons of ordinary skill in the art will readily appreciate methods for determining the efficacy of an antibody in preventing, decreasing, or inhibiting cell proliferation, cell survival, cell motility, and/or oncogenicity. However, by way of example, the methodology described elsewhere herein, including one or more of the assays referred to in the examples section, may be used.

As will be appreciated, the antibodies, or antibody fragments, or modifications thereof may be used for the general purposes of detection and purification of Artemin and/or a related ligand. The ligand may be from a natural or artificial source, such as a cell culture. Preferably, the ligand is of human origin. Additionally, as may be useful for certain applications, antibodies may be modified by labelling with a compound which provides a detectable signal. For example, enzymes, fluorescent agents, and radioisotopes can be used. Those of general skill in the art to which the invention relates will readily identify such suitable labelling systems. Thus, in addition to therapeutic use of antibodies directed against ligands, the antibodies may find use in purification of the ligands or in diagnostic applications. For example, antibodies immobilised on a solid phase would aid in purification and/or quantitation of the level of ligand in a sample. Those of ordinary skill in the art to which the invention relates will appreciate techniques by which this may be done. However, by way of example, affinity chromatography using antibodies, antibody fragments, or modifications may be used immobilised on a chromatographic support. In the case of diagnostic and purification procedures, it is not necessary for the antibody to have inhibitory activity.

It will be appreciated that ELISA or similar assays may incorporate both direct and indirect detection means, and that an antibody of the invention, or antibody fragment, or modification thereof, may be used as either capture or detection antibodies. As will be appreciated, one or more of the antibodies may be used in a single assay. For example, where two antibodies of the disclosure do not recognise the same antigenic determinant on Artemin and/or a related ligand, one antibody may be used as a capture antibody and the other antibody may be used as a detection antibody. Alternatively, the antibodies can be used in combination with previously identified antibodies to the ligands. As will be appreciated by persons of ordinary skill in the art, the detection antibody used in an ELISA may be conjugated to a detectable label as herein described. In addition to ELISA, other useful assays include western blots, radioimmunoassays, immunoprecipitation assays, immunocytochemistry, immunohistochemistry, flow cytometry, Luminex® assays, and cytometric bead arrays.

Information of use in diagnosing or generally monitoring the status of a subject may be gained by making a direct comparison of the level of Artemin and/or a related ligand in a test sample, with that of a determined base level or standard. For example, the average serum level of a ligand for a normal subject can be determined (i.e., a subject known not to present a medical disorder as described herein). These concentrations may be used as base levels, with a result above this range being indicative of a medical disorder. Preferably, the level necessary to be indicative of a disorder is a statistically significant increase of those ranges identified as normal. However, even where there is no statistically significant increase, results obtained may provide valuable information about the status of a subject. It should be appreciated that the normal ranges of ligand may differ in different body fluids and tissues. Similarly, normal levels of localised ligand may fall outside the range for normal levels in serum.

It should be appreciated that diagnosis or general determination of a subject's status may be made by comparing the level of Artemin and/or a related ligand present in a test sample against a database of results obtained from a range of other subjects. Instead of utilising a standard or base level concentration of ligand obtained from a number of normal subjects, the base level concentration may be determined from a single subject during a period when they were known not to present a medical disorder, or during a period of an active medical disorder. This may be particularly applicable to cases of ongoing and/or intermittent disease events or disorders where constant monitoring of the subjects status is required. For example, a base level may be determined during a period of remission from the disorder and the diagnostic procedure carried out at various times thereafter to assess status. This may provide valuable information pertaining to progression of a disorder, or help in assessing whether treatment of the disorder is proving successful.

In one aspect of the disclosure, the antibodies can be used for immunohistochemistry-based applications. For example, antibody staining can be used for the diagnosis of abnormal cells, such as those found in tumors, or for the characterization of particular cellular events such as cell proliferation or cell death, or for evaluating the localization and differential expression of proteins, e.g., Artemin and related ligands, in biological tissue. For immunohistochemistry, the antibody-ligand interaction can be visualized by various means. In particular, the antibody can be conjugated to an enzyme, such as peroxidase, which can catalyse a color-producing reaction. Alternatively, the antibody can be tagged to a fluorophore, such as FITC, rhodamine, Texas Red, Alexa Fluor®, or DyLight™ Fluor, which can be viewed by immunofluorescence microscopy. Fluorophor tagging is particularly useful for confocal laser scanning microscopy, which is highly sensitive and can be used to visualise interactions between multiple proteins. As another approach, secondary antibodies can be used to amplify the antibody signal. The secondary antibodies can be conjugated, for example, to biotin or a reporter enzyme such as alkaline phosphatase or horseradish peroxidase, or to fluorescent agents as described in detail herein. For immunohistochemistry, any cells or tissues from a biopsy can be used, or any biological sample as described herein.

As described herein, antibodies produced in accordance with the disclosure may find particular use as therapeutic agents, for example, for preventing, decreasing, or inhibiting cell proliferation, cell survival, cell motility, and/or oncogenicity. In one broad embodiment the disclosure provides a method of blocking the interaction of at least one ligand with one or more receptors, or more broadly, blocking the interaction of a ligand with a binding agent, the method comprising contacting the antibody, antibody fragment, or modification thereof in accordance with the disclosure.

This method may be conducted *in vivo* or *in vitro.*

### Compositions for inhibition of Artemin and/or related ligands

The agents for use in inhibiting Artemin and/or a related ligand (e.g., chemical compounds, such as small molecules, as well as antagonists, antibodies, antisense polynucleotides, and iRNAs) may be used on their own, or in the form of compositions in combination with one or more pharmaceutically acceptable diluents, carriers, and/or excipients. Those skilled in the art to which the invention relates will readily appreciate a variety of pharmaceutically acceptable diluents, carriers, and/or excipients which may be employed in compositions described herein. As will be appreciated, the choice of such diluents, carriers, and/or excipients will be dictated to some extent by the nature of the agent to be used, the intended dosage form of the composition, and the mode of administration. By way of example, in the case of administration of polynucleotides, such as vectors adapted to express antisense or iRNA, suitable carriers include isotonic solutions, water, aqueous saline solution, aqueous dextrose solution, and the like.

In addition to standard diluents, carriers, and/or excipients, a pharmaceutical composition may be formulated with additional constituents, or in such a manner, so as to enhance the activity of the agent or help protect the integrity of the agent. For example, the composition may further comprise adjuvants or constituents which provide protection against degradation, or decrease antigenicity of an agent, upon administration to a subject. Alternatively, the agent may be modified so as to allow for targeting to specific cells, tissues, or tumours.

Agents may be formulated to incorporate a sustained-release system. Inasmuch as this is the case, compositions may include semi-permeable polymer matrices in the form of shaped articles, e.g., films, or microcapsules. Sustained-release matrices include polylactides (U.S. Pat. No. 3,773,919; EP 58,481), copolymers of L-glutamic acid and gamma-ethyl-L-glutamate (Sidman et al., 1983, Biopolymers: 22: 547-56), poly(2-hydroxyethyl methacrylate) (Langer et al., 1981, J. Biomed. Mater. Res.: 15: 267), ethylene vinyl acetate (Langer et al., 1981, J. Biomed. Mater. Res.: 15: 267), or poly-D-(-)-3-hydroxybutyric acid (EP 133,988).

Agents of the disclosure may also be formulated into liposomes. Liposomes comprising the compound may be prepared using techniques known in the art to which the invention relates. By way of example see: DE 3,218,121, EP 52,322, EP 36,676, EP 88,046, EP 143,949, EP 142,641, Japanese Pat. Appln. 83-118008, U.S. Pat. Nos. 4,485,045 and 4,544,545, and EP 102,324. Ordinarily, the liposomes are of the small (from or about 200 to 800 Angstroms) unilamellar type in which the lipid content is greater than about 30 mol percent cholesterol, the selected proportion being adjusted for the most efficacious therapy. Agents of use in the invention may also be pegylated to increase their lifetime.

Additionally, it is contemplated that a composition in accordance with the disclosure may be formulated with other ingredients which may be of benefit to a subject in particular instances. For example, there may be benefit in incorporating, where appropriate, one or more anti-neoplastic agents. Examples of such agents include: alkylating agents, for example, chlorambucil (e.g., Leukeran™), cyclophosphamide (e.g., Endoxan™, Cycloblastin™, Neosar™, Cyclophosphamide™), ifosfamide (e.g., Holoxan™, Ifex™, Mesnex™), thiotepa (e.g., Thioplex™, Thiotepa™); and antimetabolites/S-phase inhibitors, for example, methotrexate sodium (e.g., Folex™, Abitrexate™, Edertrexate™), 5-fluorouracil (e.g., Efudix™, Efudex™), hydroxyurea (e.g., Droxia™, Hydroxyurea, Hydrea™), amsacrine, gemcitabine (e.g., Gemzar™), dacarbazine, thioguanine (e.g., Lanvis™).

Also included are antimetabolites/mitotic poisons, for example, etoposide (Etopophos™, Etoposide, Toposar™), vinblastine (e.g., Velbe™, Velban™), vindestine (e.g., Eldesine™), vinorelbine (e.g., Navelbine™), paclitaxel (e.g., Taxol™); antibiotic-type agents, for example, doxorubicin (e.g., Rubex™), bleomycin (e.g., Blenoxane™), dactinomycin (e.g., Cosmegen™), daunorubicin (e.g., Cerubidin™), mitomycin (e.g., Mutamycin™); hormonal agents, for example, aminoglutethimide (e.g., Cytadren™), anastrozole (e.g., Arimidex™), estramustine (e.g., Estracyt™, Emcyt™), goserelin (e.g., Zoladex™), hexamethylmelanine (e.g., Hexamet™), letrozole (e.g., Femara™), anastrozole (e.g., Arimidex™), and tamoxifen (e.g., Estroxyn™, Genox™, Novaldex™, Soltamox™, Tamofen™). Further included are combinations of any two or more anti-neoplastic agents (for example, Adriamycin/5-fluorouracil/cyclophosphamide (FAC); and cyclophosphamide/methotrexate/5-fluorouracil (CMF)). Particularly useful are combinations that include, for example, at least two or more agents such as cyclophosphamide (e.g., CYTOXAN), methotrexate (e.g., RHEUMATREX), 5-fluorouracil (e.g., ADRUCIL), doxorubicin (e.g., ADRIAMYCIN), and cyclophosphamide (e.g., CYTOXAN). Useful for metastatic disease are agents such as capecitabine (e.g., XELODA), doxorubicin (e.g., ADRIAMYCIN), including its liposomal formulation, gemcitabine (e.g., GEMZAR), the taxanes, including paclitaxel (e.g., TAXOL) and docetaxel (e.g., TAXOTERE), vinorelbine (e.g., NAVELBINE), and trastuzumab (e.g., HERCEPTIN). Persons of ordinary skill in the art to which the invention relates will readily appreciate examples of other agents which may be of benefit. Agents of the disclosure may also be formulated with compounds and agents, other than those specifically mentioned herein, in accordance with accepted pharmaceutical practice.

As will be appreciated, in the case of administration of polynucleotides, they may be packaged into viral delivery systems, which viral systems may themselves be formulated into compositions as herein described. Persons of skill in the art to which the invention relates may appreciate a variety of suitable viral vectors having regard to the nature of the invention described herein. However, by way of example, retroviral vectors, adenoviral vectors, and adeno-associated virus (AAV) can be used. Persons of skill in the art to which the invention relates will readily appreciate methods which may be employed to implement such vectors in the present disclosure.

However, by way of example only: the use of retroviral vectors is reported in Miller et al., 1993, Meth. Enzymol. 217:581-599, and Boesen et al., 1994, Biotherapy 6:291-302; the use of adenoviral vectors is reported for example in Kozarsky and Wilson, 1993, Current Opinion in Genetics and Development 3:499-503; Rosenfeld et al., 1991, Science 252:431-434; Rosenfeld et al., 1992, Cell 68:143-155; Mastrangeli et al., 1993, J. Clin. Invest. 91:225-234; PCT Publication WO 94/12649; and Wang, et al., 1995, Gene Therapy 2:775-783; and, the use of AAV has been reported in Walsh et al., 1993, Proc. Soc. Exp. Biol. Med. 204:289-300; U.S. Patent No. 5,436,146.

In accordance with the mode of administration to be used, and the suitable pharmaceutical excipients, diluents and/or carriers employed, compositions of the disclosure may be adapted into customary dosage forms such as solutions, orally administrable liquids, injectable liquids, tablets, coated tablets, capsules, pills, granules, suppositories, transdermal patches, suspensions, emulsions, sustained release formulations, gels, aerosols, liposomes, powders and immunoliposomes. The dosage form chosen will reflect the mode of administration desired to be used, the disorder to be treated and the nature of the agent to be used. Particularly preferred dosage forms include orally administrable tablets, gels, pills, capsules, semisolids, powders, sustained release formulations, suspensions, elixirs, aerosols, ointments, or solutions for topical administration, and injectable liquids. Skilled persons will readily recognise appropriate dosage forms and formulation methods. Generally, compositions are prepared by contacting or mixing specific agents and ingredients with one another. Then, if necessary, the product is shaped into the desired formulation. By way of example, certain methods of formulating compositions may be found in references such as Gennaro AR: Remington: The Science and Practice of Pharmacy, 20th ed., Lippincott, Williams & Wilkins, 2000.

The amount of an agent in a composition can vary widely depending on the type of composition, size of a unit dosage, kind of carriers, diluents and/or excipients, and other factors well known to those of ordinary skill in the art. In general, the final composition can comprise from 0.0001 percent by weight (% w) to 100% w of the actives of this disclosure, preferably 0.001% w to 10% w, with the remainder being any other active agents present and/or carrier(s), diluent(s) and/or excipient(s).

### Methods of treatment

While the inventors' primary studies have involved breast cancer cells, Artemin and/or related ligands are predicted to also act in the small intestine and kidney; and in the heart, prostate, uterus, normal colon, stomach, skin, trachea, brain, cerebellum, fetal brain, spinal cord, placenta, adipose tissue, cartilage; and also in the thymus. Specifically, these ligands are predicted to act in breast cancer, colon cancer, prostate cancer, endometrial cancer, lung cancer, stomach cancer, liver cancer, among others. Accordingly, the inventors contemplate the inhibition of Artemin and/or a related ligand being applicable to the treatment of a variety of disorders characterised by increased or aberrant cell proliferation, cell survival, cell motility, and/or oncogenicity.

In one embodiment, the disclosure relates to a method of preventing, reducing, or inhibiting cell proliferation, cell survival, and/or cell motility by inhibiting Artemin and/or a related ligand. Preferably, the method is for the treatment of a disorder characterised by aberrant cell proliferation, cell survival, and/or cell motility in subject. These aberrant characteristics may occur in one or more cell type within a subject and can include metastatic disorders. Specific disorders include, for example, cancer (breast cancer, colon cancer, prostate cancer, endometrial cancer, lung cancer, stomach cancer, or liver cancer, for example) and endometriosis. Examples of disorders include cancers such as adenocarcinoma, leukemia, lymphoma, melanoma, myeloma, sarcoma, teratocarcinoma, and cancers of the adrenal gland, bladder, bone, brain, breast, cervix, gall bladder, ganglia, gastrointestinal tract, oesophagus, stomach, intestine, colon, rectum, heart, kidney, liver, lung, bone marrow, muscle, reproductive tract, uterus, ovary, penis, prostate, pancreas, parathyroid, salivary glands, skin, spleen, testis, thymus, and thyroid.

In various embodiments, the disorder is an epithelial tumor of the breast, or endometrium. Regarding breast cancers, these can include epithelial tumours (e.g., from cells lining ducts or lobules) or nonepithelial tumours (e.g., from the supporting stroma), such as angiosarcomas, primary stromal sarcomas, and phyllodes tumor. Breast cancers can also include carcinomas, for example, carcinomas *in situ,* as well as invasive cancers. Carcinoma *in situ* includes proliferation of cancer cells within ducts or lobules and without invasion of stromal tissue. Lobular carcinoma *in situ* (LCIS) includes nonpalpable lesions which can indicate increased risk of subsequent invasive carcinoma in either breast. In breast cancer, invasive carcinoma generally comprises adenocarcinoma, with most comprising infiltrating ductal type carcinoma and the remainder comprising infiltrating lobular carcinoma. Rare forms of breast cancer include medullary, mucinous, and tubular carcinomas. Breast cancer disorders also include Paget's disease of the nipple, and metastatic breast cancer.

Regarding colon cancer, this can generally include cancer of the colon, rectum, and/or anus, and especially, adenocarcinomas, and also carcinomas (e.g., squamous cloacogenic carcinomas), melanomas, lymphomas, and sarcomas. Epidermoid (nonkeratinizing squamous cell or basaloid) carcinomas are also included. The colon cancer may be associated with particular types of polyps or other lesions, for example, tubular adenomas, tubulovillous adenomas (e.g., villoglandular polyps), villous (e.g., papillary) adenomas (with or without adenocarcinoma), hyperplastic polyps, hamartomas, juvenile polyps, polypoid carcinomas, pseudopolyps, lipomas, or leiomyomas. The cancer may be associated with familial polyposis and related conditions such as Gardner's syndrome or Peutz-Jeghers syndrome. The cancer may be associated, for example, with chronic fistulas, irradiated anal skin, leukoplakia, lymphogranuloma venereum, Bowen's disease (intraepithelial carcinoma), condyloma acuminatum, or human papillomavirus. In other aspects, the cancer may be associated with basal cell carcinoma, extramammary Paget's disease, cloacogenic carcinoma, or malignant melanoma. Regarding endometrial cancers, these can include adenocarcinomas and also papillary serous, clear cell, squamous, and mucinous carcinoma. Also included are precancerous conditions such as endometrial hyperplasia. The endometrial cancer may be associated with one or more of obesity, polycystic ovarian syndrome, nulliparity, late menopause, estrogen-producing tumours, anovulation (ovulatory dysfunction), and estrogen therapy without progesterone and hereditary nonpolyposis colorectal cancer (HNPCC) syndrome.

Persons of general skill in the art to which the invention relates may readily appreciate alternative types of disorder which the disclosure may be applicable, especially having regard to the expression of Artemin provided herein. In addition, it will be appreciated by those of general skill in the art to which the invention relates, having regard to the nature of the invention and the results reported herein, that the present invention is applicable to a variety of different animals. Accordingly, the diagnostic cell treatment methods can apply to any animal of interest. In particular, the invention is applicable to mammals, more particularly humans.

Persons of ordinary skill in the art to which the invention relates will appreciate various means and agents for use in inhibiting Artemin and/or a related ligand. By way of example, nucleic acid technology including iRNA, antisense, and triple helix DNA may be employed to inhibit expression. Alternatively antibodies directed against Artemin and/or a related ligand, or functional derivatives of such antibodies may be used. Exemplary agents are described in detail herein. Those agents of use in the invention will preferably exhibit one or more of the following characteristics: 1) the ability to prevent, reduce or inhibit cellular growth; 2) the ability to prevent, reduce or inhibit cell proliferation, cell survival, cell motility, and/or oncogenicity; 3) the ability to prevent, reduce or inhibit expression or activity of Artemin and/or a related ligand; 4) the ability to prevent, decrease, reduce or control metastasis of tumours. Preferably, suitable agents will exhibit two or more of these characteristics.

As shown herein, Artemin is encoded as a cellular factor that is expressed in certain cancer cells, and also by at least one subset of normal adult cells. Therefore, Artemin and related ligands can be considered tumor-associated antigens. Several approaches can be used to target these ligands based on differences in expression and access in normal and cancer cells (reviewed, generally, in Paul, Fundamental Immunology, 1999, Lippincott-Raven Publishers, Philadelphia, PA, Chapter 37). Cancer cells are likely to express tumor-associated antigens at much higher levels and such differences in expression levels between normal and cancer cells can be exploited therapeutically (see, e.g., Brown JP, et al., Quantitative analysis of melanoma-associated antigen p97 in normal and neoplastic tissues. Proc Natl Acad Sci USA 1981;78:539-543). Targeting may also be attained because of better access of antigen-specific effector cells to cancer cells than to normal cells. For example, these antigens expressed on cancer cells may be more available for binding due to incomplete glycosylation (e.g., as in the case of epithelial mucins). For cancer cells, increased expression of MHC molecules may make tumours a direct target for T cells (see, e.g., Uyttenhove C, et al. The expression of mouse gene PIA in testis does not prevent safe induction of cytolytic T cells against a P1A-encoded tumor antigen. Int J Cancer 1997;70:349-356).

Multiple immunotherapeutic strategies involving innate or acquired immunity can be used to control cancer associated with Artemin and/or a related ligand, including (a) local application of a live bacterial vaccine, e.g., BCG; (b) use of cytokines; (c) active immunization against Artemin; (d) passive therapy with antibodies against Artemin and/or a related ligand; and (e) adoptive transfer of effector cells (e.g., T cells). Active immunization against Artemin and/or a related ligand can be used to induce immune responses or passive immunization with a murine monoclonal antibody directed against Artemin and/or a related ligand. For general guidance, see, e.g., Riethmüller G, et al., Monoclonal antibody therapy for resected Dukes' C colorectal cancer: seven-year outcome of a multicenter randomized trial. J Clin Oncol 1998;16:1788-1794; Riethmüller G, et al., Randomized trial of monoclonal antibody for adjuvant therapy of resected Dukes' C colorectal carcinoma. German Cancer Aid 17-1A Study Group. Lancet 1994;343:1177-1183; Herlyn DM, et al., Inhibition of growth of colorectal carcinoma in nude mice by monoclonal antibody. Cancer Res 1980;40:717-721.

For immunization, pure antigens are often ineffective in inducing an acquired (i.e., antigen-specific) immune response unless certain adjuvants are used to stimulate innate immunity. Therefore, numerous approaches are designed to stimulate innate immunity at the site of vaccinations by the use of chemical and/or bacterial agents. Synthetic peptides used in vaccines can be designed for particular MHC haplotypes (see, e.g., Toes RE et al. Peptide vaccination can lead to enhanced tumor growth through specific T-cell tolerance induction. Proc Natl Acad Sci USA 1996;93:7855-7860). Antigenic peptides can be loaded onto heat-shock protein (or as recombinant virus-like particles) to increase the efficacy of immunization. Generally, effective induction of an immune response requires antigen presentation in an environment that provides appropriate help or secondary signals.

Several experimental designs use dendritic cells pulsed with virus-specific or tumor-associated peptides to induce tumor-reactive T cells and rejection of transplanted tumor cells. Dendritic cells can be loaded with synthetic antigenic peptides or recombinant proteins. Dendritic cells can also be loaded with one or more of: native peptides stripped from tumor cell surfaces; tumor-derived, peptide-loaded heat-shock proteins; tumor-derived mRNA; or fused tumor cells (for review, see Shurin MR. Dendritic cells presenting tumor antigen. Cancer Immunol Immunother 1996;43:158-164). One advantage of these strategies is that powerful immunity can be induced to (unique) individually distinct tumor antigens, as well as tumor-associated antigens.

For antigens for Artemin and/or a related ligand, recombinant vaccines can be developed using vaccinia, Listeria, or virus-like particles. In other approaches, genetic vaccination can be used, for example, by injecting naked DNA plasmid constructs, intramuscularly encoding the tumor antigen (see e.g., Donnelly JJ, Ulmer JB, Liu MA. DNA vaccines. Life Sci 1997;60:163-172). GM-CSF may also be used to improve the presentation of the antigen by dendritic cells at the site of injection (see, e.g., Syrengelas AD, Chen TT, Levy R. DNA immunization induces protective immunity against B-cell lymphoma. Nat Med 1996;2:1038-1041). In other approaches, vaccination with anti-idiotypic antibodies can be used.

As further approaches, passive antibody therapy or adoptive transfer of tumor-specific T cells can be used. For example, passive immunization with an antibody to Artemin and/or a related ligand can protect against challenge with tumor cells and can be therapeutic when given soon after challenge with the cancer cells (e.g., see Riethmüller G, et al. Monoclonal antibody therapy for resected Dukes' C colorectal cancer: seven-year outcome of a multicenter randomized trial. J Clin Oncol 1998;16:1788-1794; Riethmüller G, et al. Randomized trial of monoclonal antibody for adjuvant therapy of resected Dukes' C colorectal carcinoma. German Cancer Aid 17-1A Study Group. Lancet 1994;343:1177-1183; Herlyn DM, et al. Inhibition of growth of colorectal carcinoma in nude mice by monoclonal antibody. Cancer Res 1980;40:717-721).

As alternate approaches, anti-idiotypic antibody treatment can be used to induce cancer cells to go into a long-lasting dormant state (see, e.g., Miller RA, Maloney DG, Warnke R, Levy R. Treatment of B-cell lymphoma with monoclonal anti-idiotype antibody. N Engl J Med 1982;306:517-522). In addition, antibodies to tumour cells can be used as carriers for cytokines or cytotoxic agents, such as radiochemicals or natural toxins (see, e.g., Ghetie V, Vitetta E. Immunotoxins in the therapy of cancer: from bench to clinic. Pharmacol Ther 1994;63:209-234; Reisfeld RA, Gillies SD. Recombinant antibody fusion proteins for cancer immunotherapy. Curr Top Microbiol Immunol 1996;213:27-53). The recombinant antibody-cytokine or antibody-toxin fusion proteins may be used to concentrate these agents in the stroma surrounding the tumor cells. As alternatives, bispecific monoclonal antibodies can be engineered to bind effector cells as well as tumor antigens on the cancer cells. Monoclonal antibodies can also be humanized to reduce the stimulation of neutralizing anti-murine antibodies by patients. As still further approaches, adoptive transfer of T cells can be used with longer established tumor loads. T cells that have been isolated from patients can be expanded *in vitro* with IL-2 and then infused into patients who receive IL-2 as well (see, e.g., Smith CA, et al. Adoptive immunotherapy for Epstein-Barr virus-related lymphoma. Leuk Lymphoma 1996;23:213-220).

The efficacy of an agent in inhibiting Artemin and/or a related ligand may be determined having regard to the description of the invention herein and known methodology. For example, efficacy of agents may be determined by observing their ability to prevent, reduce, or inhibit expression of the ligand or one or more of the functional effects of the ligand. By way of particular example, the affect of the agent on one or more of cellular invasion, cellular migration, the level of gene transcription, and genes responsive to Artemin and/or a related ligand may be studied. Such studies may be conducted in *vitro* or in *vivo.*

The assays described herein after under the heading "Examples" may be used to determine the suitability of an agent in accordance with the invention. Specifically, RT-PCR and Northern blot analysis can be used to detect expression at the mRNA level, and Western blotting and direct or indirect immunostaining can be used to detect the expression at the protein level. To detect activity, cell-based assays for cell proliferation, cell survival, cell motility, and/or oncogenicity can be used. Regarding inhibition of metastasis, an *in vivo* assay may be used, as described, for example, in Fidler, I. J. (1973) Nat. New Biol. 242, 148-149; and Price J. E. The biology of cancer metastasis. Prog. Clin. Biol. Res., 354A: 237-255, 1990, or Kerbel R. S. What is the optimal rodent model for anti-tumor drug testing? Cancer Metastasis Rev., 17: 301-304, 1998; Killion J. J., Radinsky R., Fidler I. J. Orthotopic models are necessary to predict therapy of transplantable tumours in mice. Cancer Metastasis Rev., 17: 279-284, 1998; and Price J. E. Analyzing the metastatic phenotype. J. Cell. Biochem., 56: 16-22, 1994.

### Administration routes/regimes

The inventors contemplate administration of any of the agents or compositions of the disclosure by any means capable of delivering the desired activity (e.g., inhibition of Artemin and/or a related ligand) to a target site within the body of a subject. A target site may be any site within the body which may have or be susceptible to a disorder, and may include one or more cells, tissues or a specific tumor. For example, administration may include parenteral administration routes, systemic administration routes, oral and topical administration. Administration may be by way of injection, subcutaneous, intraorbital, ophthalmic, intraspinal, intracisternal, topical, infusion (using, e.g., slow release devices or minipumps such as osmotic pumps or skin patches), implant, aerosol, inhalation, scarification, intraperitoneal, intracapsular, intramuscular, intratumoral, intranasal, oral, buccal, transdermal, pulmonary, rectal or vaginal delivery. As will be appreciated, the administration route chosen may be dependent on the position of the target site within the body of a subject, as well as the nature of the agent (e.g., peptide, polypeptide, polynucleotide, or antibody) or composition being used.

In a specific embodiment, in the case of polynucleotides, they may be administered for example by transfection using defective or attenuated retroviral or other viral vectors (see e.g., U.S. Patent No. 4,980,286), or by direct injection of naked DNA, or by use of microparticle bombardment (e.g., a gene gun; Biolistic, DuPont); by coating with lipids or cell-surface receptors or transfecting agents; encapsulation in liposomes, microparticles, or microcapsules; by linkage to a peptide which is known to enter the nucleus; or by administering it in linkage to a ligand subject to receptor-mediated endocytosis (see, e.g., Wu and Wu, 1987, J. Biol. Chem. 262:4429-4432), which can be used to target cell types specifically expressing the receptors, and the like. In addition, nucleic acid-ligand complexes can be formed in which the ligand comprises a fusogenic viral peptide to disrupt endosomes, allowing the nucleic acid molecules to avoid lysosomal degradation.

In yet another embodiment, the polynucleotides can be targeted *in vivo* for cell specific uptake and expression, by targeting a specific receptor, as described for example in WO 92/06180 dated April 16, 1992 (Wu et al.); WO 92/22635 dated December 23, 1992 (Wilson et al.); WO 92/20316 dated November 26, 1992 (Findeis et al.); WO 93/14188 dated July 22, 1993 (Clarke et al.); and, WO 93/20221 dated October 14, 1993 (Young). Alternatively, the polynucleotides can be introduced intracellularly and incorporated within host cell DNA for expression, by homologous recombination Koller and Smithies, 1989, Proc. Natl. Acad. Sci. USA 86:8932-8935; Zijlstra et al., 1989, Nature 342:435-438. Cells into which polynucleotides can be introduced for purposes of the present invention encompass any desired, available cell type. The appropriate cell type will depend on the nature of the disorder to be treated. However, by way of example, the polynucleotide can be introduced to a cancer cell.

As will be appreciated, the dose of an agent or composition administered, the period of administration, and the general administration regime may differ between subjects depending on such variables as the nature of the condition to be treated, severity of symptoms of a subject, the size of any tumour to be treated, the target site to be treated, the mode of administration chosen, and the age, sex and/or general health of a subject. Persons of average skill in the art to which the invention relates will readily appreciate or be able to determine appropriate administration regimes having regard to such factors. It should be appreciated that administration may include a single daily dose or administration of a number of discrete divided doses as may be appropriate. The inventors also contemplate the administration regimes which combine different modes or routes of administration. For example, intratumoural injection and systemic administration could be combined.

It should be appreciated that a method of the disclosure may comprise further steps such as the administration of additional agents or compositions which may be beneficial to a subject having regard to the condition to be treated. For example, other agents of use in treating proliferative disorders (such as the anti-neoplastic agents mentioned above) could be administered. It should be appreciated that such additional agents and compositions may be administered concurrently with the agents and compositions of the disclosure, or in a sequential manner. For example, the additional agents or compositions could be administered before or after administration of the agents or compositions of the disclosure. It should be appreciated in relation to sequential delivery of agents or compositions, that sequential administration of one agent or composition after the other need not occur immediately, although this may be preferable. There may be a time delay between delivery of the agents or compositions. The period of the delay will depend on factors such as the condition to be treated and the nature of the compositions or agents to be delivered. However, by way of example, the inventors contemplate periods of between hours to several days or months.

### Diagnostic methods and compositions

In one embodiment, the disclosure relates to use of one or more reagents of the disclosure in a method of diagnosing a disorder associated with cell proliferation, cell survival, cell motility, and/or oncogenicity. Preferably, the method is for the diagnosis of a disorder characterised by aberrant cell proliferation, cell survival, cell motility, and/or oncogenicity in a subject. This aberrant cell growth and/or proliferation may occur in one or more cell type within a subject and can include metastatic or other disorders. These disorders can include, for example, cancer (breast cancer, colon cancer, prostate cancer, endometrial cancer, lung cancer, stomach cancer, liver cancer, for example) and endometriosis. Exemplary disorders are described in detail herein.

In accordance with the disclosure, antibodies which specifically bind Artemin and/or a related ligand may be used for the diagnosis of conditions or disorders characterized by altered expression, or in assays to monitor patients being treated with inhibitors to one or more of these ligands. The antibodies useful for diagnostic purposes may be prepared in the same manner as those described above. Diagnostic assays include methods which utilize the antibody and a label to detect the corresponding peptide or polypeptide in human body fluids or extracts of cells or tissues. The antibodies may be used with or without modification, and may be labeled by joining them, either covalently or non-covalently, with a reporter molecule. A wide variety of reporter molecules which are known in the art may be used, several of which are described herein.

A variety of protocols, including ELISA, RIA, and FACS, are known in the art and provide a basis for diagnosing altered or abnormal levels of expression for Artemin and/or a related ligand. Normal or standard values for expression are established by combining body fluids or cell extracts taken from normal mammalian subjects, preferably human, with antibody under conditions suitable for complex formation. The amount of standard complex formation may be quantified by various methods, but preferably by photometric means. Quantities expressed in subject, control, and disease, samples from biopsied tissues are compared with the standard values. Deviation between standard and subject values establishes the parameters for diagnosing disorders.

In another embodiment of the disclosure, the polynucleotides for Artemin or a related ligand may be used for diagnostic purposes. The polynucleotides which may be used include oligonucleotides, complementary RNA and DNA molecules, and PNAs. The polynucleotides may be used to detect and quantitate gene expression in biopsied tissues in which expression may be correlated with disorders. The diagnostic assay may be used to distinguish between absence, presence, and altered expression, and to monitor regulation of levels during therapeutic intervention.

In one aspect, nucleic acid hybridization can be performed with probes or primers (e.g., PCR primers) which are capable of detecting polynucleotide sequences for Artemin or a related ligand, including genomic sequences and closely related molecules. The specificity of the probe or primer, whether it is made from a highly specific region, e.g., 10 unique nucleotides in the 5' regulatory region, or a less specific region, e.g., especially in the 3' coding region, and the stringency of the hybridization or amplification (maximal, high, intermediate, or low) will determine whether the probe or primer identifies only naturally occurring sequences, alleles, or related sequences. Probes and primers may also be used for the detection of related sequences, and should preferably contain at least 50% of the nucleotides from any native sequence. The hybridization probes of the subject invention may be DNA or RNA and may derived from the nucleotide sequence of the invention, e.g., SEQ ID NO: 35 to 171, or complements, or modified sequences of SEQ_ID NO: 35 to 171, or from genomic sequences including promoter, enhancer elements, and introns of the naturally occurring sequences.

Means for producing specific hybridization probes for DNAs for Artemin or a related ligand include the cloning of nucleic acid sequences or modified sequences into vectors for the production of mRNA probes. Such vectors are known in the art, commercially available, and may be used to synthesize RNA probes *in vitro* by means of the addition of the appropriate RNA polymerases and the appropriate labeled nucleotides. Hybridization probes may be labeled by a variety of reporter groups, for example, radionuclides such as ³²P or ³⁵S, or enzymatic labels, such as alkaline phosphatase coupled to the probe via avidin/biotin coupling systems, and the like.

Polynucleotide sequences for Artemin or a related ligand may be used for the diagnosis of disorders which are associated with either increased or decreased expression. The polynucleotide sequences may be used in Southern or northern analysis; dot blot or other membrane-based technologies; in PCR technologies; or in dipstick, pin, ELISA assays; or microarrays utilizing fluids or tissues from patient biopsies to detect altered expression. Such qualitative or quantitative methods are well known in the art.

In a particular aspect, the nucleotide sequences for Artemin or a related ligand may be useful in assays that detect activation or induction of various cancers, particularly those mentioned above. The nucleotide sequences may be labeled by standard methods, and added to a fluid or tissue sample from a patient under conditions suitable for the formation of hybridization complexes. After a suitable incubation period, the sample is washed and the signal is quantitated and compared with a standard value. If the amount of signal in the biopsied or extracted sample is significantly altered from that of a comparable control sample, the nucleotide sequences have hybridized with nucleotide sequences in the sample, and the presence of altered levels of nucleotide sequences in the sample indicates the presence of the associated disorder. Such assays may also be used to evaluate the efficacy of a particular therapeutic treatment regimen in animal studies, in clinical trials, or in monitoring the treatment of an individual patient.

In order to provide a basis for the diagnosis of a disorder associated with expression of Artemin and/or a related ligand, a normal or standard profile for expression is established. This may be accomplished by combining body fluids or cell extracts taken from normal subjects, either animal or human, with a sequence, or a modification thereof, for Artemin or a related ligand, under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for a disorder. Deviation between standard and subject values is used to establish the presence of the disorder.

Once the disorder is diagnosed and a treatment protocol is initiated, hybridization assays may be repeated on a regular basis to evaluate whether the level of expression in the patient begins to approximate that which is observed in the normal patient. The results obtained from successive assays may be used to show the efficacy of treatment over a period ranging from several days to months. With respect to cancer, the presence of a relatively high amount of transcript in biopsied tissue from an individual may indicate a predisposition for the development of the disorder, or may provide a means for detecting the disorder prior to the appearance of actual clinical symptoms. A more definitive diagnosis of this type may allow health professionals to employ preventative measures or aggressive treatment earlier thereby preventing the development or further progression of the cancer.

Additional diagnostic uses for oligonucleotides of the disclosure may involve the use of PCR. Such oligomers may be chemically synthesized, generated enzymatically, or produced *in vitro.* Oligomers will preferably consist of two nucleotide sequences, one with sense orientation (5'.fwdarw.3') and another with antisense orientation (3'.fwdarw.5'), employed under optimized conditions for identification of a specific gene or condition. The same two oligomers, nested sets of oligomers, or even a degenerate pool of oligomers may be employed under less stringent conditions for detection and/or quantitation of closely related DNA or RNA sequences.

Methods which may also be used to quantitate the expression of Artemin or a related ligand include radiolabeling or biotinylating nucleotides, coamplification of a control nucleic acid, and standard curves onto which the experimental results are interpolated (Melby, P. C. et al. (1993) J. Immunol. Methods, 159:235-244; Duplaa, C. et al. (1993) Anal. Biochem. 229-236). The speed of quantitation of multiple samples may be accelerated by running the assay in an ELISA format where the oligomer of interest is presented in various dilutions and a spectrophotometric or colorimetric response gives rapid quantitation.

In further embodiments of the disclosure, oligonucleotides or longer fragments derived from any of the polynucleotide sequences described herein may be used as targets in a microarray. The microarray can be used to monitor the expression level of large numbers of genes simultaneously (e.g., to produce a transcript image), and to identify genetic variants, mutations and polymorphisms. This information may be used to determine gene function, to understand the genetic basis of the disorder, to diagnose the disorder, and to develop and monitor the activities of therapeutic agents. In one embodiment, the microarray is prepared and used according to the methods known in the art such as those described in PCT application WO 95/11995 (Chee et al.), Lockhart, D. J. et al. (1996; Nat. Biotech. 14: 1675-1680) and Schena, M. et al. (1996; Proc. Natl. Acad. Sci. 93: 10614-10619).

The microarray is preferably composed of a large number of unique, single stranded nucleic acid sequences, usually either synthetic antisense oligonucleotides or fragments of cDNAs, fixed to a solid support. The oligonucleotides are preferably about 6 to 60 nucleotides in length, more preferably about 15 to 30 nucleotides in length, and most preferably about 20 to 25 nucleotides in length. For a certain type of microarray, it may be preferable to use oligonucleotides which are 7 to 10 nucleotides in length. The microarray may contain oligonucleotides which cover the known 5' or 3' sequence, or may contain sequential oligonucleotides which cover the full length sequence; or unique oligonucleotides selected from particular areas along the length of the sequence.

Polynucleotides used in the microarray may be oligonucleotides that are specific to a gene or genes of interest in which at least a fragment of the sequence is known or that are specific to one or more unidentified cDNAs which are common to a particular cell or tissue type or to a normal, developmental, or disease state. In certain situations, it may be appropriate to use pairs of oligonucleotides on a microarray. The pairs will be identical, except for one nucleotide preferably located in the centre of the sequence. The second oligonucleotide in the pair (mismatched by one) serves as a control. The number of oligonucleotide pairs may range from 1 to 1,000,000.

In order to produce oligonucleotides to a known sequence for a microarray, the gene of interest is examined using a computer algorithm which starts at the 5' or more preferably at the 3' end of the nucleotide sequence. The algorithm identifies oligomers of defined length that are unique to the gene, have a GC content within a range suitable for hybridization, and lack predicted secondary structure that may interfere with hybridization. In one aspect, the oligomers are synthesized at designated areas on a substrate using a light-directed chemical process. The substrate may be paper, nylon or any other type of membrane, filter, chip, glass slide, or any other suitable solid support.

In one aspect, the oligonucleotides may be synthesized on the surface of the substrate by using a chemical coupling procedure and an ink jet application apparatus, such as that described in PCT application WO 95/251116 (Baldeschweiler et al.). In another aspect, a gridded array analogous to a dot or slot blot (e.g., HYBRIDOT apparatus, Life Technologies) may be used to arrange and link cDNA fragments or oligonucleotides to the surface of a substrate using a vacuum system, thermal, UV, mechanical or chemical bonding procedures. In yet another aspect, an array may be produced by hand or by using available devices, materials, and machines (including multichannel pipettors or robotic instruments; Brinkmann, Westbury, NY) and may include about 8, 24, 96, 384, 1536 or 6144 oligonucleotides, or any other multiple from 2 to 1,000,000, which lends itself to the efficient use of commercially available instrumentation.

In order to conduct sample analysis using the microarrays, polynucleotides are extracted from a biological sample. The biological samples may be obtained from any bodily fluid (e.g., blood, urine, saliva, phlegm, gastric juices, etc.), cultured cells, biopsies, or other tissue preparations. To produce probes, the polynucleotides extracted from the sample are used to produce nucleic acid sequences which are complementary to the oligonucleotides on the microarray. If the microarray consists of cDNAs, antisense RNAs are appropriate probes. Therefore, in one aspect, mRNA is used to produce cDNA which, in turn and in the presence of fluorescent nucleotides, is used to produce fragment or oligonucleotide antisense RNA probes. These fluorescently labeled probes are incubated with the microarray so that the probe sequences hybridize to the cDNA oligonucleotides of the microarray. In another aspect, nucleic acid sequences used as probes can include polynucleotides, fragments, and complementary or antisense sequences produced using restriction enzymes, PCR technologies, and oligolabeling kits (Amersham Pharmacia Biotech), which are well known in the area of hybridization technology.

Incubation conditions are adjusted so that hybridization occurs with precise complementary matches or with various degrees of less complementarity. After removal of non-hybridized probes, a scanner is used to determine the levels and patterns of fluorescence. The scanned images are examined to determine degree of complementarity and the relative abundance of each oligonucleotide sequence on the microarray. A detection system may be used to measure the absence, presence, and amount of hybridization for all of the distinct sequences simultaneously. This data may be used for large scale correlation studies or functional analysis of the sequences, mutations, variants, or polymorphisms among samples (Heller, R. A. et al., (1997) Proc. Natl. Acad. Sci. 94:2150-55).

In another embodiment of the disclosure, the nucleic acid sequences of the disclosure may be used to generate hybridization probes which are useful for mapping the naturally occurring genomic sequence. The sequences may be mapped to a particular chromosome, to a specific region of a chromosome, or to artificial chromosome constructions, such as human artificial chromosomes (HACs), yeast artificial chromosomes (YACs), bacterial artificial chromosomes (BACs), bacterial PI constructions or single chromosome cDNA libraries (e.g., Price, C. M. (1993) Blood Rev. 7:127-134; Trask, B. J. (1991) Trends Genet. 7:149-154).

Fluorescent *in situ* hybridization (FISH as described, e.g., in Verma et al. (1988) Human Chromosomes: A Manual of Basic Techniques, Pergamon Press, New York, NY) may be correlated with other physical chromosome mapping techniques and genetic map data. Examples of genetic map data can be found in various scientific journals or at the Online Mendelian Inheritance in Man (OMIM) site. Correlation between the location of the gene for Artemin or a related ligand on a physical chromosomal map and a specific disorder, or predisposition to a specific disorder, may help delimit the region of DNA associated with that disorder. The nucleotide sequences of the disclosure may be used to detect differences in gene sequences between normal, carrier, and affected individuals.

*In situ* hybridization of chromosomal preparations and physical mapping techniques, linkage analysis using established chromosomal markers, may be used to extend genetic maps. Often the placement of a gene on the chromosome of another mammalian species, such as mouse, may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once the disorder has been crudely localized by genetic linkage to a particular genomic region, for example, AT to 11q22-23 (e.g., Gatti, R. A. et al. (1988) Nature 336:577-580), any sequences mapping to that area may represent associated or regulatory genes for further investigation.

The nucleotide sequence may also be used to detect differences in the chromosomal location due to translocation, inversion, etc., among normal, carrier, and affected individuals.

In another embodiment of the disclosure, the antibodies may be used for imuunohistochemistry-based applications. In accordance with standard immunohistochemistry techniques, thin slices (e.g., about 4-40 µm) can be taken from the tissue of interest, or the tissue can be used whole. The slicing can be accomplished through the use of a microtome, and slices can be mounted on slides. The tissue can then be treated to rupture the cell membranes, e.g., using detergent such as Triton X-100. Additional unmasking steps can also be used, as well as blocking steps to minimize non-specific binding. For direct immunohistochemistry, the labeled antibody of interest (e.g. FITC conjugated antiserum) is used to bind the antigen in the tissue sections. For indirect immunohistochemistry, an unlabeled primary antibody is used to bind to the tissue antigen, and a labeled secondary antibody is used to react with the primary antibody.

In another embodiment , Artemin or a related ligand, or functional or immunogenic fragments or modifications thereof, can be used for screening libraries of compounds in any of a variety of drug screening techniques. The fragment employed in such screening may be free in solution, affixed to a solid support, borne on a cell surface, or located intracellularly. The formation of binding complexes, between the polypeptide or peptide and the agent being tested, may be measured. In a further embodiment, one may use competitive drug screening assays in which neutralizing antibodies capable of binding Artemin or a related ligand specifically compete with a test compound for binding to the polypeptide or peptide. In this same manner, the antibodies can be used to detect the presence of any amino acid sequence which shares one or more antigenic determinants with Artemin and/or a related ligand.

Another technique for drug screening which may be used provides for high throughput screening of compounds having suitable binding affinity to the protein of interest as described in WO 84/03564. In this method, as applied to Artemin or a related ligand, large numbers of different small test compounds are synthesized on a solid substrate, such as plastic pins or some other surface. The test compounds are reacted with the polypeptide, or modifications thereof, and washed. Bound polypeptide or peptide is then detected by methods well known in the art. Purified polypeptide or peptide can also be coated directly onto plates for use in the aforementioned drug screening techniques. Alternatively, non-neutralizing antibodies can be used to capture the polypeptide, or a modification thereof, and immobilize it on a solid support.

In additional embodiments of the disclosure, the nucleotide sequences for Artemin or a related ligand may be used in any molecular biology techniques that have yet to be developed, provided the new techniques rely on properties of nucleotide sequences that are currently known, including, but not limited to, such properties as the triplet genetic code and specific base pair interactions.

### Kits for treatment or diagnosis

The agents and compositions of the disclosure may be used in kits suitable for controlling or inhibiting Artemin and/or a related ligand, or for the treatment of a disorder as defined herein. In other aspects, the agents and compositions may be used in diagnostic kits. Kits can comprise at least one agent of the disclosure in a suitable container. The agents may be formulated suitable for direct administration to a subject for example, as agents or pharmaceutical compositions. Alternatively, the kit may comprise one or more agents in one container and pharmaceutical diluents, carriers and/or excipients in another; the contents of each container being mixed together prior to administration. The kit may also comprise additional agents and compositions in further separate containers as may be necessary for a particular application. Any container suitable for storing and/or administering an agent or composition may be used in a kit of the disclosure.

Suitable containers will be appreciated by persons skilled in the art. By way of example, such containers include vials and syringes. The containers may be suitably sterilised and hermetically sealed. Further, kits can also comprise instructions for the use and administration of the components of the kit. The invention is further elucidated with reference to the examples below.

### EXAMPLES

The examples described herein are for purposes of illustrating embodiments of the invention. Other embodiments, methods, and types of analyses are within the scope of persons of ordinary skill in the molecular diagnostic arts and need not be described in detail herein.

### EXAMPLE 1: MATERIALS AND METHODS

### Cell culture

All carcinoma cell lines for lung cancer (A549), stomach cancer (AGS), colon cancer (Colo320DM and DLD-1), prostate cancer (DU145 and PC3), pancreas cancer (BxPC3), endometrial cancer (RL95-2 and AN3), breast cancer (MCF-7, T47D, BT549, and MDA-231), and liver cancer (HepG2) were obtained from the ATCC. All cells were cultured in conditions as recommended, except that the MCF-7 cell line was cultured in RPMI 1640 medium supplemented with 10% heat-inactivated foetal bovine serum (FBS), 100 units/ml penicillin, 100 µg/ml streptomycin, and 2 mM L-glutamine in a humidified incubator in 5% CO₂ at 37°C.

### Plasmid constructs

To construct the Artemin expression plasmid, the *Bam*HI and *Bgl*II fragment containing the coding sequence for human Artemin transcript variant 4 (NM_057090) from IMAGE cDNA clone 5453642 was subcloned into pCMV6-XL4 at *Bgl*II site. The resultant plasmid was designated pCMV6-XL4-ARTN. In order to construct Artemin expression plasmid for stable transfection, two oligos, 5'-atcGCCGCCACCATGGaacttggacttggaggcc tctccacgctgtcccactgcccctggc-3' (forward, Kozak sequence in upper case; SEQ ID NO: 48) and 5'-ctaggccaggggcagtgggacagcgtggagaggcctccaagtccaagttccatggcggcggcgat-3' (reverse; SEQ ID NO: 49) were annealed to form the 5' sequence of human Artemin with a Kozak sequence at its 5' end, and an *Avr*II overhang at its 3' end. These annealed oligos were used together with the *Avr*II/*Age*I fragment released from pCMV6-XL4-ARTN, containing the rest of the coding sequence for human Artemin, for cloning into the pIRESneo3 vector between *EcoRV* and *Age*I sites. The resultant plasmid was designated pIRESneo3-ARTN.

For transient transfection, the *Eco*RV/*Eco*RI fragment of pIRESneo3-ARTN was subcloned into the pCMV vector to obtain pCMV-ARTN. To visualize the knock-down efficiency of siRNA, the Artemin cDNA was inserted into the 3'UTR of an EGFP expression vector plasmid. Briefly, the *Eco*RI/*Kpn*I fragment containing the full cDNA of Artemin was released from the pCMV6-XL4-ARTN plasmid and subcloned into the pEGFP-C1 vector between the *Eco*RI/*Kpn*I sites after filling-in of both *Eco*RI sites of the insert and vector. A stop codon was created after ligation of the two filled-in *Eco*RI ends, so that only the EGFP will be expressed with the sequence of Artemin situated in the 3'-UTR. For convenience, this plasmid was designated as pEGFP-ARTN.

To design siRNA oligonucleotides to target Artemin, a DNA sequence, AA(N₁₉), was selected as AACTGGCCTGTACTCACTCAT (SEQ ID NO: 45). This sequence is common for all four Artemin transcript variants. A BLAST search against the human genome sequence showed that only the Artemin gene would be targeted. A pair of oligonucleotides, 5'-gatccgctggcctgtactcactcatttcaagagaatgagtgagtacaggccagttttttggaaa-3' (forward; SEQ ID NO: 50) and 5'-agcttttccaaaaaactggcctgtactcactcattctcttgaaatgagtgagtcaggccagcg-3' (reverse; SEQ ID NO: 51) were cloned into the pSilencer 2.1-U6 hygro vector (Ambion) according to the manufacturer's instructions. The resultant plasmid was designated pSilencer-ARTN. As a negative control, pSilencer-CK was used to encode siRNA having no significant sequence similarity to human gene sequences (Ambion).

The Glutathione S-transferase (GST) Gene Fusion System from Amersham Biosciences was used to produce recombinant Artemin protein in *E. coli.* The human Artemin cDNA fragment coding for the mature C-terminal peptide of 113 amino acids was cloned into the pGEX-4T1 vector (Amersham Biosciences) between the 5' *Bam*HI and 3' *Eco*RI recognition sites to generate plasmid pGEX-4T1-ARTN. This was used for the expression of GST fusion proteins in *E. coli.*

The *Bam*HI fragment of pGEX-4T1-ARTN was subcloned into pQE30 vector (Qiagen) in frame with the N-terminal His tag coding sequence. The resultant construct, pQE30-ARTN, encodes an N-terminal peptide of MRGSHHHHHHGS (SEQ ID NO:33) followed by the mature Artemin protein comprising 113 amino acids. For each construct, the insert was verified by DNA sequencing.

### Establishing stable cell lines expressing Artemin

Cells were stably transfected with the pIRESneo3-ARTN plasmid using Saint-Mix transfection reagent (Synvolux Therapeutics B.V., the Netherlands). As a control, cells were also stably transfected with the empty vector pIRESneo3. Cell clones were selected by addition of G418 (Bio-Rad Laboratories, CA) in the medium. Transfected cell lines were generated as pools of positive cell clones. The overexpression of Artemin in established stables was confirmed by RT-PCR and Western blotting.

### Establishing Artemin depleted cell lines by plasmid-based siRNA,.

To test the efficiency of Artemin specific siRNA, assays were performed using the pSilencer-ARTN plasmid encoding siRNA to target the Artemin transcript or the negative control siRNA plasmid pSilencer-CK, together with the pEGFP-C1 vector (EGFP) or the pEGFP-ARTN plasmid in which the Artemin cDNA was inserted within the 3'UTR of EGFP coding sequence. Plasmids were transiently co-transfected into MCF-7 cells. After 24h of transfection, the expression of EGFP was visualised with a UV-visible fluorescence microscope.

To establish Artemin depleted cell lines, the pSilencer-ARTN plasmid or the pSilencer-CK plasmid was stably transfected into cells. Cell clones were selected by addition of hygromycin in the medium. Transfected cell lines were generated as pools of positive cell clones. The expression of Artemin in the stable clones was determined by RT-PCR and Western blotting.

### Preparation of total RNA

Total RNA was isolated from cultured cells with Trizol reagent (Invitrogen) at 1 ml/10 cm² according to the manufacturer's instructions. RNA was resuspended in diethyl pyrocarbonate-treated nuclease-free water. RNA samples were further treated with DNase I for 30 min at 37°C. The reaction was stopped by addition of 25 mM EDTA and incubation at 65°C for 15 min. RNA samples were then purified by extraction in phenol/chloroform (pH 5.2, phenol:chloroform:isoamyl alcohol at 25:24:1) followed by an additional chloroform extraction and ethanol precipitation. Quantification and purity of the RNA was assessed by A₂₆₀/A₂₈₀ absorption, and RNA quality was assessed by agarose gel electrophoresis. RNA samples with ratios of A₂₆₀/A₂₈₀ greater than 1.6 were stored at - 80°C for further analysis.

### Reverse transcription-PCR

One-step reverse transcription (RT)-PCR kit (Qiagen) was used to determine the presence of GDNF ligands (Artemin and Persephin), GFRα1-4 and RET using gene-specific primers (Table 1, see further below). For RT-PCR, the following procedure was employed. To start, 1 µg of total RNA was diluted to 0.1 µg/µl to minimize the variation of sample handling. This dilution was treated by DNase I for 15 min, followed by inactivation of DNase by adding EDTA to 5 mM and heating to 70°C for 15 min. The DNase-treated RNA was then mixed with a master cocktail containing RT-PCR buffer, sense and antisense primers, dNTPs, RNase inhibitor, an enzyme mixture containing reverse transcriptase (Omniscript and Sensiscript) and HotStart Taq DNA polymerase at the concentrations recommended by the manufacturer to a final volume of 50 µl.

The temperature-cycle protocol included: 60 min at 50°C for the reverse transcription reaction, followed by denaturation and activation of HotStart DNA polymerase for 15 min at 95°C, and PCR amplification for 20 sec at 95°C, 30 sec at 54-62°C, and 1 min at 72°C for 30 cycles. A final extension for 5 min at 72°C was performed at the end of the cycles. β-actin was similarly amplified by RT-PCR using 0.2 µg of total RNA as an internal control. Ten microlitres of each of the RT-PCR product was fractionated on 1% agarose gels. The identity of RT-PCR product was confirmed by the size, restriction enzyme digestion, and DNA sequencing.

### mRNA expression assays

For mRNA expression studies, a cDNA panel (Primgen) consisting of 10 ng of first-strand cDNA from various human tissues was screened by PCR with the Artemin specific primers (Table 1, see further below). As recommended by the manufacturer, human beta-2-microglobulin gene was used as the cDNA input control. The expression of Artemin in a variety of cancer cell lines was also determined by RT-PCR. The cell lines included A549 human lung carcinoma cells; AGS human stomach cancer cells; Colo320DM and DLD-1 neuroendocrine colon carcinoma cells; DU145 and PC3 human prostate cancer cells; BxPC3 pancreatic cancer; RL95-2 endometrial carcinoma cells; HepG2 human liver cancer cells; and T47D, BT459, MCF-7 and MDA-231 human breast cancer cells.

### Immunoblotting

Cells were washed twice with ice-cold phosphate-buffered saline (PBS) and lysed at 4°C in lysis buffer (20 mM Tris•HCl, pH 7.4, 150 mM NaCl, 1 mM EDTA, 1 mM EGTA, 1% Triton^{®} X-100, 1% Nonidet P-40, 1 µg/ml protease inhibitor cocktail (GE Healthcare) and 0.1 mM PMSF). The lysates were next sonicated and then cleared by centrifugation at 15,000 x g for 15 min at 4°C. SDS-polyacrylamide gel electrophoresis (PAGE) sample buffer (50 mM Tris•HCl, pH 6.8; 2% SDS; 2% β-mercaptoethanol, and bromophenol blue) was added to each sample and the samples were boiled for 5 min. Samples were subjected to discontinuous SDS-PAGE with a 12.5% resolving gel and transferred to nitrocellulose membranes (Hybond™ C-extra) using standard electroblotting procedures. Membranes were blocked with 5% non-fat dry milk in PBS with 0.1% Tween^{®} 20 (PBST) for 1 h at room temperature. The blots were then immunolabelled with primary antibodies in PBST containing 1% non-fat dry milk at 4°C overnight.

After incubation with appropriate secondary antibodies at room temperature, immunolabelling was detected by ECL plus™ chemiluminescence as described by the manufacturer (GE Healthcare). Blots were stripped and reprobed with monoclonal antibody against β-actin to ensure equal loading of the cell lysate proteins. Blots were stripped by incubation for 30 min at 50°C in a solution containing 62.5 mM Tris•HCl, pH 6.7; 2% SDS; and 0.7% β-mercaptoethanol. Blots were then washed for 30 min with several changes of PBST at room temperature. Efficacy of stripping was determined by re-exposure of the membranes to ECL plus™. Thereafter, blots were re-blocked and immunolabelled as described above.

### Cell number assay

Cells were seeded onto 6-well plates at a density of 5 × 10⁴ cells per well, in triplicate, in 3 ml of complete RPMI medium containing 10% or 0.2% FBS. Cells were cultured under these conditions for up to 10 days with media change every other day until harvesting. To compare the cell proliferation rate, the total cell number in each well was quantified with a haemocytometer every 2 days. Cell viability was assessed by using trypan blue.

### Cell proliferation assay

MTT (3-[4,5-dimethylthiazol-2-yl]-2,5-diphenyl tetrazolium bromide) assays were used measure cell proliferation. Cells were seeded into 96 well flat bottom tissue culture microplates in 100 µl RPMI-1640 culture medium containing 10% FBS at a concentration of 5 × 10³ cells/well. On assay day, 10 µl of the MTT labelling reagent was added at a final concentration 0.5 mg/ml to each well and the cells were labelled for 5h in the incubator. At the end of the incubation period, the media was removed and the converted dye was solubilised with 100 µl solubilising solution (0.01N HCl in 10% SDS in water). The plates were then sealed and placed at 37°C overnight to completely solubilise the purple formazan crystals. The absorbance of the converted dye was measured using Spectra Max 250 multiplate reader at a wavelength of 570 nm with background subtraction at 650 nm. Each treatment was performed at least in triplicate.

Cell cycle kinetics were measured by bromodeoxyuridine (BrdU) incorporation using a BrdU staining kit (Zymed) in accordance with the manufacturer's instructions. Briefly, cells were seeded on glass coverslips in 6-well plates in full media and incubated overnight before 18 hours of serum starvation in serum-free media. Cells were then pulse labelled with BrdU at a concentration of 10 µM in serum-free media for 45 min. After fixing, denaturing, and blocking, cells were incubated with biotinylated anti-BrdU mouse monoclonal antibody. Cells were then incubated with streptavidin-peroxidase followed by incubation with diaminobenzidine (DAB) chromogen solution for colour development in the presence of hydrogen peroxide. The cell nuclei were counterstained with hematoxylin. Cells with dark brown nuclear staining were counted as BrdU labelled cells. For quantification, 10 random fields per coverslip (magnification of 40×) were documented by photomicroscopy. The percentage of BrdU positive cell nuclei relative to the total number of cell nuclei was calculated. A minimum of 500 cells per coverslip were counted.

### Apoptosis assay

Cells were seeded onto 6-well plates and were serum depleted for 24 hours. Multiple methods were used to detect apoptosis. In one set of experiments, apoptotic cell death was measured by fluorescent microscopic analysis of cell DNA staining patterns with karyophilic Hoechst 33258 (Del, B. G., Z. Darzynkiewicz, C. Degraef, R. Mosselmans, D. Fokan, and P. Galand. 1999. Comparison of methods based on annexin-V binding, DNA content or TUNEL for evaluating cell death in HL-60 and adherent MCF-7 cells. Cell Prolif. 32:25-37). In brief, cells were fixed with 4% paraformaldehyde, permeabilized with 0.6% Tween^{®} 20, and stained with Hoechst 33258 (1:10,000; Molecular Probes, Eugene, OR) for 5 min at room temperature. Following washing with PBS, nuclear morphology was examined under a UV-visible fluorescence microscope with a 40x objective. Cells with apoptotic nuclear characteristics such as nuclear condensation and fragmentation were scored as apoptotic.

In a second set of experiments, apoptotic cells were stained with fluorescent isothiocyanate-conjugated annexin V and propidium iodide (PI) using an Annexin V-FLUOS staining kit (Roche Applied Science) according to the manufacturer's instructions. Samples were analysed for phosphatidylserine (PS) externalization under a fluorescence microscope. Combined staining with annexin V and PI was used to determine early and late apoptosis. Cells were considered early apoptotic when they were annexin V-positive and PI-negative and late-apoptotic when they were both annexin V- and PI-positive. For each treatment, 200-300 cells were examined.

### Anoikis assay

Regular cell culture six-well plates were coated with a film of poly-HEMA according to the procedure reported by Folkman and Moscona (Folkman, J. and Moscona, A. (1978). Role of cell shape in growth control. Nature 278, 345-349). Briefly, a solution of 10 mg/ml of polyHEMA in 95% ethanol was mixed overnight. This was centrifuged at 800 × g to remove undissolved particles. The resulting solution was placed in the culture plates, and dried in the tissue culture hood. This was repeated once. Before use, the coated dishes were washed twice with phosphate buffered saline (PBS).

For suspension culture, near-confluent cells on regular plastic tissue culture flasks were trypsinized to produce a single cell suspension. Trypsin was inactivated by the addition of 10% FBS. Trypsinized cells were then counted and plated at a density of 50,000 cells per well in 2 ml of growth media using six-well dishes coated with polyHEMA. Cells were fed with 1 ml of fresh media every other day. On the days indicated, cells were collected by pipetting, washed with PBS, and treated with trypsin to dislodge cell clumps. Viable cells were counted in a hemocytometer using the trypan blue exclusion procedure.

### Soft agar assay jor colony formation

For soft agar colony formation assay, cells were cultured in 6-well plates first covered with a layer of agar (0.5%). Cells were grown in RPMI 1640 media containing 10% FBS. The middle layer included 5 × 10³ cells in 0.35% agar in RPMI 1640 media containing 10% FBS. Media was added as the top layer to prevent drying of the agarose gels. The plates were incubated at 37°C in a humidified incubator for 14 days. After this, cells were stained with 0.5 ml of 0.005% crystal violet in distilled water for 1 h and distained for 2 h in water. Colonies were inspected and photographed.

### Soft agar growth assays

To perform soft agar colony formation assays, 50 µl of 0.5% agarose in serum containing medium was plated onto each well of 96-well flat-bottom microplates, as a base agar layer. Next, 5 × 10³ cells in a 100 µl mixture of 0.35% agarose in serum containing medium were plated onto the base layer. Subsequently, 100 µl of the medium was added to prevent drying of the agarose gels. For antibody treatment, the antibodies at the indicated concentrations were added into the top agar layer and the culture medium to ensure the complete exposure of cells to antibodies. The plates were incubated at 37°C in a humidified incubator. The top culture medium including the antibodies was changed every 3 days. After 10 days, 20 µl of 5 mg/ml MTT (Sigma Chemical Company, St Louis, MO, USA) was added into each well and incubated at 37°C for 4 hours followed by adding 100 µl of acidified lysis buffer (0.01% HCl in 10% SDS). The plate was incubated at 42°C to ensure a homogenous mixture. The plate was shaken briefly and then read at 595 nm (test wavelength) and 690 nm (reference wavelength) using the Synergy™ 2 Multi-Mode Microplate Reader (BioTek instruments, Inc., Vermont, USA).

### Wound-healing assays

For *in vitro* wound-healing assays, cells were grown as a monolayer on regular cell culture dishes until confluence. A 4-mm scrape wound was made with a sterile pipette tip in the confluent layer. Displaced cells were removed with three washes with PBS and the remaining cells were bathed in fresh culture medium. The medium was replaced every day. The wound edges were photographed periodically.

### Cell migration and invasion

Cell migration was assayed using modified Boyden chambers containing polycarbonate membranes (tissue culture treated, 6.5 mm diameter, 10 µm thickness, 8 µm pores, Transwell^{®}; Costar, Cambridge, MA) as previously described (Doerr, M. E. and J. I. Jones. 1996. The roles of integrins and extracellular matrix proteins in the insulin-like growth factor I-stimulated chemotaxis of human breast cancer cells. J. Biol. Chem. 271:2443-2447), with some modification. Cell invasion assays were carried using the same protocol, except that a layer of Matrigel™ was polymerized onto the membranes according to the manufacturer's specifications. This was done prior to the addition of cells to the upper wells.

To ensure single cell suspension for migration/invasion assay, cells at near confluence were trypsinized the day before the assay, passed through an 18G needle several times and incubated at least 12 hours prior to assays. The next day, cells were harvested with 5 mM EDTA in PBS, pH 7.4, washed twice, and then suspended in serum-free RPMI/BSA. A 100 µl volume of cells (1.0 × 10⁵) was loaded into Transwell^{®} inserts. The lower chambers were filled with 600 µl of RPMI containing 10% FBS. The loaded chamber was placed in a 37°C incubator in a humidified atmosphere with 5% CO₂. After 24 h, the inserts were removed and the cells were then fixed with 4% paraformaldehyde. Cells (and Matrigel™) on the upper surface were removed by scraping with cotton swabs. Those that migrated to the lower surface of the membrane were stained with crystal violet and identified microscopically

Cell counts were obtained by counting all cells in six grids on one row across the centre of the round filter. This was done using a Lovins Microslide field finder (Gurley, Troy, NY) under a microscope at a total magnification of 100 x. Pictures were taken from representative fields of each membrane. All data presented are the means ± standard deviation (SD) of triplicate analysis.

### 3D-culture inside Matrigel™

For the 3D Matrigel™ culture, monolayer grown cells were harvested by trypsinization, taken up in the complete medium, and separated into single-cell suspension by several passages through a Pasteur pipette. After this, 50 µl complete medium containing 5,000 cells was added to 150 µl of growth factor reduced Matrigel™ (Becton Dickinson, Bedford, MA) diluted 1:1 with complete media with 20% FBS. This was gently mixed, deposited onto Nunc 4-well slides precoated with the Matrigel, and incubated at 37°C until the Matrigel solidified. The cells were then covered with complete medium and grown at 37°C under 5% CO₂ for up to 24 days with a change of media every 2 days. Organoid formation was counted in randomly chosen fields with light microscopy.

### Real-Time PCR and Reverse Transcription-PCR

For real-time PCR, total RNA was converted to cDNA using SuperScript™ III First-Strand Synthesis SuperMix for qRT-PCR (Invitrogen, CA) as per the manufacturer's instructions. The ABI 7700 ^{®} real-time PCR system (Applied Biosystems, USA) was used for this analysis. Multiple gene markers distributed around the genome and three housekeeping genes were used for real-time PCR analysis using the SYBR^{®} GreenER™ qPCR SuperMix for ABI PRISM^{®} (Invitrogen, CA). The sequence information for the primers is listed in Table 2, see further below. For the reaction, 5 ng total cDNA from both test genes and endogenous control genes was added to a 20 µl reaction mixture containing SYBR^{®} GreenER™ qPCR SuperMix for ABI PRISM^{®}, and 200 nM each primer. Triplicate reactions were performed for each marker in a 384-well plate using a two-step amplification program of initial denaturation at 95°C for 10 min, followed by 40 cycles of 95°C for 20 s and 60°C for 30 s. A melting curve analysis step was carried out at the end of the amplification, consisting of denaturation at 95°C for 1 min and re-annealing at 55°C for 1 min.

Standard curves were generated from each experimental plate using serial 5-fold dilutions of untreated cDNA. The geometric mean of Ct-value for each reaction was calculated. Amplification efficiencies were calculated according to the equation E = 10^{(-l/slope)} (Heid CA, Stevens J, Livak KJ, Williams PM (1996). Real time quantitative PCR. Genome Res 6: 986-94.) and ranged from 90-104% for all gene markers. No non-specific amplification or primer dimers were observed in any of the reactions as confirmed by the melt curve analysis. To compensate for potential differences between markers, the relative expression was computed, based on the efficiency (E) value, normalized to a panel of housekeeping genes, β-action, HPRT, and GAPDH (e.g. relative expression = 2 ^{-(*Ct*,Sample - *C*t,HKG)-(*C*t,Control - *C*t,HKG)}) and the Ct difference (Δ) of sample versus control (ΔCt _{sample-control}) (e.g. relative expression = 2^{-ddCt}).

### Production of recombinant human Artemin protein in bacteria

*E.coli* Rosetta-gami™ B (DE3) pLysS competent cells transformed with the plasmid pQE30-ARTN were grown in 1 litre LB containing 100 µg/ml Carbenicillin at 37°C until A₆₀₀ reached 0.5. Protein expression was then induced by addition of isopropyl-β-D-thiogalactopyranoside (IPTG) to a final concentration of 0.2 mM and the cultures were incubated for an additional 5 to 6h. The cells were harvested and the pellet was resuspended in 50 ml of 20 mM Tris-HCl (pH 8.0), 1 mM EDTA and 0.1 mg/ml lysozyme. The cells were disruption by sonication followed by a centrifugation at 10,000g for 30 minutes at 4°C to isolate the inclusion bodies. The inclusion bodies were washed by 2% Triton X-100, 20 mM Tris-HCl (pH 8.0) and 1 mM EDTA twice, and then by 20 mM Tris-HCl (pH 8.0) to remove the detergent and EDTA.

Subsequently, the inclusion bodies were solubilised in 50 ml of solubilising buffer (6 M guanidine hydrochloride, 10 mM Tris-HCl and 100 mM NaH₂PO4, pH 8.0) and rocked overnight at 4°C. After a centrifugation at 10,000g for 30 minutes at 4°C, the supernatant was mixed with 4 ml of Ni-NTA (Qiagen) at 50% slurry and rocked for 1 hour at 4°C. The lysate-resin mixture was then loaded into an empty column, followed by wash with 20 ml of 25 mM imidazole, 10 mM Tri-HCl, 100 mM NaH₂PO₄, 500 mM NaCl, 8 M Urea (pH 8). The protein was eluted with 1 ml of elution buffer containing 250 mM imidazole, 10 mM Tris-HCl, 100 mM NaH₂PO₄, 150 mM NaCl, 8 M Urea (pH 8.0). The fractions were collected and analysed by SDS-PAGE and visualised by Coomassie blue staining. The yield of purified protein was estimated by Bradford's assay.

### Production of rabbit polyclonal antibodies

Polyclonal antisera against human Artemin was generated using subcutaneous and intramuscular injections of the immunogen into rabbits as described by Bean (Eric S. Bean (2001) Polyclonal Antibodies. In: Basic Methods in Antibody Production and Characterization antibodies. Howard, G. and Bethel D. (ed.), CRC Press, 5:31-50, 2000). The antibodies were affinity purified from the antisera using standard methodology.

### Production of chicken polyclonal antibodies to Artemin

*Immunization:* Chickens were immunized with recombinant human Artemin (∼0.5 mg/chicken) with complete Freund's adjuvant on Day 0 and boosted again on days 9, 29, and 39 (∼0.17 mg/chicken). Eggs were collected pre- and post-immunizations. *IgY extraction from egg yolk:* The eggs were collected and egg yolk separated and pooled. The purification was performed at 4°C. Egg yolk was diluted and centrifuged. IgY was precipitated from the clear supernatant. The precipitate was separated by centrifugation and dissolved in PBS. The pre- and post-immunization chicken IgY samples were filter sterilised to yield a final concentration of ∼5 mg/ml with purity equal or greater than 90%, and stored at 4°C.

Results are described in detail further below.

**Table 1. Primer sequences used to detect gene specific transcripts in PCR and RT-PCR.**

| **Genes** | **Primer** | **Primer Sequence (5'-3')** | **SEQ ID NO:** |
|---|---|---|---|
| Artemin | Forward: | CTTTGCAGACTGGACCCTTACC | 52 |
| | Reverse: | AGCTCCCATGAGTGAGTACAGG | 53 |
| Persephin | Forward: | ATGGCCGTAGGGAAGTTCCTG | 54 |
| | Reverse: | TCAGCCACCACAGCCGCAGGC | 55 |
| GFRα1 | Forward: | TTGCAGGACTCCTGCAAGACG | 56 |
| | Reverse: | GACCACAGCTTGGAGGAGCAG | 57 |
| GFRα2 | Forward: | CAGCTGCTCCTATGAGGACAAG | 58 |
| | Reverse: | CTGGGATGATATTTGTCGTGAGC | 59 |
| GFRα3 | Forward: | CTGCTCACTTTCTTCGAGAAGG | 60 |
| | Reverse: | CAGGGTTTTCATTCTGGTGTGC | 61 |
| GFRα4 | Forward: | ATGGTGCCATTCAGGCCTTTGC | 62 |
| | Reverse: | ATGGTCTCTGACCTGCTCTAGG | 63 |
| RET | Forward: | CGTGAAGAGGAGCCAGGGTC | 64 |
| | Reverse: | TAACCATCATCTTCTCCAGGTCT | 65 |
| β-2-microglobulin | Forward: | TCTCGCTCCGTGGCCTTAGCTG | 66 |
| | Reverse: | AACATGTCTCGATCCCACTTAACTATC | 67 |
| β-actin | Forward: | ATGATATCGCCGCGCTCG | 68 |
| | Reverse: | CGCTCGGTGAGGATCTTCA | 69 |

**Table 2: The sequences of the oligonucleotide primers used for real-time PCR.**

| **Genes** | **Abbr.** | **Primer Sequence (5'-3')** | | **Amplicon size** | **SEQ ID NO:** |
|---|---|---|---|---|---|
| v-akt murine thymoma viral oncogene homolog 1 | *AKT1* | Forward: | GCACAAACGAGGGGAGTACAT | 113 bp | 70 |
| | | Reverse: | CCTCACGTTGGTCCACATC | | 71 |
| Ataxia telangiectasia mutated | *ATM* | Forward: | TGGATCCAGCTATTTGGTTTGA | 82 bp | 72 |
| | | Reverse: | CCAAGTATGTAACCAACAATAGAAGAAGTA G | | 73 |
| BCL2-antagonist of cell death | *BAD* | Forward: | CCCAGAGTTTGAGCCGAGTG | 249 bp | 74 |
| | | Reverse: | CCCATCCCTTCGTCGTCCT | | 75 |
| BCL2-associated X protein | *BAX* | Forward: | GGGTGGTTGGGTGAGACTC | 199 bp | 76 |
| | | Reverse: | AGACACGTAAGGAAAACGCATTA | | 77 |
| B-cell CLL/lymphoma 2 | *BCL2* | Forward: | TCCGCATCAGGAAGGCTAGA | 113 bp | 78 |
| | | Reverse: | AGGACCAGGCCTCCAAGCT | | 79 |
| BCL2-like 1 | *BCL2L1* | Forward: | ATGGCAGCAGTAAAGCAAGC | 149 bp | 80 |
| | | Reverse: | CGGAAGAGTTCATTCACTACCTGT | | 81 |
| Beta-actin | *β-actin* | Forward: | TTCCTGGGCATGGAGTC | 84 bp | 82 |
| | | Reverse: | CAGGTCTTTGCGGATGTC | | 83 |
| Breast cancer 1, early onset | *BRCA1* | Forward: | CATGCTGAAACTTCTCAACCAGAA | 81 bp | 84 |
| | | Reverse: | TGTAGGCTCCTTTTGGTTATATCATTC | | 85 |
| Caspase 7, apoptosis-related cysteine peptidase | *CASP7* | Forward: | AGTGACAGGTATGGGCGTTCG | 274 bp | 86 |
| | | Reverse: | GCATCTATCCCCCCTAAAGTGG | | 87 |
| Cyclin D1 | *CCND1* | Forward: | ACGAAGGTCTGCGCGTGTT | 323 bp | 88 |
| | | Reverse: | CCGCTGGCCATGAACTACCT | | 89 |
| Cell division cycle 25A | *CDC25A* | Forward: | TAAGACCTGTATCTCGTGGCTG | 131 bp | 90 |
| | | Reverse: | CCCTGGTTCACTGCTATCTCT | | 91 |
| Cyclin-dependent kinase 2 | *CDK2* | Forward: | GCTAGCAGACTTTGGACTAGCCAG | 85 bp | 92 |
| | | Reverse: | AGCTCGGTACCACAGGGTCA | | 93 |
| Cyclin-dependent kinase 4 | *CDK4* | Forward: | CTGGTGTTTGAGCATGTAGACC | 102 | 94 |
| | | Reverse: | AAACTGGCGCATCAGATCCTT | | 95 |
| Cyclin-dependent kinase inhibitor 1A (p21, Cip1) | *CDKN1A* | Forward: | CCTCATCCCGTGTTCTCCTTT | 97 bp | 96 |
| | | Reverse: | GTACCACCCAGCGGACAAGT | | 97 |
| Cyclin-dependent kinase inhibitor 2A (melanoma, p16, inhibits CDK4) | *CDKN2A* | Forward: | CAACGCACCGAATAGTTACGG | 96 bp | 98 |
| | | Reverse: | AACTTCGTCCTCCAGAGTCGC | | 99 |
| CASP8 and FADD-like apoptosis regulator | *CFLAR* | Forward: | GTGGAGACCCACCTGCTCA | 96 bp | 100 |
| | | Reverse: | GGACACATCAGATTTATCCAAATCC | | 101 |
| CHK2 checkpoint homolog | *CHEK2* | Forward: | AGTGGTGGGGAATAAACGCC | 117 bp | 102 |
| | | Reverse: | TCTGGCTTTAAGTCACGGTGTA | | 103 |
| Cyclin E1 | *CCNE1* | Forward: | ATCAGCACTTTCTTGAGCAACA | 122 bp | 104 |
| | | Reverse: | TTGTGCCAAGTAAAAGGTCTCC | | 105 |
| E2F transcription factor 1 | *E2F1* | Forward: | AGATGGTTATGGTGATCAAAGCC | 72 bp | 106 |
| | | Reverse: | ATCTGAAAGTTCTCCGAAGAGTCC | | 107 |
| v-erb-b2 erythroblastic leukemia viral oncogene homolog 2 | *ERBB2* | Forward: | ACTGGCCCTCATCCACCATA | 104 bp | 108 |
| | | Reverse: | GGTTGGCAGTGTGGAGCAG | | 109 |
| v-ets erythroblastosis virus E26 oncogene homolog 2 | *ETS2* | Forward: | CCCCTGTGGCTAACAGTTACA | 222 bp | 110 |
| | | Reverse: | AGGTAGCTTTTAAGGCTTGACTC | | 111 |
| v-fos FBJ murine osteosarcoma viral oncogene homolog | *FOS* | Forward: | TGCCTCTCCTCAATGACCCTGA | 162 bp | 112 |
| | | Reverse: | ATAGGTCCATGTCTGGCACGGA | | 113 |
| Glyceraldehyde-3-phosphate dehydrogenase | *GAPDH* | Forward: | TGCACCACCAACTGCTTAGC | 87 bp | 114 |
| | | Reverse: | GGCATGGACTGTGGTCATGAG | | 115 |
| Hypoxanthine phosphoribosyltransferase | *HPRT* | Forward: | TGACACTGGCAAAACAATGCA | 94 bp | 116 |
| | | Reverse: | GGTCCTTTTCACCAGCAAGCT | | 117 |
| HIV-1 Tat interactive protein 2, 30kDa | *HTATIP2* | Forward: | CGGAGGGATTTGTTCGTGTTG | 104 bp | 118 |
| | | Reverse: | AGCTCCTTTAGAGGATAGCAAGT | | 119 |
| v-jun sarcoma virus 17 oncogene homolog | *JUN* | Forward: | CTCCAAGTGCCGAAAAAGGAAG | 118 bp | 120 |
| | | Reverse: | CACCTGTTCCCTGAGCATGTTG | | 121 |
| Mitogen-activated protein kinase kinase 1 | *MAP2K1* | Forward: | CAATGGCGGTGTGGTGTTC | 114 bp | 122 |
| | | Reverse: | AGCTCCCTTATGATCTGGTTCC | | 123 |
| MET proto-oncogene (hepatocyte growth factor receptor) | *MET* | Forward: | TGGTGCAGAGGAGCAATGG | 111 bp | 124 |
| | | Reverse: | CATTCTGGATGGGTGTTTCCG | | 125 |
| Matrix metallopeptidase 1 | *MMP1* | Forward: | AGCTAGCTCAGGATGACATTGATG | 74 bp | 126 |
| | | Reverse: | GCCGATGGGCTGGACAG | | 127 |
| Matrix metallopeptidase 2 | *MMP2* | Forward: | CAAAAACAAGAAGACATACATCTT | 232 bp | 128 |
| | | Reverse: | GCTTCCAAACTTCACGCTC | | 129 |
| Matrix metallopeptidase 9 | *MMP9* | Forward: | TGGGGGGCAACTCGGC | 224 bp | 130 |
| | | Reverse: | GGAATGATCTAAGCCCAG | | 131 |
| Metastasis associated 1 | *MTA1* | Forward: | GCTGTTACACCACACAGTCTT | 166 bp | 132 |
| | | Reverse: | GGACTCATGTTACTGCGGTTT | | 133 |
| Metastasis associated 2 | *MTA2* | Forward: | CCGACGGCCTTATGCTCCT | 145 bp | 134 |
| | | Reverse: | CTGGGCCACCAGATCTTTGAC | | 135 |
| v-myc myelocytomatosis viral oncogene homolog (avian) | *MYC* | Forward: | TGCTGCCAAGAGGGTCAAGT | 118 bp | 136 |
| | | Reverse: | GTGTGTTCGCCTCTTGACATTC | | 137 |
| Nuclear factor of kappa light polypeptide gene enhancer in B-cells 1 (p105) | *NFKB1* | Forward: | TGCCAACAGATGGCCCATAC | 123 bp | 138 |
| | | Reverse: | TGTTCTTTTCACTAGAGGCACCA | | 139 |
| Nuclear factor of kappa light polypeptide gene enhancer in B-cells inhibitor, alpha | *NFKBIA* | Forward: | CTCCGAGACTTTCGAGGAAATAC | 135 bp | 140 |
| | | Reverse: | GCCATTGTAGTTGGTAGCCTTCA | | 141 |
| Non-metastatic cells 1, protein (NM23A) | *NME1* | Forward: | CTGCAGCCGGAGTTCAAAC | 68 bp | 142 |
| | | Reverse: | GCAATGAAGGTACGCTCACAGT | | 143 |
| Phosphoinositide-3-kinase, regulatory subunit 1 (p85 alpha) | *PIK3R1* | Forward: | GATTCTCAGCAGCCAGCTCTGAT | 91 bp | 144 |
| | | Reverse: | GCAGGCTGTCGTTCATTCCAT | | 145 |
| Plasminogen activator, urokinase | *PLAU* | Forward: | CACGCAAGGGGAGATGAA | 341 bp | 146 |
| | | Reverse: | ACAGCATTTTGGTGGTGACTT | | 147 |
| Plasminogen activator, urokinase receptor | *PLAUR* | Forward: | AATGGCCGCCAGTGTTACAG | 227 bp | 148 |
| | | Reverse: | CAGGAGACATCAATGTGGTTC | | 149 |
| v-raf-1 murine leukemia viral oncogene homolog 1 | *RAF1* | Forward: | TTTCCTGGATCATGTTCCCCT | 153 bp | 150 |
| | | Reverse: | ACTTTGGTGCTACAGTGCTCA | | 151 |
| Retinoblastoma 1 | *RB1* | Forward: | GAACATCGAATCATGGAATCCCT | 116 bp | 152 |
| | | Reverse: | AGAGGACAAGCAGATTCAAGGTGAT | | 153 |
| S100 calcium binding protein A4 | *S100A4* | Forward: | GATGAGCAACTTGGACAGCAA | 123 | 154 |
| | | Reverse: | CTGGGCTGCTTATCTGGGAAG | | 155 |
| Serpin peptidase inhibitor, clade B (ovalbumin), member 5 | *SERPINB5* | Forward: | CTACTTTGTTGGCAAGTGGATGAA | 90 bp | 156 |
| | | Reverse: | ACTGGTTTGGTGTCTGTCTTGTTG | | 157 |
| Serpin peptidase inhibitor, clade E (nexin, | *SERPINE1* | Forward: | CACAAATCAGACGGCAGCACT | 85 bp | 158 |
| plasminogen activator inhibitor type 1) | | Reverse: | CATCGGGCGTGGTGAACTC | | 159 |
| Synuclein, gamma (breast cancer-specific protein 1) | *SNCG* | Forward: | TGAGCAGCGTCAACACTGTG | 64 bp | 160 |
| | | Reverse: | GAGGTGACCGCGATGTTCTC | | 161 |
| Telomerase reverse transcriptase | *TERT* | Forward: | GGAGCAAGTTGCAAAGCATTG | 182 bp | 162 |
| | | Reverse: | TCCCACGACGTAGTCCATGTT | | 163 |
| Tumor necrosis factor receptor superfamily, member 10b | *TNFRSF10B* | Forward: | AAGACCCTTGTGCTCGTTGT | 144 bp | 164 |
| | | Reverse: | AGGTGGACACAATCCCTCTG | | 165 |
| Tumor necrosis factor receptor superfamily, member 1A | *TNFRSF1A* | Forward: | TGCCTACCCCAGATTGAGAA | 169 bp | 166 |
| | | Reverse: | ATTTCCCACAAACAATGGAGTAG | | 167 |
| Tumor necrosis factor receptor superfamily, member 25 | *TNFRSF25* | Forward: | ACTGCCAACCATGCCTAGACTG | 155 bp | 168 |
| | | Reverse: | GAGCCTCCATCCCAGCTTC | | 169 |
| Tumor protein p53 (Li-Fraumeni syndrome) | *TP53* | Forward: | TGCAGCTGTGGGTTGATTCC | 396 bp | 170 |
| | | Reverse: | AAACACGCACCTCAAAGCTGTTC | | 171 |

**Table 3: Expression of functional genes in MCF7-ARTN relative to MCF7-Vec cells.**

| **Functional Gene Grouping** | **Gene** | **Fold change** | ***p-value*** |
|---|---|---|---|
| | *Cyclin D1* | -1.03 | 3.21E-03 |
| | *ATM* | -1.27 | 2.26E-02 |
| | *BRCA1* | -1.37 | 8.99E-03 |
| | *Cyclin E1* | -1.04 | 4.00E-03 |
| | *CDC25A* | -1.11 | 2.66E-02 |
| **Cell Cycle Control & DNA Damage Repair** | *CDK2* | -1.13 | 2.70E-05 |
| | *CDK4* | -1.14 | 2.70E-04 |
| | *CDKN1A* | 1.08 | 2.18E-05 |
| | *CDKN2A* | -2.76 | 2.19E-02 |
| | *CHEK2* | -1.01 | 7.13E-03 |
| | *E2F1* | -1.05 | 5.04E-02 |
| | *CDKN1B* | -1.07 | 3.19E-04 |
| | *RB1* | -1.00 | 1.92E-03 |
| | *S100A4* | 1.27 | 2.41E-01 |
| | *TP53* | 0.00 | 2.85E-05 |
| | *APAF1* | 1.13 | 1.53E-02 |
| | *BAD* | 1.03 | 4.83E-03 |
| | *BAX* | -1.75 | 2.35E-02 |
| | *BCL2* | 8.40 | 1.05E-02 |
| **Apoptosis and Cell Senescence** | *BCL2L1* | -1.22 | 4.59E-02 |
| | *CFLAR* | -1.35 | 2.76E-02 |
| | *CASP7* | 1.04 | 4.20E-04 |
| | *HTATIP2* | 1.02 | 1.13E-03 |
| | *TERT* | 2.52 | 1.18E-02 |
| | *TNFRSF1A* | -1.12 | 1.71E-02 |
| | *TNFRSF10B* | -1.27 | 3.30E-02 |
| | *TNFRSF25* | -1.25 | 1.19E-02 |
| | *AKT1* | -1.24 | 2.17E-04 |
| | *ERBB2* | -1.03 | 3.62E-03 |
| | *ETS2* | -1.49 | 7.53E-03 |
| | *FOS* | -1.70 | 5.61E-04 |
| | *JUN* | -1.36 | 3.22E-04 |
| **Signal Transduction Molecules and Transcription Factors** | *MAP2K1* | -1.12 | 8.32E-04 |
| | *MYC* | 1.01 | 3.83E-04 |
| | *NFKB1* | -1.41 | 6.03E-04 |
| | *NFKBIA* | -1.03 | 1.71E-02 |
| | *PIK3R1* | -1.04 | 2.58E-03 |
| | *RAF1* | -1.22 | 2.29E-05 |
| | *SNCG* | 1.67 | 4.99E-01 |
| | *MET* | -1.22 | 1.18E-03 |
| | *MMP1* | 7.60 | 2.64E-05 |
| | *MMP2* | -1.32 | 2.33E-02 |
| | *MMP9* | 1.29 | 1.49E-01 |
| **Invasion and Metastasis** | *MTA1* | -2.27 | 1.29E-03 |
| | *MTA2* | -1.41 | 3.12E-02 |
| | *NME1* | -2.12 | 1.51E-02 |
| | *PLAU* | 3.81 | 3.39E-02 |
| | *PLAUR* | -1.04 | 1.40E-02 |
| | *SERPINB5* | 1.29 | 4.54E-02 |
| | *SERPINE1* | 3.10 | 3.24E-03 |

| | | | |
|---|---|---|---|
| *Average of three experiments represents each gene fold-changes (*p-value* <0.05). A positive value indicates gene up-regulation and a negative value indicates gene down-regulation. | | | |

### EXAMPLE 2: RESULTS

### Artemin is oncogenic for human mammary carcinoma cells

### Expression of Artemin in human tissues and carcinoma cell lines

The expression of Artemin and Persephin in mammalian carcinoma MCF-7 cells was analysed by RT-PCR. As shown in Figure 1A, specific bands of Artemin and Persephin were observed in MCF-7 cells. The expression profile of Artemin in normal tissues was determined by use of a commercially-available panel of cDNAs from various human tissues. Screening for mRNA expression by PCR with primers specific for human Artemin revealed that the Artemin gene was expressed in a number of non-neural tissues (Figure 1B). The highest expression was observed in cerebellum followed by normal colon. Expression was also detected in prostate, uterus, stomach, kidney, trachea, fetal brain, adipose, and cartilage. Expression of Artemin was not observed in heart, skeletal muscle, small intestine, spleen, thymus, skin, liver, fetal liver, lung, brain, spinal cord, placenta, adrenal gland, stimulated PBL, pancreas, salivary gland, thyroid, umbilical cord, ovary or PBL.

The expression of Artemin in a number of human cancer cell lines was also examined by RT-PCR. As shown in Figure 1C, Artemin was expressed in all the cancer cell lines tested. Higher expression was detected in DLD-1 colon, PC3 prostate and RL95-2 endometrial carcinoma cell lines. Moderate expression was observed in BxPC3 pancreas carcinoma cell line, and in four breast cancer cell lines, two of which (T47D and MCF-7) are estrogen receptor positive and two (BT549 and MDA-231) are estrogen receptor negative. Limited expression was observed in A549 lung, AGS stomach, Colo320DM colon, DU145 prostate and HepG2 liver cancer cell lines.

The expression of GFRα isoforms and RET was also determined by RT-PCR in MCF-7, T47D and BT549 mammary carcinoma cells (Figure 1D). The expression of GFRα and 3, and RET in MCF-7 cells was easily detected while GFRα2 was expressed at very low levels and required more cycles to detect its expression. As observed in Figure 1D, none of the three cell lines expressed GFRα4 at detectable levels. The expression pattern of these receptors in T47D and BT549 cells was quite similar to that in MCF-7 except that T47D exhibited a much lower level of GFRα1 expression and BT549 exhibited considerably higher expression of GFRα2.

### Effect of forced expression of Artemin on mammary carcinoma cell number

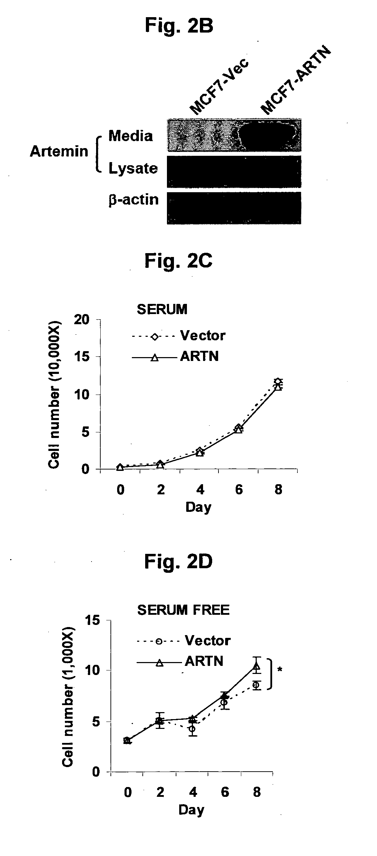

A cell model system was established to determine the functional consequences of Artemin expression in mammary carcinoma cells. MCF-7 cells were stably transfected with an Artemin expression plasmid pIRESneo3-ARTN (designated MCF7-ARTN) or the empty vector pIRESneo3 (designated MCF7-Vec). The forced expression of Artemin in MCF7-ARTN cells was verified at mRNA levels (Figure 2A) and protein levels (Figure 2B, middle panel) as compared to the control MCF7-Vec cells. The forced expression of Artemin also produced increased Artemin protein in conditioned media (Figure 2B, top panel).

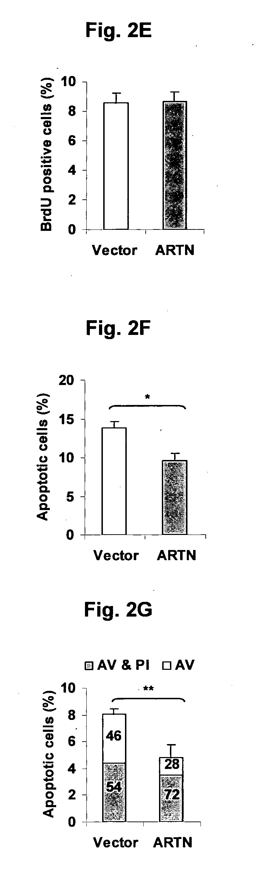

We first determined whether forced expression of Artemin affected total mammary carcinoma cell number in monolayer culture. As observed in Figure 2C, forced expression of Artemin in MCF-7 cells did not significantly increase cell number in serum replete conditions over a period of 8 days. However, in serum reduced (0.2%) media, MCF7-ARTN cells increased in number significantly faster than MCF7-Vec cells (Figure 2D). The total number of MCF7-ARTN cells was 23% more than that of MCF7-Vec cells after 8 days (p < 0.05). The effect of forced expression of Artemin on mammary carcinoma cell entry into S-phase was assessed by BrdU incorporation. As observed in Figure 2E, forced expression of Artemin in MCF-7 cells did not alter BrdU incorporation.

### Forced expression of Artemin increases cell survival

The effect of forced expression of Artemin on the survival of MCF-7 cells in serum deprived conditions was examined using two different methods. Estimation of apoptosis with Hoechst 33258 dye (Figure 2F) demonstrated a significant reduction in apoptosis in MCF7-ARTN versus MCF7-Vec cells (p < 0.05). Phosphatidylserine externalization is a unique marker for apoptosis. Cells were therefore stained with fluorescent isothiocyanate-conjugated Annexin V and propidium iodide (PI). As observed in Figure 2G, MCF7-ARTN cells were resistant to serum-deprivation-induced apoptosis compared with the control MCF7-Vec cells. Of the apoptotic cells, 28% of MCF7-ARTN cells were early apoptotic (annexin V-positive and PI negative cells) as compared to 46% of MCF7-Vec cells, indicating that Artemin protects cells from early apoptosis.

### Forced expression of Artemin upregulates BCL2

Real-time PCR (Table 3, see above) was performed to assess the effect of ARTN on the relative expression levels of key genes involved in cell proliferation and survival. Forced expression of Artemin only minimally altered the expression of most cell cycle-related markers, including cyclin D1 and E1, ATM, CDC25A, and CDK2 and 4, but did significantly decrease the expression of cyclin-dependent kinase inhibitor-2A (CDKN2A). CDKN2A, encoding p16(INK4a), has been recognised as a tumor suppressor gene that induces a G1 cell cycle arrest by inhibiting the phosphorylation of the Rb protein by the cyclin-dependent kinases CDK4 and CDK6 (Stott *et al.,* 1998).

The expression of BCL2 in MCF7-ARTN cells was increased by 8.4 fold compared with the control MCF7-Vec cells (Table 3, see above). BCL2 is a well described survival gene (Zinkel *et al.,* 2006). Another gene significantly increased by Artemin was telomerase-reverse transcriptase (TERT). TERT, in addition to its role in immortalization also suppresses apoptosis (Zhang *et al.,* 2003). It is of worth to note that γ-synuclein (SNCG, also referred as breast cancer-specific gene 1) was upregulated by Artemin by 1.67 fold. SNCG has been observed to protect tumor cells from apoptosis; and stimulate cell motility and metastasis (Wu *et al.,* 2003).

### Forced expression of Artemin promotes anchorage-independent growth

The effect of forced expression of Artemin on anchorage independent growth was examined in suspension culture and by colony formation in soft agar. As observed in Figure 3A, MCF7-ARTN cells grew faster than the control MCF7-Vec cells in suspension culture. The total number of MCF7-ARTN cells was 42% more than that of MCF7-Vec cells after 10 days (p < 0.01). When MCF7-ARTN cells were plated in soft agar, the number of colonies formed was significantly increased by two fold compared with MCF7-Vec cells (Figure 3B).

MCF7-ARTN cells and MCF7-Vec control cells were also cultured in 3D Matrigel. Concordant with colony formation in soft agar, an increase in colony number was observed for MCF7-ARTN cells compared with MCF7-Vec cells (data not shown). Moreover, the colonies formed by MCF7-ARTN cells in Matrigel were larger in size and the cells in the colonies exhibited more motile and invasive characteristics compared with those formed by MCF7-Vec cells (Figure 3C). While MCF7-Vec cells produced circumscribed colonies, a larger number of MCF7-ARTN cells spread from the main bulk of the colony, indicative that Artemin promoted local invasive behaviour.

### Forced expression of Artemin enhances cell migration and invasion

Fluorescence microscopy (Figure 3D) was used to visualize filamentous actin. This revealed that MCF7-Vec cells grew in clumps with prominent cell to cell contact typical of an epithelial phenotype, whereas MCF7-ARTN cells were scattered and displayed little intercellular contact. The effects of Artemin on cell mobility were therefore evaluated first by use of the standard Transwell chamber and subsequently by wound healing assay. We found two-fold more MCF7-ARTN cells migrated through Transwell inserts than did control MCF7-Vec cells over a 24h period (Figure 3E).

The invasive capacity of MCF7-ARTN cells was further assessed using Transwell chambers coated with Matrigel. Invasion of MCF7-ARTN cells was enhanced 2-fold compared with the control MCF7-Vec cells over a 48h period (Figure 3E). A wound-healing assay was performed as an alternative method for monitoring cell migration. As observed in Figure 3F, while monolayer culture of MCF7-ARTN cells demonstrated complete wound healing within 72h, MCF7-Vec cells did not. Forced expression of Artemin therefore enhances the motility and invasive capacity of MCF-7 cells. Concordant with this observation, real-time PCR demonstrated that forced expression of Artemin significantly increased the expression of a number of genes involved in invasion and metastasis, including MMP1 (matrix metalloproteinase 1; by 7.6 fold), PLAU (3.8 fold) and SERPINE1 (3.1 fold) (Table 3, see above).

### Forced expression of Artemin promotes MCF-7 tumor xenograft growth in vivo

Evidence from the *above in vitro* studies clearly indicated an oncogenic role for Artemin. We wanted to determine whether Artemin could enhance tumorigenicity *in vivo.* For this, we used a xenograft model, whereby MCF-7 cells were injected into the mammary fat pad of immunodeficient nude mice. Both MCF7-ARTN and MCF7-Vec cells formed palpable and measurable tumors. However, larger tumor volumes were observed for MCF7-ARTN cells as compared to the MCF7-Vec control cells (Figure 4A). Statistically significant enhancement of tumor volume was achieved by week 2 (p = 0.023). The tumors formed by MCF7-ARTN cells were more than double the size of tumors formed by MCF7-Vec cells after six and one half weeks.

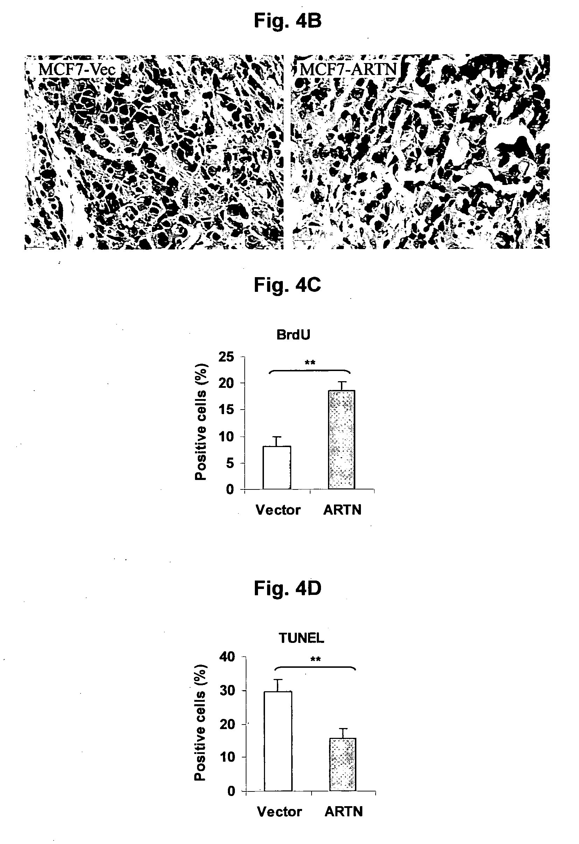

MCF-7 is a rather well differentiated tumor cell line as reflected by its estrogen dependent growth. Haematoxylin and Eosin histology demonstrated that tumors formed by MCF7-Vec cells were well differentiated (left panel, Figure 4B), whereas those formed by MCF7-ARTN cells displayed poorly-differentiated characteristics (right panel, Figure 4B). The MCF7-Vec control cells showed regularly-shaped and uniform nuclei, and clear cytoplasmic boundaries. In contrast, MCF7-ARTN cells formed small spindle shaped pleomorphic cells having irregular hyperchromatic nuclei, as well as indistinct cytoplasmic borders and scanty cytoplasm.

To quantify proliferation and apoptosis within the tumor, BrdU incorporation and TUNEL labelling were performed on tumor sections. The MCF7-ARTN tumors exhibited a higher percentage of BrdU labelled nuclei compared with MCF7-Vec tumors (Figure 4C). The level of apoptosis measured by TUNEL labelling also demonstrated Artemin enhanced tumor cell survival. There were significantly fewer TUNEL-positive cells in the MCF7-ARTN tumors compared with MCF7-Vec tumors (Figure 4D).

### Forced expression of Artemin enhanced anchorage-independent growth, cell migration and invasion in T47D and BT549 cells

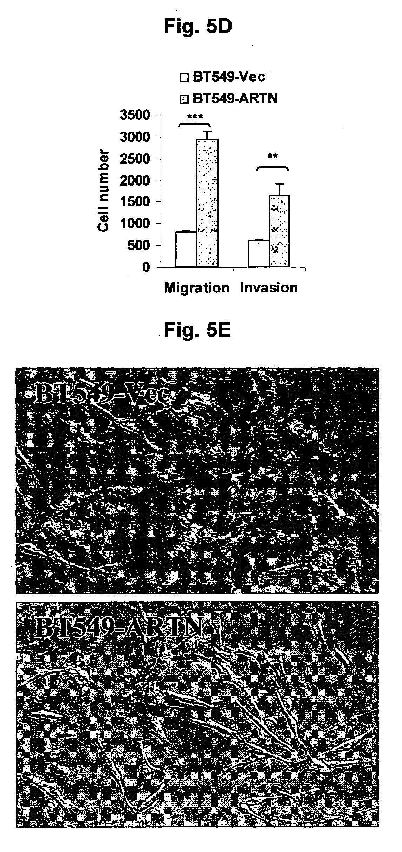

We also stably forced the expression of Artemin in estrogen receptor positive T47D and estrogen receptor negative BT549 cells (Figure 5A). As observed in Figures 5B-D, T47D-ARTN and BT549-ARTN cells with forced expression of Artemin both exhibited significant increases in colony formation in soft agar, and migration and invasion in Transwell assays compared with the control T47D-Vec and BT549-Vec cells, respectively. The effects were more pronounced in BT549 cells than in T47D cells. T47D-ARTN cells formed 35% more colonies than T47D-Vec control cells in soft agar, while BT549-ARTN cells formed 64% more colonies than BT-549-Vec control cells (Figure 5B). Compared to T47D-Vec cells, T47D-ARTN cells exhibited 37 and 74% more migration and invasion, respectively (Figure 5C). However, compared to BT549-Vec control cells, BT549-ARTN cells exhibited 272 and 169 % more migration and invasion, respectively (Figure 5D). In addition, BT549 cells with forced expression of Artemin exhibited a more mesenchymal morphology compared with the control cells (Figure 5E).

### Depletion of endogenous Artemin by siRNA significantly increases apoptotic cell death

We next depleted endogenous Artemin by plasmid vector-based siRNA and examined the behaviour of MCF-7 cells. To determine the knock-down efficacy of siRNA, Artemin cDNA was inserted within the 3'UTR of the EGFP expression vector pEGFP (pEGFP-ARTN). The efficiency of siRNA targeting the Artemin transcript was determined by examination of the expression level of EGFP protein using fluorescence microscopy. As observed in Figure 6A, the Artemin specific siRNA (ARTN-RNAi) construct effectively reduced the fluorescence of EGFP in cells transiently transfected with pEGFP-ARTN compared with the siRNA negative control (CK-RNAi). ARTN-RNAi did not reduce EGFP expression in pEGFP transfected cells compared with the CK-RNAi.

Two stable MCF-7 cell lines, MCF7-siRNA and MCF7-CK, were established by transfection of the Artemin specific siRNA plasmid (pSilencer-ARTN) or the negative control plasmid (pSilencer-CK), respectively. The Artemin expression level in these cell lines was determined by both RT-PCR and Western blot. As observed in Figure 6B, the expression of endogenous Artemin was reduced in MCF7-siRNA cells at both the mRNA level and the protein level compared with the MCF7-CK control cells.

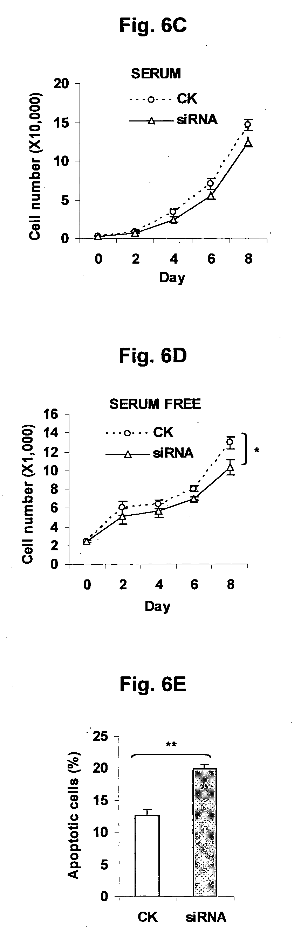

Depletion of endogenous Artemin by siRNA did not affect MCF-7 cell numbers in serum replete conditions, but significantly decreased (20%) cell numbers in serum reduced (0.2%) conditions (Figures 6C and D). As expected, depletion of endogenous Artemin by siRNA significantly increased apoptotic cell death induced by serum deprivation. Based on nuclear morphology after Hoechst 33258 visualization, only 13% of MCF7-CK cells, versus 20% of MCF7-siRNA cells, were identified as apoptotic after serum deprivation for 24h (Figure 6E). Similar fold changes were observed with Annexin-V labelling. As seen in Figure 6F, the percentage of apoptotic cells with the MCF7-CK construct, versus the MCF7-siRNA construct, showed a 2 fold increase as a result of depletion of Artemin. Of the apoptotic cells, the ratios of early and late apoptosis were approximately 1 to 3.55 (29% vs. 71%) for MCF7-siRNA cells; and 1 to 2.45 (22% vs. 78%) for MCF7-CK cells. This once indicated that depletion of endogenous Artemin preferentially increased early apoptotic cells.

### Depletion of endogenous Artemin by siRNA significantly inhibits anchorage-independent growth in MCF 7 cells

We found also that siRNA mediated depletion of Artemin in MCF-7 cells inhibited anchorage-independent growth of these cells. As observed in Figure 7A, the number of colonies formed in soft agar by MCF7-siRNA cells was significantly reduced (by 24%) as compared to MCF7-CK control cells. When grown in 3D Matrigel, a decrease in colony number was also observed for MCF7-siRNA cells compared with MCF7-CK cells (data not shown). In addition, the morphology of colonies formed by MCF7-siRNA cells in Matrigel differed from that of MCF7-CK cells (Figure 7B). Artemin-depleted MCF7-siRNA cells formed smaller colonies with a shrunken appearance, while MCF7-CK control cells formed larger, more voluminous colonies.

### Depletion of endogenous Artemin by siRNA impairs cell migration and invasion

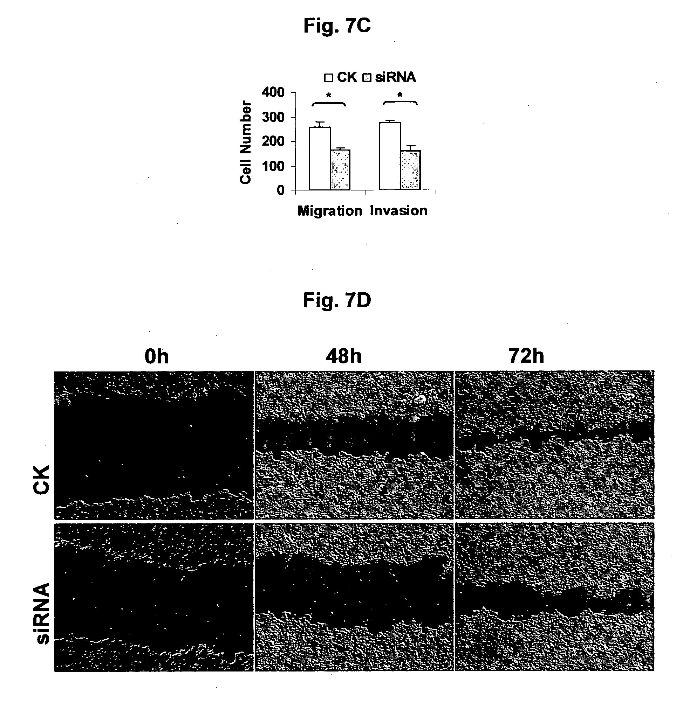

We assessed whether depletion of endogenous Artemin would impair motility/invasiveness of MCF-7 cells. As observed in Figure 7C, the number of cells that migrated through the non-coated (migration) or Matrigel-coated (invasion) porous filters of Transwell inserts were significantly reduced in Artemin depleted MCF7-siRNA cells, as compared to MCF7-CK control cells. Artemin depleted MCF7-siRNA cells also displayed a significant reduction in wound-induced migration. As shown in Figure 7D, the wound created in a monolayer of MCF7-siRNA closed less rapidly than MCF7-CK cells.

### Production of recombinant human Artemin protein in bacteria

The mature Artemin protein sequence was expressed in bacteria as a His-fusion (His-Artemin) using pQE30-ARTN plasmid. The production of the His-Artemin fusion was successfully induced by the addition of IPTG (Figure 8A). The identity of the protein was confirmed by Western blot analysis by goat anti-Artemin polyclonal antibodies (R&D Systems) (Figure 8B). The induced His-Artemin protein was purified using Ni-NTA (Qiagen). Recombinant Artemin protein had a sequence of mrgshhhhhhgs AGGPGSRARAAGARGCRLRSQLVPVRALGLGHRSDELVRFRFCSGSCRRARSPHDLSLASLLGA GALRPPPGSRPVSQPCCRPTRYEAVSFMDVNSTWRTVDRLSATACGCLG (SEQ ID NO: 34; the tag sequence is shown in lower case). Purified protein was analysed by SDS-PAGE and visualised by Coomassie blue staining. This showed a 13.5 kDa band as expected (Figure 8C).

### Rabbit polyclonal antibodies against Artemin reduce the viability and inhibit cell invasion of MCF-7 cells

Rabbit polyclonal antibodies against Artemin were generated. As shown in Figure 9A, the antiserum was able to recognise not only endogenous Artemin in MCF7-Vec cells but also forced expression of Artemin in MCF7-ARTN cells. Consistent with observations from forced expression, treatment of MCF-7 cells with the rabbit antiserum to Artemin did not affect MCF-7 cell number in monolayer when grown in serum-replete media (Figure 9B), but significantly reduced cell numbers when grown in serum free media (Figure 9C). These results are also in agreement with those obtained by the depletion of endogenous Artemin by siRNA. Further more, the antisera to Artemin was also able to inhibit MCF-7 cell invasion in a dose dependent manner (Figure 9D). A 51.3% and 74.6% reduction was observed at 200 µg/ml and 400 µg/ml of the antibodies, respectively. Further increases in antibody concentration provided no further inhibition.

### Production of chicken polyclonal antibodies against Artemin

We also produced chicken polyclonal antibodies using recombinant human Artemin. As shown in Figure 10, the chicken antibodies against Artemin were able to specifically recognise not only the endogenous Artemin in BT549-Vec cells but also forced Artemin expression in BT549-ARTN cells.

### Chicken polyclonal antibodies against Artemin reduce cell viability and anchorage-independent growth and inhibit invasion of MCF-7 cells

The viability of MCF-7 cells grown in media containing low serum (0.2%) was significantly reduced by chicken antibodies against Artemin. While the preimmune control had little effect, chicken polyclonal antibodies against Artemin reduced cell viability in a dose dependent manner (Figure 11A). At 200 µg/ml, cell viability was reduced to 67.7% compared with the IgY preimmune control. At 400 µg/ml, cell viability was reduced to 57.5% of the IgY treated control. At 800 µg/ml, cell viability was further reduced to 48.3% of the IgY treated control.

The chicken polyclonal antibodies against Artemin significantly inhibited anchorage-independent growth of MCF-7 cells in soft agar (Figure 11B). We observed inhibition by the antibodies in a dose dependent manner. At 200 µg/ml, soft agar growth was reduced to 83.9% compared with the IgY preimmune control. At 400 µg/ml, soft agar growth was reduced to 66.0% of IgY treated control. However, no further reductions were observed for concentrations from 400 to 600 µg/ml.

The invasiveness of MCF-7 cells was also impaired by chicken polyclonal antibodies against Artemin in a dose dependent fashion (Figure 11C). Significant inhibition was achieved with 600 µg/ml anti-Artemin antibodies, compared with the IgY preimmune control.

### Up-regulation of Artemin expression in Tamoxifen-resistant MCF-7 cells

We hypothesized that Artemin expression level might determine the sensitivity of chemotherapy drugs. After being progressively exposed to increasing concentrations of Tamoxifen, MCF-7 cells acquired the ability to grow in high concentration of Tamoxifen and became Tamoxifen-resistant. As shown in Figure 12, Artemin expression in Tamoxifen-resistant MCF-7 cells was significantly increased compared with the parent Tamoxifen-sensitive MCF-7 cells.

### Forced expression of Artemin significantly abrogates chemotherapy-induced death

MCF-7 cells are known to be ER-positive and, as disclosed above, Artemin was able to protect these cells against apoptosis. The effect of Artemin expression on the action of Tamoxifen was further analyzed by MTT assay. As shown in Figure 13A, Tamoxifen caused cell death in a dose dependent manner in both MCF7-ARTN cells with forced expression of Artemin and MCF7-Vec control cells. However, MCF7-ARTN cells showed significantly higher survival rates at all tested concentrations of Tamoxifen except 0.1 µM.

This result may be explained as Tamoxifen at lower concentrations (0.1-1 µM) has been shown to induce a cell-cycle arrest and pharmacological concentrations (above 5 µM) has been shown to induce apoptosis (Otto A, Paddenberg R, Schubert S, Mannhertz H 1996 Cell-cycle arrest, micronucleus formation, and cell death in growth inhibition of MCF-7 breast cancer cells by tamoxifen and cisplatin. J Cancer Res Clin Oncol 22:603-612. Perry R, Kang Y, Greaves B 1995 Effects of tamoxifen on growth and apoptosis of estrogen-dependent and -independent human breast cancer cells. Ann Surg Oncol 2:238-245). Similar protective effects of Artemin on Doxorubicin- and Taxol-induced cell death were also observed (Figures 13B and 13C). These results show that forced overexpression of Artemin is able to abrogate chemotherapy-induced cell death.

### Forced overexpression of Artemin significantly increases anchorage-independent growth in the presence of Tamoxifen.

The protective effect of Artemin against chemotherapeutic agents was further evaluated in soft agar assays. Consistent with the observations described above (see, e.g., Figure 3B), forced expression of Artemin resulted in more than a 2 fold increase in colony formation in the absence of Tamoxifen (Figure 14A). Tamoxifen reduced the number of colonies formed in soft agar at concentrations of 1 µM and 5 µM (Figure 14A). However, forced expression of Artemin abrogated this effect (Figure 14B). Forced expression of Artemin also diminished the effect of Tamoxifen on colony formation in Matrigel (Figure 14C).

### Synergistic inhibition of anchorage-independent growth of MCF-7 cells by chicken polyclonal antibodies against Artemin and anti-estrogenic agents

As shown in Figures 15A and 15B, both anti-estrogenic agents, Tamoxifen and Faslodex (ICI 182,780) effectively inhibited anchorage-independent growth of MCF-7 cells in soft agar. Tamoxifen (Figure 15A) and Faslodex (Figure 15B) reduced growth to 40.4 and 55.8%, respectively, compared with controls. Under the same experimental conditions, chicken polyclonal antibodies against Artemin reduced growth to 72.3%, compared with the preimmune control. Moreover, soft agar colony formation was dramatically reduced by the combination of Tamoxifen (Figure 15A) or Faslodex (Figure **15B****)** with anti-Artemin antibodies. The anchorage independent growth of MCF-7 was almost completely inhibited by the anti-Artemin antibodies together with either Tamoxifen (Figure 15A) or Faslodex (Figure 15B), with the colony formation reduced to 7.1 and 10.0%, compared with the controls, respectively.

### EXAMPLE 3: RESULTS

### Artemin is oncogenic for endometrial carcinoma cells

### Forced production of autocrine ARTN induces cellular growth of human endometrial carcinoma cells.

Human endometrial carcinoma RL95-2 cells were stably transfected with either Artemin expressing plasmid pIRESneo3-ARTN (designated RL95-2-ARTN) or the empty pIRESneo3 vector as control (RL95-2-CK). Increased expression of Artemin in RL95-2-ARTN cells, compared with control RL95-2-CK cells, was detected at mRNA level by RT-PCR and at protein level by Western blot analyses (Figure 16).

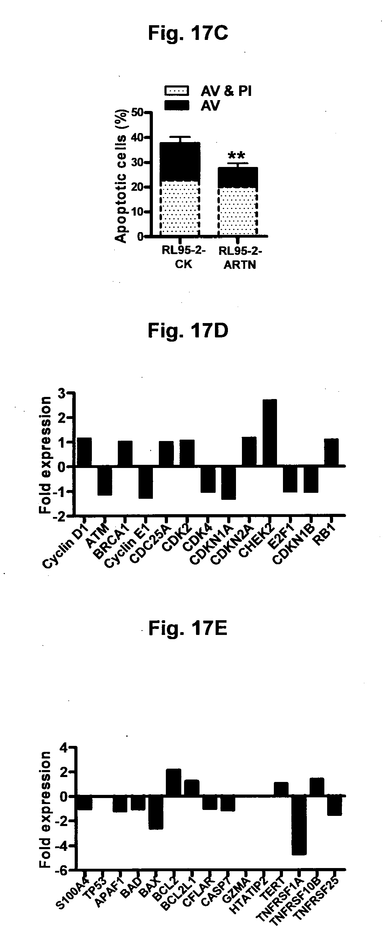

Forced expression of ARTN in RL95-2 cells significantly increased cellular growth as indicated by total cell assay (Figure 17A). Increased cell growth may result from the net outcome of increased cell cycle progression and/or a decrease in apoptosis. We demonstrated here that forced expression of ARTN in RL95-2 cells significantly increased mitogenesis in presence or absence of FBS (Figure 17B) and subsequently diminished apoptotic cell death as a consequence of serum deprivation (Figure 17C) as compared to RL95-2-CK cells.

Transcriptional regulatory genes involve in cell cycle progression and cell survival are directly associated with activation of signalling pathways which involved or promote oncogenic behaviour of cells (Evan and Littlewood, 1998; Nagasawa et al., 1985). To verify functional outcome of forced production of Artemin by RL95-2 cells, we utilized real time PCR to evaluate transcriptional level of various gene markers involved in cell cycle progression and cell survival. Forced expression of Artemin in RL95-2 cells resulted in up-regulation of mRNA expression of cell cycle progression-required genes *DDND1*, *CDK2, CDC25A, BRCA1, CHEK2* and *RB1*; and anti-apoptotic genes *Bcl-2* and *BCL2L1;* and *TERT* (Figures 17D and 17E). Forced expression of Artemin also resulted in down-regulation of expression of *BAD, BAX,* and *CASP7* (Figure 17E). Thus, forced production of Artemin enhances cell cycle progression and reduced apoptosis. It subsequently activates genes required for both proliferation and cell survival, and results increased cellular growth of endometrial carcinoma cells.

### Forced expression of ARTN in endometrial carcinoma cells enhances anchorage independent growth and invasion potential

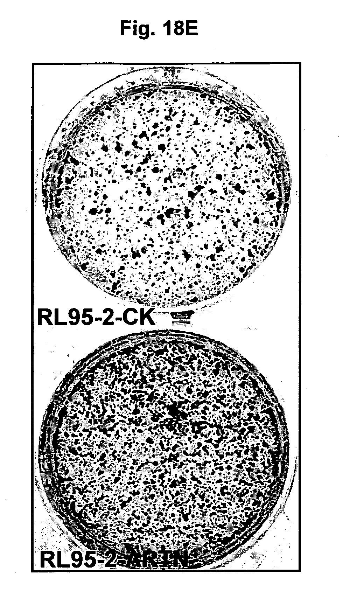

We next assessed whether forced expression Artemin affected the ability of RL95-2 cells to form colonies in soft agar assay. The results showed that RL95-2-ARTN cells formed more colonies in soft agar compared with RL95-2-CK cells (Figure 18A). RL95-2-ARTN cells also formed much bigger colonies than those formed by RL95-2-CK cells. In addition, RL95-2-ARTN cells demonstrated increased growth in suspension culture (Figure 18B) and exhibited dramatic foci formation (Figure 18E) compared with RL95-2-CK cells. This indicated that Artemin expression promoted anchorage independent growth of RL95-2 cells.

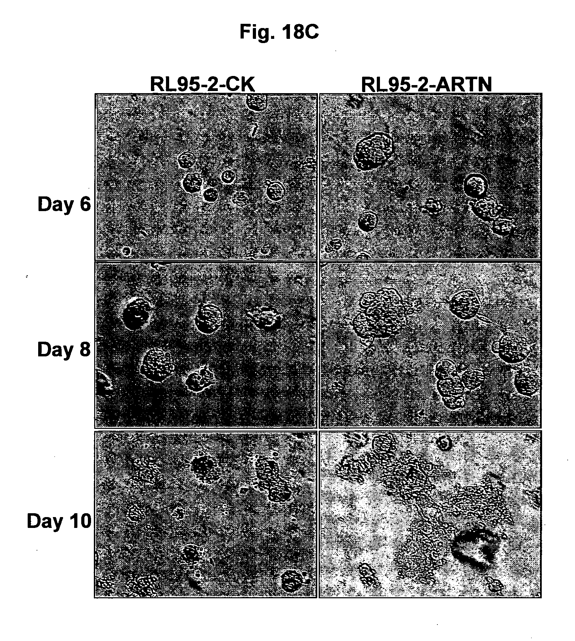

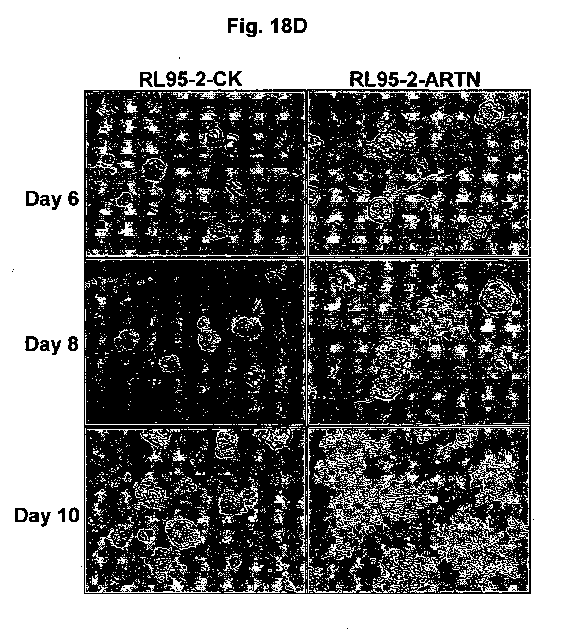

In order to examine the effect of forced expression of Artemin on morphological architecture, 2D and 3D Matrigel cultures were performed. RL95-2-ARTN cells formed colonies with a branching morphology in 2D (Figure 18D) and 3D (Figure 18C) Matrigel culture. RL95-2-ARTN cells exhibited mesenchymal branching morphology in 2D Matrigel culture (Figure 18D).

### Forced expression ARTN in endometrial carcinoma cell promotes mesenchymal phenotype and increased cell migration and invasion

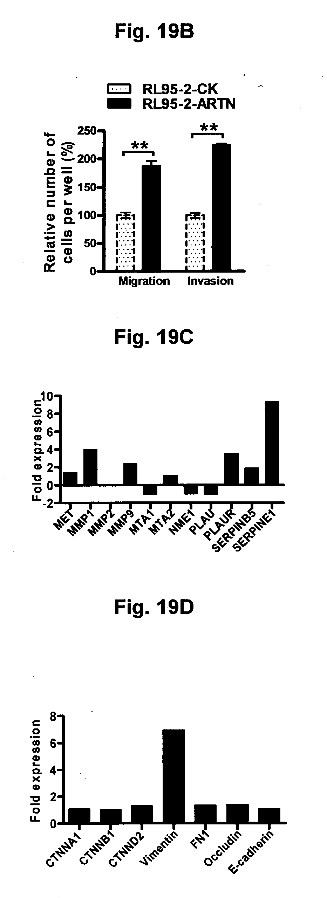

To closely examine the morphological effect of Artemin in RL95-2 cells, colony formation was monitored on a daily basis, starting from single cell status. Forced expression of Artemin in RL95-2 cells induced an EMT transition. The cells acquired a dramatic spindle cell morphology (Figure 19A), indicating invasive capabilities. As shown in Figure 19B, more than two fold the number of RL95-2-ARTN cells compared to RL95-2-CK cells were observed with migratory and invasive characteristics.

Real time PCR demonstrated that several invasion and metastasis-involved genes were found to be increased in RL95-2-ARTN cells compared with RL95-2-CK cells, including, e.g., *MMP1, MMP9, PLAUR, SERPINB5,* and *SERPINE1* (Figure 19C). Vimentin, a specific marker of the mesenchymal phenotype, was up-regulated 7 fold in RL95-2-ARTN cells relative to RL95-2-CK cells (Figure 19D).

### Depletion of ARTN by siRNA in endometrial carcinoma cells reduces oncogenic phenotype and induced apoptosis

Plasmid based siRNA was used to deplete ARTN expression. RL95-2 cells were stably transfected with the pSilencer-ARTN plasmid (RL95-2-siARTN) or the negative control pSilencer-CK plasmid (RL95-2-siCK). Depleted expression of Artemin was confirmed at the mRNA level by RT-PCR, and at the protein level by Western blot analysis (Figure 16).

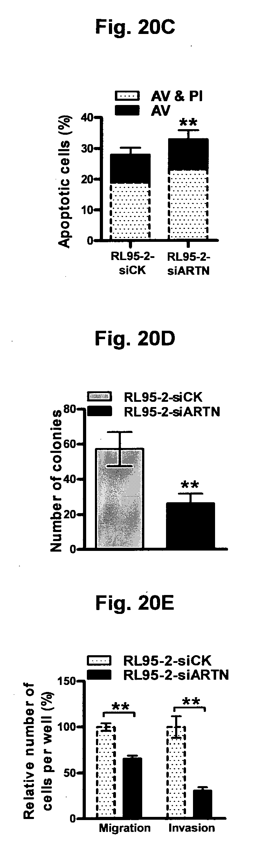

As shown by total cell number assay, the growth of RL95-siARTN cells was significantly slower than RL95-2-siCK cells (Figure 20A). BrdU incorporation and apoptosis assays demonstrated that RL95-2-siARTN cells exhibited a decrease in cell cycle entry (Figure 20B) and an increase in apoptosis (Figure 20C). Depletion of Artemin also impaired anchorage independent growth (Figure 20D), cell migration and invasion (Figure 20E).

### Forced expression of Artemin enhances anchorage-independent growth, cell migration, and invasion in endometrial carcinoma AN3 cells, while depletion of Artemin impairs the same

To extend our observations in RL95-2 cells to other endometrial carcinoma cells, AN3 cells were stably transfected with either Artemin expressing plasmid pIRESneo3-ARTN (AN3-ARTN) or the empty pIRESneo3 vector as control (AN3-CK). The forced expression of Artemin in AN3-ARTN cells was confirmed at the mRNA level by RT-PCR, and at the protein level by Western blot analysis (Figure 16). Similar to RL95-2 cells, forced expression of Artemin in AN3 cells increased anchorage-independent growth (Figure 21 A) and also promoted cell migration and invasion (Figure 21C).

To deplete endogenous Artemin expression in AN3 cells, cells were stably transfected with the pSilencer-ARTN plasmid (AN3-siARTN) or with the negative control pSilencer-CK plasmid (AN3-siCK). The depleted expression of Artemin in AN3-siARTN cells was confirmed at the mRNA level by RT-PCR, and at the protein level by Western blot analysis (Figure 16). In contrast to forced expression, depletion of Artemin in AN3 cells decreased anchorage-independent growth (Figure 21B) as well as cell migration and invasion (Figure 21D).

### Chicken polyclonal antibodies against Artemin reduce cell viability and anchorage-independent growth and inhibit invasion of endometrial carcinoma cells

The cell viability of RL95-2 cells grown in media containing low serum (0.2%) was not significantly affected by the preimmune control IgY. However, it was significantly reduced by chicken polyclonal antibodies against Artemin in a dose dependent manner. At 200 µg/ml, both anti-Artemin antibodies and the preimmune control IgY reduced cell viability to almost to the same extent (Figure 22A). However, as the concentration increased to 400, 600 and 800 µg/ml, the effect of the control IgY on cell viability was without effect, whereas the anti-Artemin antibodies demonstrated a significant inhibitory effect.

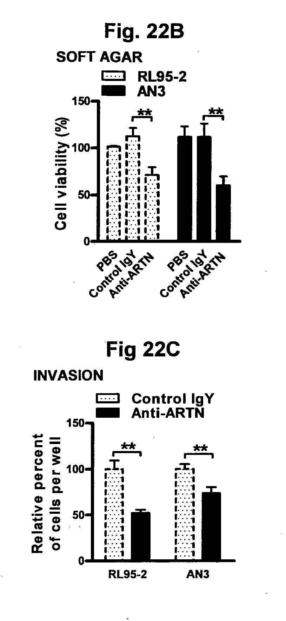

The anchorage-independent growth of both RL95-2 and AN3 cells in soft agar was significantly inhibited by chicken polyclonal antibodies against Artemin (Figure 22B). The preimmune control IgY at 400 µg/ml did not affect the colony formation significantly compared to PBS control, but the anti-Artemin antibodies at the same concentration significantly reduced the colony formation. The viability was reduced by 37.1 and 46.1% for RL95-2 and AN3 cells, respectively, compared with the control IgY.

The invasion of both RL95-2 and AN3 cells was also significantly inhibited by the chicken polyclonal antibodies against Artemin as revealed in Transwell invasion assays (Figure 22C). Compared with the control IgY, the anti-Artemin antibodies reduced the invasion of RL95-2 and AN3 cells by 47.7 and 26.3%, respectively.

Throughout this specification, and any claims which follow, unless the context requires otherwise, the words "comprise," "comprising" and the like, are to be construed in an inclusive sense as opposed to an exclusive sense, that is to say, in the sense of "including, but not limited to".

### REFERENCES

Airaksinen MS and Saarma M. (2002). The GDNF family: signalling, biological functions and therapeutic value. Nat Rev Neurosci 3: 383-394.
Airavaara M, Planken A, Gaddnas H, Piepponen TP, Saarma M, and Ahtee L. (2004). Increased extracellular dopamine concentrations and FosB/DeltaFosB expression in striatal brain areas of heterozygous GDNF knockout mice. Eur J Neurosci 20: 2336-2344.
Baudet C, Mikaels A, Westphal H, Johansen J, Johansen TE, and Ernfors P. (2000). Positive and negative interactions of GDNF, NTN and ART in developing sensory neuron subpopulations, and their collaboration with neurotrophins. Development 127: 4335-4344.
Bespalov MM and Saarma M. (2007). GDNF family receptor complexes are emerging drug targets. Trends Pharmacol Sci 28: 68-74.
Ceyhan GO, Bergmann F, Kadihasanoglu M, Erkan M, Park W, Hinz U et al. (2006a). The neurotrophic factor artemin influences the extent of neural damage and growth in chronic pancreatitis. Gut*.*
Ceyhan GO, Giese NA, Erkan M, Kerscher AG, Wente MN, Giese T et al. (2006b). The neurotrophic factor artemin promotes pancreatic cancer invasion. Ann Surg 244: 274-281.
Coulpier M, Anders J, and Ibanez CF. (2002). Coordinated activation of autophosphorylation sites in the RET receptor tyrosine kinase: importance of tyrosine 1062 for GDNF mediated neuronal differentiation and survival. J Biol Chem 277: 1991-1999.
Garces A, Livet J, Grillet N, Henderson CE, and Delapeyriere O. (2001). Responsiveness to neurturin of subpopulations of embryonic rat spinal motoneuron does not correlate with expression of GFR alpha 1 or GFR alpha 2. Dev Dyn 220: 189-197.
Gardell LR, Wang R, Ehrenfels C, Ossipov MH, Rossomando AJ, Miller S et al. (2003). Multiple actions of systemic artemin in experimental neuropathy. Nat Med 9: 1383-1389.
Gill SS, Patel NK, Hotton GR, O'Sullivan K, McCarter R, Bunnage M et al. (2003). Direct brain infusion of glial cell line-derived neurotrophic factor in Parkinson disease. Nat Med 9: 589-595.
Golden JP, Milbrandt J, and Johnson EM, Jr. (2003). Neurturin and persephin promote the survival of embryonic basal forebrain cholinergic neurons in vitro. Exp Neurol 184: 447-455.
He DY, McGough NN, Ravindranathan A, Jeanblanc J, Logrip ML, Phamluong K et al. (2005). Glial cell line-derived neurotrophic factor mediates the desirable actions of the anti-addiction drug ibogaine against alcohol consumption. JNeurosci 25: 619-628.
Henderson CE, Phillips HS, Pollock RA, Davies AM, Lemeulle C, Armanini M et al. (1994). GDNF: a potent survival factor for motoneurons present in peripheral nerve and muscle. Science 266: 1062-1064.
Horger BA, Nishimura MC, Armanini MP, Wang LC, Poulsen KT, Rosenblad C et al. (1998). Neurturin exerts potent actions on survival and function of midbrain dopaminergic neurons. J Neurosci 18: 4929-4937.
Ito Y, Okada Y, Sato M, Sawai H, Funahashi H, Murase T et al. (2005). Expression of glial cell line-derived neurotrophic factor family members and their receptors in pancreatic cancers. Surgery 138: 788-794.
Knowles PP, Murray-Rust J, Kjaer S, Scott RP, Hanrahan S, Santoro M et al. (2006). Structure and chemical inhibition of the RET tyrosine kinase domain. J Biol Chem 281: 33577-33587.
Rosenblad C, Gronborg M, Hansen C, Blom N, Meyer M, Johansen J et al. (2000). In vivo protection of nigral dopamine neurons by lentiviral gene transfer of the novel GDNF-family member neublastin/artemin. Mol Cell Neurosci 15: 199-214.
Santoro M, Melillo RM, Carlomagno F, Vecchio G, and Fusco A. (2004). Minireview: RET: normal and abnormal functions. Endocrinology 145: 5448-5451.
Stott FJ, Bates S, James MC, McConnell BB, Starborg M, Brookes S et al. (1998). The alternative product from the human CDKN2A locus, p14(ARF), participates in a regulatory feedback loop with p53 and MDM2. EMBO J 17: 5001-5014.
Takahashi M. (2001). The GDNF/RET signaling pathway and human diseases. Cytokine Growth Factor Rev 12: 361-373.
Tomac AC, Agulnick AD, Haughey N, Chang CF, Zhang Y, Backman C et al. (2002). Effects of cerebral ischemia in mice deficient in Persephin. Proc Natl Acad Sci USA 99: 9521-9526.
Wu K, Weng Z, Tao Q, Lin G, Wu X, Qian H et al. (2003). Stage-specific expression of breast cancer-specific gene gamma-synuclein. Cancer Epidemiol Biomarkers Prev 12: 920-925.
Zhang P, Chan SL, Fu W, Mendoza M, and Mattson MP. (2003). TERT suppresses apoptotis at a premitochondrial step by a mechanism requiring reverse transcriptase activity and 14-3-3 protein-binding ability. FASEB J 17: 767-769.
Zinkel S, Gross A, and Yang E. (2006). BCL2 family in DNA damage and cell cycle control. Cell Death Differ 13: 1351-1359.

## Claims

1. An agent that inhibits the biological activity of Artemin on cancer cell proliferation, cancer cell survival, or cancer cell oncogenicity, wherein Artemin comprises an amino acid sequence selected from SEQ ID NO: 1 to 3, SEQ ID NO. 5 to 32 or 34, amino acids 40 - 220 of SEQ ID NO. 1, amino acids 57 - 237 of SEQ ID NO. 2, amino acids 48 - 228 of SEQ ID NO. 3, or an amino acid sequence encoded by the nucleotide sequence of any one of SEQ ID NO: 35 to 42, for use in the treatment of mammary carcinoma or endometrial carcinoma in a subject, wherein the agent is
a) an antisense nucleotide molecule directed to a nucleotide sequence for Artemin, or
b) an interfering RNA molecule directed to a nucleotide sequence for Artemin, or
c) an antibody molecule which binds to an amino acid sequence for Artemin, or
d) a vector expressing any one of a) to c), or
e) a host cell comprising any one of a) to d).

2. The agent for use according to claim 1, wherein the agent is
a) an antibody molecule which binds to an amino acid sequence selected from the group consisting of SEQ ID NO:1-3, or 5-32 or 34, or
b) a vector expressing a), or
c) a host cell comprising any one of a) to b).

3. The agent for use according to claim 2, wherein the agent is a monoclonal antibody which binds to an amino acid sequence selected from the group consisting of SEQ ID NO:1-3, 5-32 or 34.

4. The agent for use according to claim 3, wherein the agent is a monoclonal antibody which binds to a polynucleotide comprising the nucleic acid sequence of any one of SEQ ID NO:35-42.

5. A method of monitoring mammary carcinoma or endometrial carcinoma in a subject, the method comprising contacting a sample from the subject with at least one agent, and determining the level of the polynucleotide for Artemin or the level of the Artemin polypeptide in the sample, wherein an elevated level of the polynucleotide for Artemin or of the Artemin polypeptide in the sample relative to a control sample indicates a predisposition for the development of mammary carcinoma or endometrial carcinoma or the presence of mammary carcinoma or endometrial carcinoma, and wherein the agent is
a) an antisense nucleotide molecule directed to a nucleotide sequence for Artemin, or
b) an interfering RNA molecule directed to a nucleotide sequence for Artemin, or
c) an antibody molecule which binds to an amino acid sequence for Artemin.

6. An antibody molecule which binds to an amino acid sequence selected from the group consisting of SEQ ID NO:1-3, 5-32 or 34, or a vector expressing said antibody molecule, or a host cell comprising said vector, for for use in a method of treating mammary carcinoma or endometrial carcinoma in a subject.

7. A method for assessing the presence of mammary carcinoma or endometrial carcinoma in a subject comprising contacting at least one polynucleotide which binds to the nucleic acid sequence of any one of SEQ ID NO:35-42 with a sample from the subject; and determining the level of the nucleic acid sequence in the sample, wherein an elevated level of the nucleic acid sequence in the sample relative to a control sample indicates the presence of mammary carcinoma or endometrial carcinoma.

8. A method for assessing the presence of mammary carcinoma or endometrial carcinoma in a subject comprising contacting at least one antibody molecule which binds to an amino acid sequence selected from the group consisting of SEQ ID NO:1-3, 5-32 or 34 with a sample from the subject; and determining the level of the amino acid sequence in the sample, wherein an elevated level of the amino acid sequence in the sample relative to a control sample indicates the presence of mammary carcinoma or endometrial carcinoma.

9. A composition comprising an agent as claimed in any one of claims 1 to 4, or an antibody as claimed in claim 6, and one or more pharmaceutically acceptable diluents, carriers, and/or excipients for use in the treatment of mammary carcinoma or endometrial carcinoma in a subject.

10. The composition for use according to claim 9, wherein the the agent is a monoclonal antibody which binds to an amino acid sequence selected from the group consisting of SEQ ID NO:1-3, or 5-32 or 34.

## Patentansprüche

1. Mittel, dass die biologische Wirkung von Artemin auf Krebszellproliferation, Krebszellüberleben oder Krebszellonkogenität inhibiert, wobei Artemin Folgendes beinhaltet: eine Aminosäuresequenz, die ausgewählt ist aus SEQ ID Nr. 1 bis 3, SEQ ID Nr. 5 bis 32 oder 34, Aminosäuren 40 - 220 von SEQ ID Nr. 1, Aminosäuren 57 - 237 von SEQ ID Nr. 2, Aminosäuren 48 - 228 von SEQ ID Nr. 3 oder eine Aminosäuresequenz, die durch die Nucleotidsequenz von beliebigen aus SEQ ID Nr. 35 bis 42 kodiert ist, zur Verwendung bei der Behandlung eines Mammakarzinoms oder Endometriumkarzinoms in einem Subjekt, wobei das Mittel Folgendes ist:
a) ein Antisense-Nucleotidmolekül, das auf eine Nucleotidsequenz für Artemin gerichtet ist, oder
b) ein interferierendes RNA-Molekül, das auf eine Nucleotidsequenz für Artemin gerichtet ist, oder
c) ein Antikörpermolekül, das sich an eine Aminosäuresequenz für Artemin bindet, oder
d) ein Vektor, der beliebige aus a) bis c) exprimiert, oder
e) eine Wirtszelle, die beliebige aus a) bis d) beinhaltet.

2. Mittel zur Verwendung nach Anspruch 1, wobei das Mittel Folgendes ist:
a) ein Antikörpermolekül, das sich an eine Aminosäuresequenz bindet, die aus der Gruppe bestehend aus SEQ ID Nr. 1-3 oder 5-32 oder 34 ausgewählt ist, oder
b) ein Vektor, der a) exprimiert, oder
c) eine Wirtszelle, die beliebige aus a) bis b) beinhaltet.

3. Mittel zur Verwendung nach Anspruch 2, wobei das Mittel ein monoklonaler Antikörper ist, der sich an eine Aminosäuresequenz bindet, die aus der Gruppe bestehend aus SEQ ID Nr. 1-3, 5-32 oder 34 ausgewählt ist.

4. Mittel zur Verwendung nach Anspruch 3, wobei das Mittel ein monoklonaler Antikörper ist, der sich an ein Polynucleotid bindet, das die Nucleinsäuresequenz von einem aus SEQ ID Nr. 35-42 beinhaltet.

5. Verfahren zum Überwachen eines Mammakarzinoms oder Endometriumkarzinoms in einem Subjekt, wobei das Verfahren Folgendes beinhaltet: Inkontaktbringen einer Probe des Subjekts mit wenigstens einem Mittel und Bestimmen des Niveaus des Polynucleotids für Artemin oder des Niveaus des Artemin-Polypeptids in der Probe, wobei ein erhöhtes Niveau des Polynucleotids für Artemin oder des Artemin-Polypeptids in der Probe relativ zu einer Kontrollprobe eine Veranlagung zur Bildung eines Mammakarzinoms oder Endometriumkarzinoms oder die Anwesenheit eines Mammakarzinoms oder Endometriumkarzinoms anzeigt, und wobei das Mittel Folgendes ist:
a) ein Antisense-Nucleotidmolekül, das auf eine Nucleotidsequenz für Artemin gerichtet ist, oder
b) ein interferierendes RNA-Molekül, das auf eine Nucleotidsequenz für Artemin gerichtet ist, oder
c) ein Antikörpermolekül, das sich an eine Aminosäuresequenz für Artemin bindet.

6. Antikörpermolekül, das sich an eine Aminosäuresequenz bindet, die ausgewählt ist aus der Gruppe bestehend aus SEQ ID Nr. 1-3, 5-32 oder 34, oder ein Vektor, der das genannte Antikörpermolekül exprimiert, oder eine Wirtszelle, die den genannten Vektor beinhaltet, zur Verwendung in einem Verfahren zur Behandlung eines Mammakarzinoms oder Endometriumkarzinoms in einem Subjekt.

7. Verfahren zum Beurteilen der Anwesenheit eines Mammakarzinoms oder Endometriumkarzinoms in einem Subjekt, das Folgendes beinhaltet: Inkontaktbringen wenigstens eines Polynucleotids, das sich an die Nucleinsäuresequenz von beliebigen aus SEQ ID Nr. 35-42 bindet, mit einer Probe des Subjekts; und Bestimmen des Niveaus der Nucleinsäuresequenz in der Probe, wobei ein erhöhtes Niveau der Nucleinsäuresequenz in der Probe relativ zu einer Kontrollprobe die Anwesenheit eines Mammakarzinoms oder Endometriumkarzinoms anzeigt.

8. Verfahren zum Beurteilen der Anwesenheit eines Mammakarzinoms oder Endometriumkarzinoms in einem Subjekt, das Folgendes beinhaltet: Inkontaktbringen von wenigstens einem Antikörpermolekül, das sich an eine Aminosäuresequenz bindet, die aus der Gruppe bestehend aus SEQ ID Nr. 1-3, 5-32 oder 34 ausgewählt ist, mit einer Probe des Subjekts; und Bestimmen des Niveaus der Aminosäuresequenz in der Probe, wobei ein erhöhter Wert der Aminosäuresequenz in der Probe relativ zu einer Kontrollprobe die Anwesenheit eines Mammakarzinoms oder Endometriumkarzinoms anzeigt.

9. Zusammensetzung, die ein Mittel nach einem der Ansprüche 1 bis 4 oder einen Antikörper nach Anspruch 6 und ein oder mehrere pharmazeutisch akzeptable Verdünnungsmittel, Träger und/oder Exzipienten beinhaltet, zur Verwendung bei der Behandlung eines Mammakarzinoms oder Endometriumkarzinoms in einem Subjekt.

10. Zusammensetzung zur Verwendung nach Anspruch 9, wobei das Mittel ein monoklonaler Antikörper ist, der sich an eine Aminosäuresequenz bindet, die aus der Gruppe bestehend aus SEQ ID Nr. 1-3 oder 5-32 oder 34 ausgewählt ist.

## Revendications

1. Agent qui inhibe l'activité biologique de l'artémine sur la prolifération de cellules cancéreuses, la survie de cellules cancéreuses, ou l'oncogénicité de cellules cancéreuses, où l'artémine comprend une séquence d'acides aminés sélectionnée parmi les SEQ ID NO : 1 à 3, les SEQ ID NO : 5 à 32 ou 34, les acides aminés 40-220 de la SEQ ID NO : 1, les acides aminés 57-237 de la SEQ ID NO : 2, les acides aminés 48-228 de la SEQ ID NO : 3, ou une séquence d'acides aminés codée par la séquence de nucléotides de l'une quelconque des SEQ ID NO : 35 à 42, à utiliser dans le traitement du carcinome mammaire ou du carcinome de l'endomètre chez un sujet, où l'agent est
a) une molécule nucléotidique anti-sens dirigée contre une séquence de nucléotides pour l'artémine, ou bien
b) une molécule d'ARN interférent dirigée contre une séquence de nucléotides pour l'artémine, ou bien
c) une molécule d'anticorps qui se lie à une séquence d'acides aminés pour l'artémine, ou bien
d) un vecteur exprimant l'un quelconque d'entre a) à c), ou bien
e) une cellule hôte comprenant l'un quelconque d'entre a) à d).

2. Agent à utiliser selon la revendication 1, où l'agent est
a) une molécule d'anticorps qui se lie à une séquence d'acides aminés sélectionnée parmi le groupe consistant en les SEQ ID NO : 1-3, ou 5-32 ou 34, ou bien
b) un vecteur exprimant a), ou bien
c) une cellule hôte comprenant l'un quelconque d'entre a) à b).

3. Agent à utiliser selon la revendication 2, où l'agent est un anticorps monoclonal qui se lie à une séquence d'acides aminés sélectionnée parmi le groupe consistant en les SEQ ID NO : 1-3, 5-32 ou 34.

4. Agent à utiliser selon la revendication 3, où l'agent est un anticorps monoclonal qui se lie à un polynucléotide comprenant la séquence d'acide nucléique de l'une quelconque des SEQ ID NO : 35-42.

5. Méthode de surveillance du carcinome mammaire ou du carcinome de l'endomètre chez un sujet, la méthode comprenant mettre un échantillon du sujet en contact avec au moins un agent, et déterminer le niveau du polynucléotide pour l'artémine ou le niveau du polypeptide artémine dans l'échantillon, dans laquelle un niveau élevé du polynucléotide pour l'artémine ou du polypeptide artémine dans l'échantillon par rapport à un échantillon témoin indique une prédisposition au développement du carcinome mammaire ou du carcinome de l'endomètre ou la présence de carcinome mammaire ou de carcinome de l'endomètre et dans laquelle l'agent est
a) une molécule nucléotidique anti-sens dirigée contre une séquence de nucléotides pour l'artémine, ou bien
b) une molécule d'ARN interférent dirigée contre une séquence de nucléotides pour l'artémine, ou bien
c) une molécule d'anticorps qui se lie à une séquence d'acides aminés pour l'artémine.

6. Molécule d'anticorps qui se lie à une séquence d'acides aminés sélectionnée parmi le groupe consistant en les SEQ ID NO : 1-3, 5-32 ou 34, ou bien un vecteur exprimant ladite molécule d'anticorps, ou bien une cellule hôte comprenant ledit vecteur, à utiliser dans une méthode de traitement de carcinome mammaire ou de carcinome de l'endomètre chez un sujet.

7. Méthode d'évaluation de la présence de carcinome mammaire ou de carcinome de l'endomètre chez un sujet comprenant mettre au moins un polynucléotide qui se lie à la séquence d'acide nucléique de l'une quelconque des SEQ ID NO : 35-42, en contact avec un échantillon du sujet ; et déterminer le niveau de la séquence d'acide nucléique dans l'échantillon, où un niveau élevé de la séquence d'acide nucléique dans l'échantillon par rapport à un échantillon témoin indique la présence de carcinome mammaire ou de carcinome de l'endomètre.

8. Méthode d'évaluation de la présence de carcinome mammaire ou de carcinome de l'endomètre chez un sujet comprenant mettre au moins une molécule d'anticorps qui se lie à une séquence d'acides aminés sélectionnée parmi le groupe consistant en les SEQ ID NO : 1-3, 5-32 ou 34, en contact avec un échantillon du sujet ; et déterminer le niveau de la séquence d'acides aminés dans l'échantillon, où un niveau élevé de la séquence d'acides aminés dans l'échantillon par rapport à un échantillon témoin indique la présence de carcinome mammaire ou de carcinome de l'endomètre.

9. Composition comprenant un agent selon l'une quelconque des revendications 1 à 4, ou un anticorps selon la revendication 6, et un ou plusieurs diluants, véhicules et/ou excipients pharmaceutiquement acceptables, à utiliser dans le traitement du carcinome mammaire ou du carcinome de l'endomètre chez un sujet.

10. Composition à utiliser selon la revendication 9, dans laquelle l'agent est un anticorps monoclonal qui se lie à une séquence d'acides aminés sélectionnée parmi le groupe consistant en les SEQ ID NO : 1-3 ou 5-32 ou 34.
